# EUROPEAN PATENT APPLICATION

(11) **EP 4 151 227 A1**
(43) Date of publication of application: **22.03.2023**
(21) Application number: 21804472.5
(22) Date of filing: 11.05.2021
(51) Int. Cl.: A61K 38/08, A61K 38/10, A61K 38/16, A61K 39/00, A61P 35/00, A61P 35/02

(54) **PHARMACEUTICAL COMPOSITION FOR TREATING CANCER**

(30) Priority: 12.05.2020 JP 2020083905
(71) Applicant: Sumitomo Pharma Co., Ltd., Osaka 541-0045 (JP); International Institute of Cancer Immunology, Inc., Osaka 564-0053 (JP)
(72) Inventor: GOTO, Masashi, Osaka-shi, Osaka 554-0022 (JP); SUGINOBE, Natsuko, Osaka-shi, Osaka 554-0022 (JP); NAKAMURA, Megumi, Osaka-shi, Osaka 554-0022 (JP)
(74) Representative: Vossius & Partner Patentanwälte Rechtsanwälte mbB
(86) International application number: PCT/JP2021/017929
(87) International publication number: WO 2021/230247

(57) **Abstract**

The present disclosure includes a pharmaceutical composition for treating a cancer in an HLA-A*02:07, HLA-A^{∗}03:01, HLA-B^{∗}15:01, or HLA-B*27:05-positive subject comprising a WT1-derived cancer antigen peptide or a peptide conjugate containing the peptide.

## Description

### Technical Field

The present application claims the priority of Japanese Patent Application No. 2020-083905, which is incorporated herein by reference in its entirety.

The present disclosure belongs to the field of cancer immunotherapy, and particularly relates to use of cancer antigen peptides derived from WT1 protein.

### Background

WT1 is a cancer antigen protein that is highly expressed in leukemia and various solid cancers and particularly attracts attention among cancer-vaccine target antigens. Cellular immunity, particularly cytotoxic T cells (also called as cytotoxic T lymphocytes, hereinafter, referred to as CTLs) play an important role in cancer cell clearance by a living body. CTLs that attack cancer cells are derived from precursor T cells through their differentiation and proliferation, upon recognition by precursor T cells of a peptide from a cancer antigen protein (i.e., cancer antigen peptide) complexed with an MHC class I molecule. As the WT1-derived cancer antigen peptide, for example, WT1₁₂₆₋₁₃₄ peptide (RMFPNAPYL, SEQ ID NO: 2), WT1₂₃₅₋₂₄₃ peptide (CMTWNQMNL, SEQ ID NO: 3), WT1₁₀₋₁₈ peptide (ALLPAVPSL, SEQ ID NO: 4), WT1₁₈₇₋₁₉₅ peptide (SLGEQQYSV, SEQ ID NO: 5), WT1₃₀₂₋₃₁₀ peptide (RVPGVAPTL, SEQ ID NO: 6) and WT1₃₇₋₄₅ peptide (VLDFAPPGA, SEQ ID NO: 7), are known.

For example, WT1₁₂₆₋₁₃₄ peptide is a cancer antigen peptide that can induce HLA-A*02:01-restricted peptide specific CTLs. Since CTLs specific to the WT1₁₂₆₋₁₃₄ peptide show cytotoxic activity against HLA-A*02:01-positive WT1 expressing cancer cells, a cancer vaccine containing the WT1₁₂₆₋₁₃₄ peptide has been subjected to a clinical trial in HLA-A*02:01-positive patients.

### Summary

### Technical Problem

One object of the present disclosure is to expand the range of subjects to which a WT1-derived cancer antigen peptide or a peptide conjugate containing the peptide is applicable.

### Solution to Problem

By using peripheral blood of cancer patients who received a cancer peptide vaccine, the inventors found HLA class I restriction for WT1-derived cancer antigen peptides which could not be identified by binding prediction to HLA class I or assays using T cells derived from peripheral blood of cancer patients before administration of the cancer peptide vaccine, and based on the findings accomplished the present invention.

In an embodiment, the present disclosure provides a pharmaceutical composition for treating a cancer in an HLA-A*02:07, HLA-A*03:01, HLA-B*15:01, or HLA-B^{∗}27:05-positive subject, comprising:
(a) an MHC class I-restricted peptide consisting of 7 to 30 amino acid residues or a pharmaceutically acceptable salt thereof, wherein the MHC class I-restricted peptide is a peptide comprising an amino acid sequence selected from RMFPNAPYL (SEQ ID NO: 2), CMTWNQMNL (SEQ ID NO: 3), ALLPAVPSL (SEQ ID NO: 4), SLGEQQYSV (SEQ ID NO: 5), RVPGVAPTL (SEQ ID NO: 6) and VLDFAPPGA (SEQ ID NO: 7), or a peptide comprising an amino acid sequence which has one or several amino acids deleted from, substituted in, and/or added to the amino acid sequence selected from SEQ ID NOs: 2 to 7 and having an ability to induce CTLs; or
(b) a compound represented by the formula (1) or a pharmaceutically acceptable salt thereof: 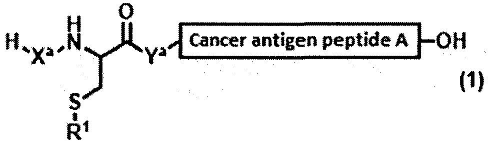 wherein X^{a} and Y^{a} each independently represent a single bond or a divalent peptide group consisting of 1 to 4 amino acid residues, provided that the sum of the numbers of amino acid residues in X^{a} and Y^{a} is an integer of 0 to 4;

cancer antigen peptide A is an MHC class I-restricted peptide consisting of 7 to 30 amino acid residues, wherein an amino group of an N-terminal amino acid of the cancer antigen peptide A binds to Y^{a} in the formula (1), and a carbonyl group of a C-terminal amino acid of the cancer antigen peptide A binds to the hydroxyl group in the formula (1),
R¹ is a hydrogen atom,
a group represented by the formula (2): 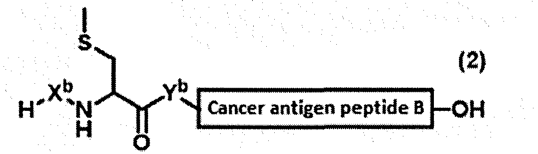 wherein X^{b} and Y^{b} each independently represent a single bond or a divalent peptide group consisting of 1 to 4 amino acid residues, provided that the sum of the numbers of amino acid residues in X^{b} and Y^{b} is an integer of 0 to 4,
cancer antigen peptide B is an MHC class I-restricted peptide consisting of 7 to 30 amino acid residues, wherein an amino group of an N-terminal amino acid of the cancer antigen peptide B binds to Y^{b} in the formula (2), and a carbonyl group of a C-terminal amino acid of the cancer antigen peptide B binds to the hydroxyl group in the formula (2), and
the sulfur atom in the formula (2) binds to the sulfur atom in the formula (1) via a disulfide bond,
or cancer antigen peptide C, wherein the cancer antigen peptide C is an MHC class I-restricted peptide consisting of 7 to 30 amino acid residues including one cysteine residue or an MHC class II-restricted peptide consisting of 7 to 30 amino acid residues including one cysteine residue, and a sulfur atom of the cysteine residue of the cancer antigen peptide C binds to the sulfur atom in the formula (1) via a disulfide bond, and optionally a peptide consisting of 1 to 4 amino acid residues binds to an N-terminus of the cancer antigen peptide C,
provided that when R¹ is a hydrogen atom, the cancer antigen peptide A is a peptide comprising an amino acid sequence selected from RMFPNAPYL (SEQ ID NO: 2), CMTWNQMNL (SEQ ID NO: 3), ALLPAVPSL (SEQ ID NO: 4), SLGEQQYSV (SEQ ID NO: 5), RVPGVAPTL (SEQ ID NO: 6) and VLDFAPPGA (SEQ ID NO: 7), or a peptide comprising an amino acid sequence which has one or several amino acids deleted from, substituted in, and/or added to the amino acid sequence selected from SEQ ID NOs: 2 to 7 and having an ability to induce CTLs;
when R¹ is the group represented by the formula (2), the cancer antigen peptide A and/or cancer antigen peptide B is a peptide comprising an amino acid sequence selected from RMFPNAPYL (SEQ ID NO: 2), CMTWNQMNL (SEQ ID NO: 3), ALLPAVPSL (SEQ ID NO: 4), SLGEQQYSV (SEQ ID NO: 5), RVPGVAPTL (SEQ ID NO: 6) and VLDFAPPGA (SEQ ID NO: 7), or a peptide comprising an amino acid sequence which has one or several amino acids deleted from, substituted in, and/or added to the amino acid sequence selected from SEQ ID Nos: 2 to 7 and having an ability to induce CTLs;
when R¹ is the cancer antigen peptide C, the cancer antigen peptide A and/or cancer antigen peptide C is a peptide comprising an amino acid sequence selected from RMFPNAPYL (SEQ ID NO: 2), CMTWNQMNL (SEQ ID NO: 3), ALLPAVPSL (SEQ ID NO: 4), SLGEQQYSV (SEQ ID NO: 5), RVPGVAPTL (SEQ ID NO: 6) and VLDFAPPGA (SEQ ID NO: 7), or a peptide comprising an amino acid sequence which has one or several amino acids deleted from, substituted in, and/or added to the amino acid sequence selected from SEQ ID Nos: 2 to 7 and having an ability to induce CTLs;
when R¹ is the group represented by the formula (2) and the cancer antigen peptide B includes one cysteine residue, a sulfur atom of the cysteine residue of the cancer antigen peptide B optionally binds, via a disulfide bond, to
a sulfur atom in the formula (3): 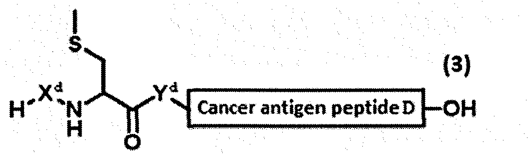 wherein X^{d} and Y^{d} each independently represent a single bond or a divalent peptide group consisting of 1 to 4 amino acid residues, provided that the sum of the numbers of amino acid residues in X^{d} and Y^{d} is an integer of 0 to 4, and
cancer antigen peptide D is an MHC class II-restricted peptide consisting of 7 to 30 amino acid residues, wherein an amino group of an N-terminal amino acid of the cancer antigen peptide D binds to Y^{d} in the formula (3), and a carbonyl group of a C-terminal amino acid of the cancer antigen peptide D binds to the hydroxyl group in the formula (3),
or a sulfur atom of a cysteine residue of cancer antigen peptide E, wherein the cancer antigen peptide E is an MHC class II-restricted peptide consisting of 7 to 30 amino acid residues including one cysteine residue; and
when R¹ is the cancer antigen peptide C and a peptide consisting of 1 to 4 amino acid residues including one cysteine residue binds to the N terminus of the cancer antigen peptide C, a sulfur atom of the cysteine residue of the peptide binding to the N terminus of the cancer antigen peptide C optionally binds, via a disulfide bond, to
a sulfur atom in the formula (3): 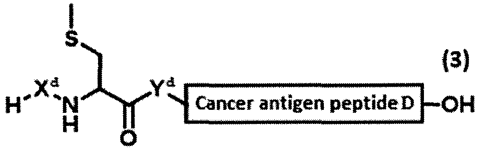 wherein X^{d} and Y^{d} each independently represent a single bond or a divalent peptide group consisting of 1 to 4 amino acid residues, provided that the sum of the numbers of amino acid residues in X^{d} and Y^{d} is an integer of 0 to 4, and
cancer antigen peptide D is an MHC class II-restricted peptide consisting of 7 to 30 amino acid residues, wherein an amino group of an N-terminal amino acid of the cancer antigen peptide D binds to Y^{d} in the formula (3), and a carbonyl group of a C-terminal amino acid of the cancer antigen peptide D binds to the hydroxyl group in the formula (3),
or a sulfur atom of a cysteine residue of cancer antigen peptide E, wherein the cancer antigen peptide E is an MHC class II-restricted peptide consisting of 7 to 30 amino acid residues including one cysteine residue.

### Effects of Invention

The present disclosure makes it possible to use a WT1-derived cancer antigen peptide such as WT1₁₂₆₋₁₃₄ peptide or a peptide conjugate containing the peptide for treating a cancer of an HLA-A*02:07, HLA-A*03:01, HLA-B*15:01 or HLA-B^{∗}27:05-positive subject, and expands the range of subjects to which a WT1-derived cancer antigen peptide or a peptide conjugate containing the peptide is applicable.

### Brief Description of Drawings

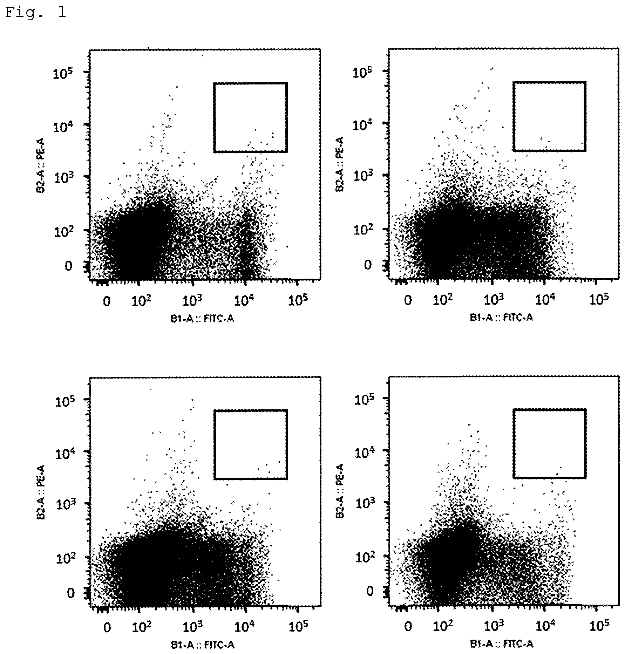

Figure 1 shows the results of tetramer assay using peripheral blood mononuclear cells (PBMCs) derived from HLA-A^{∗}03:01-positive patients. HLA-A^{∗}03:01-restricted WT1₁₂₆₋₁₃₄-specific CD8-positive T cells were analyzed in PBMCs derived from 4 patients which were prepared before administration of the medical agent and cultured in the presence of the compound of formula (5).

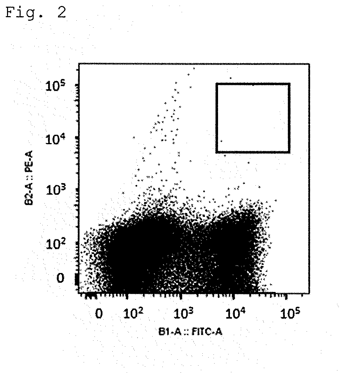

Figure 2 shows the results of tetramer assay using PBMCs derived from an HLA-B*15:01-positive patient. HLA-B^{∗}15:01-restricted WT1₁₂₆₋₁₃₄-specific CD8-positive T cells were analyzed in patient-derived PBMCs which were prepared before administration of the medical agent and cultured in the presence of the compound of formula (5).

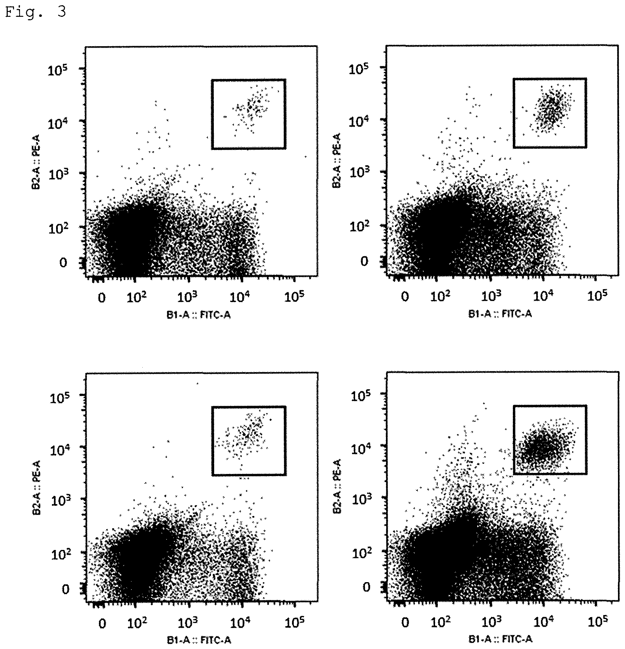

Figure 3 shows the results of tetramer assay using PBMCs derived from HLA-A*03:01-positive patient. HLA-A^{∗}03:01-restricted WT1₁₂₆₋₁₃₄-specific CD8-positive T cells were analyzed in PBMCs derived from 4 patients which were prepared after administration of the medical agent and cultured in the presence of the compound of formula (5).

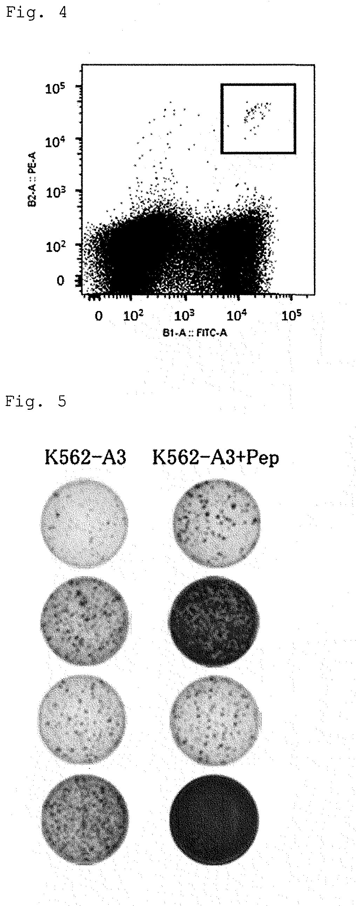

Figure 4 shows the results of tetramer assay using PBMCs derived from an HLA-B*15:01-positive patient. HLA-B^{∗}15:01-restricted WT1₁₂₆₋₁₃₄-specific CD8-positive T cells were analyzed in patient-derived PBMCs which were prepared after administration of the medical agent and cultured in the presence of the compound of formula (5).
Figure 5 shows the results of ELISPOT assay using HLA tetramer-positive T cells. PBMCs containing WT1₁₂₆₋₁₃₄/HLA-A*03:01 tetramer-positive CD8-positive T cells were stimulated with HLA-A^{∗}03:01-expressing K562 cells pulsed with WT1₁₂₆₋₁₃₄ peptide ("K562-A3 + Pep") or HLA-A*03:01 expressing K562 cells not pulsed with the peptide ("K562-A3"). The results of 4 subjects are shown.

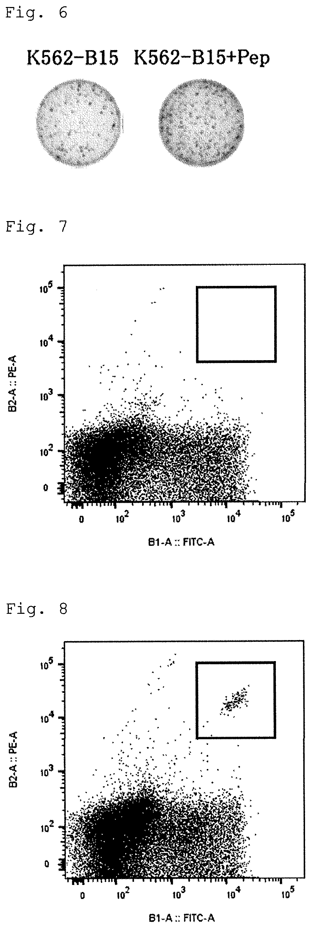

Figure 6 shows the results of ELISPOT assay using HLA tetramer-positive T cells. PBMCs containing WT1₁₂₆₋₁₃₄/HLA-B*15:01 tetramer-positive CD8-positive T cells were stimulated with HLA-B^{∗}15:01-expressing K562 cells pulsed with WT1₁₂₆₋₁₃₄ peptide ("K562-B15 + Pep") or HLA-B*15:01 expressing K562 cells not pulsed with the peptide ("K562-B15"). The results of one subject are shown.
Figure 7 shows the results of tetramer assay using PBMCs derived from an HLA-A*02: 07-positive patients. HLA-A^{∗}02:07-restricted WT1₁₂₆₋₁₃₄-specific CD8-positive T cells were analyzed in patient-derived PBMCs which were prepared before administration of the medical agent and cultured in the presence of the compound of formula (5).
Figure 8 shows the results of tetramer assay using PBMCs derived from an HLA-A*02:07-positive patients. HLA-A^{∗}02:07-restricted WT1₁₂₆₋₁₃₄-specific CD8-positive T cells were analyzed in patient-derived PBMCs which were prepared after administration of the medical agent and cultured in the presence of the compound of formula (5).

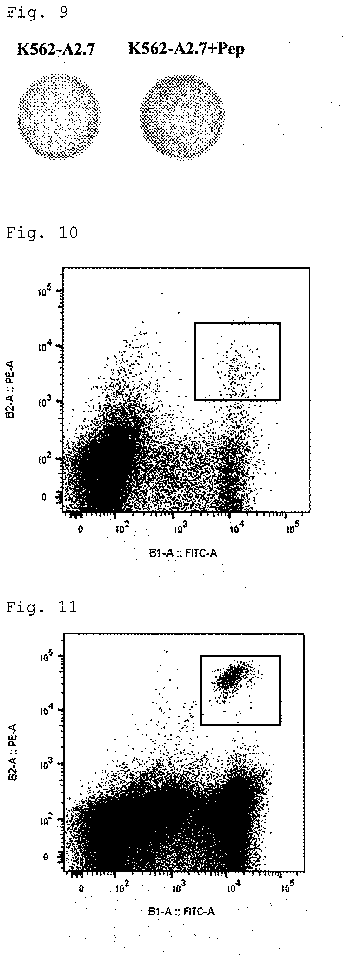

Figure 9 shows the results of ELISPOT assay using HLA tetramer-positive T cells. PBMCs containing WT1₁₂₆₋₁₃₄/HLA-A*02:07 tetramer-positive CD8-positive T cells were stimulated with HLA-A*02:07 expressing K562 cells pulsed with WT1₁₂₆₋₁₃₄ peptide ("K562-A2.7 + Pep") or HLA-A*02:07 expressing K562 cells not pulsed with the peptide ("K562-A2.7"). The results of one subject are shown.
Figure 10 shows the results of tetramer assay using PBMCs derived from an HLA-A*03:01-positive patient. HLA-A*03:01-restricted WT1₁₂₆₋₁₃₄-specific CD8-positive T cells were analyzed in patient-derived PBMCs which were prepared after administration of the medical agent and cultured in the presence of the peptide of SEQ ID NO: 13.
Figure 11 shows the results of tetramer assay using PBMCs derived from HLA-B*15:01-positive patients. HLA-B*15:01-restricted WT1₁₂₆₋₁₃₄-specific CD8-positive T cells were analyzed in patient-derived PBMCs which were prepared after administration of the medical agent and cultured in the presence of the peptide of SEQ ID NO: 13.

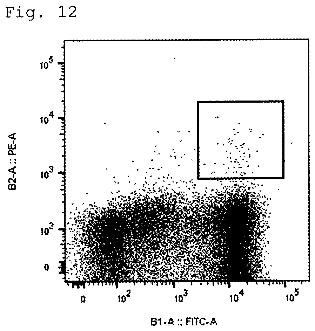

Figure 12 shows the results of tetramer assay using PBMCs derived from HLA-A*02:07-positive patients. HLA-A*02:07-restricted WT1₁₂₆₋₁₃₄-specific CD8-positive T cells were analyzed in patient-derived PBMCs which were prepared after administration of the medical agent and cultured in the presence of the peptide of SEQ ID NO: 13.

### Description of Embodiments

The "amino acid residue" as used herein refers to a single amino acid unit among amino acids constituting a peptide or a protein molecule. The "amino acid residue" may be a natural or non-natural α-amino acid residue, β-amino acid residue, γ-amino acid residue or δ-amino acid residue; more specifically, a natural α-amino acid residue, ornithine residue, homoserine residue, homocysteine residue, β-alanine residue, γ-aminobutanoic acid or δ-aminopentanoic acid. When an "amino acid residue" is optically active, it includes L-form and D-form, and is preferably L-form.

The abbreviations of "amino acid residues" used herein stand for the followings:
Ala or A: alanine residue
Arg or R: arginine residue
Asn or N: asparagine residue
Asp or D: aspartic acid residue
Cys or C: cysteine residue
Gln or Q: glutamine residue
Glu or E: glutamic acid residue
Gly or G: glycine residue
His or H: histidine residue
lie or I: isoleucine residue
Leu or L: leucine residue
Lys or K: lysine residue
Met or M: methionine residue
Phe or F: phenylalanine residue
Pro or P: proline residue
Ser or S: serine residue
Thr or T: threonine residue
Trp or W: tryptophan residue
Tyr or Y: tyrosine residue
Val or V: valine residue
Abu: 2-aminobutyric acid residue (also referred to as α-aminobutyric acid residue)
Orn: ornithine residue
Cit: citrulline residue

The amino acid sequence of a "peptide" is described herein so that the N-terminal amino acid residue is positioned on the left side and the C-terminal amino acid residue is positioned on the right side, in accordance with a usual description method. Unless otherwise indicated, in the "peptide", the amino group of the N-terminal amino acid residue binds to a hydrogen atom (to be a free amino group) and the carbonyl group of the C-terminal amino acid residue binds to a hydroxyl group. A divalent peptide group refers to a peptide group which is able to bind to other chemical moieties via the N-terminal amino group and via the C-terminal carbonyl group. In a peptide corresponding to a partial structure of a compound represented by the formula (1), for example, the compound of formula (4) or formula (5), unless otherwise indicated, the amino group of the N-terminal amino acid residue binds to a hydrogen atom and the carbonyl group of the C-terminal amino acid residue binds to a hydroxyl group.

The present disclosure relates to use of
(a) an MHC class I-restricted peptide consisting of 7 to 30 amino acid residues which is a peptide comprising an amino acid sequence selected from RMFPNAPYL (SEQ ID NO: 2), CMTWNQMNL (SEQ ID NO: 3), ALLPAVPSL (SEQ ID NO: 4), SLGEQQYSV (SEQ ID NO: 5), RVPGVAPTL (SEQ ID NO: 6) and VLDFAPPGA (SEQ ID NO: 7) or a peptide comprising an amino acid sequence which has one or several amino acids deleted from, substituted in, and/or added to the amino acid sequence selected from SEQ ID Nos: 2 to 7 and having an ability to induce CTLs, or a pharmaceutically acceptable salt thereof (herein also referred to as the peptide of (a)); or
(b) a compound represented by the formula (1): 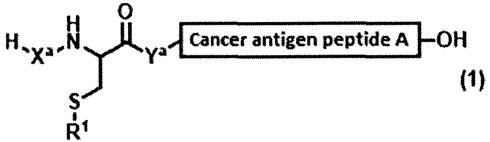 or a pharmaceutically acceptable salt thereof.

"Cancer antigen peptide A" refers to an MHC class I-restricted peptide consisting of 7 to 30 amino acid residues. In the formula (1), the amino group of the N-terminal amino acid of the cancer antigen peptide A binds to Y^{a} in the formula (1) and the carbonyl group of the C-terminal amino acid of the cancer antigen peptide A binds to the hydroxyl group in the formula (1).

"X^{a}" and "Y^{a}" each independently represent a single bond or a divalent peptide group consisting of 1 to 4 amino acid residues. The sum of the numbers of amino acid residues in X^{a} and Y^{a} is an integer of 0 to 4. For example, when the sum of the number of amino acid residues in X^{a} and Y^{a} is an integer of 0, both X^{a} and Y^{a} are single bonds. When the sum of the number of amino acid residues in X^{a} and Y^{a} is an integer of 4, each of X^{a} and Y^{a} may be a divalent peptide group consisting of two amino acid residues; X^{a} may be a divalent peptide group consisting of three amino acid residues and Y^{a} may be a divalent peptide group consisting of one amino acid residue; or X^{a} may be a divalent peptide group consisting of four amino acid residues and Y^{a} may be a single bond.

The sum of the number of amino acid residues in X^{a} and Y^{a} is preferably 0 to 2, more preferably 0 to 1, and most preferably zero. That is, most preferably, both X^{a} and Y^{a} are single bonds.

When the sum of the number of amino acid residues in X^{a} and Y^{a} is an integer of 2, X^{a} may be a divalent peptide group consisting of two amino acid residues and Y^{a} may be a single bond; X^{a} and Y^{a} may independently be a divalent peptide group consisting of one amino acid residue; or X^{a} may be a single bond and Y^{a} is a divalent peptide group consisting of two amino acid residues.

When the sum of the number of amino acid residues in X^{a} and Y^{a} is an integer of 1, X^{a} may be a divalent peptide group consisting of one amino acid residue and Y^{a} may be a single bond; or X^{a} may be a single bond and Y^{a} may be a divalent peptide group consisting of one amino acid residue. In a preferred embodiment, X^{a} is a single bond and Y^{a} is a residue of alanine, leucine or methionine; or X^{a} is a residue of alanine, leucine or methionine and Y^{a} is a single bond.

In the formula (1), "R¹" is a hydrogen atom, a group represented by the formula (2): 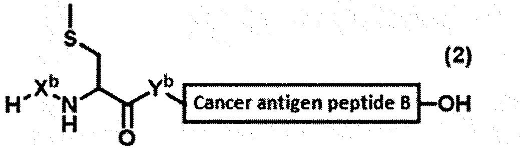 or cancer antigen peptide C. Preferably, "R¹" is a group represented by the formula (2) or cancer antigen peptide C and more preferably cancer antigen peptide C.

When R¹ is a hydrogen atom, the compound represented by the formula (1) is a compound (i.e., peptide) represented by the formula (1-1): 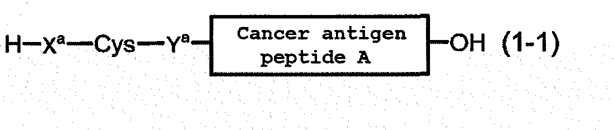 wherein X^{a}, Y^{a} and cancer antigen peptide A have the same meanings as defined above in relation to the formula (1), and Cys represents a cysteine residue.

"Cancer antigen peptide B" is an MHC class I-restricted peptide consisting of 7 to 30 amino acid residues. In the formula (2), the amino group of the N-terminal amino acid of the cancer antigen peptide B binds to Y^{b} in the formula (2) (or the formula (1-2)), and a carbonyl group of the C-terminal amino acid binds to the hydroxyl group in the formula (2).

"X^{b}" and "Y^{b}" each independently represent a single bond or a divalent peptide group consisting of 1 to 4 amino acid residues. The sum of the numbers of amino acid residues in X^{b} and Y^{b} is an integer of 0 to 4. For example, when the sum of the number of amino acid residues in X^{b} and Y^{b} is an integer of 0, both X^{b} and Y^{b} are single bonds. When the sum of the number of amino acid residues in X^{b} and Y^{b} is an integer of 4, each of X^{b} and Y^{b} may be a divalent peptide group consisting of two amino acid residues; X^{b} may be a divalent peptide group consisting of three amino acid residues and Y^{b} may be a divalent peptide group consisting of one amino acid residue; or X^{b} may be a divalent peptide group consisting of four amino acid residues and Y^{b} may be a single bond.

The sum of the number of amino acid residues in X^{b} and Y^{b} is preferably an integer of 0 to 2, more preferably an integer of 0 to 1, or most preferably zero. That is, most preferably, X^{b} and Y^{b} are both single bonds.

When the sum of the number of amino acid residues in X^{b} and Y^{b} is an integer of 2, X^{b} may be a divalent peptide group consisting of two amino acid residues and Y^{b} may be a single bond; each of X^{b} and Y^{b} may independently be a divalent peptide group consisting of one amino acid residue; or X^{b} may be a single bond and Y^{b} may be a divalent peptide group consisting of two amino acid residues.

When the sum of the number of amino acid residues in X^{b} and Y^{b} is an integer of 1, X^{b} may be a divalent peptide group consisting of one amino acid residue and Y^{b} may be a single bond; or X^{b} may be a single bond and Y^{b} may be a divalent peptide group consisting of one amino acid residue. In a preferred embodiment, X^{b} is a single bond and Y^{b} is a residue of alanine, leucine or methionine, or X^{b} is a residue of alanine, leucine or methionine, and Y^{b} is a single bond.

When R¹ is a group represented by the formula (2), the compound represented by the formula (1) is a compound represented by the formula (1-2): 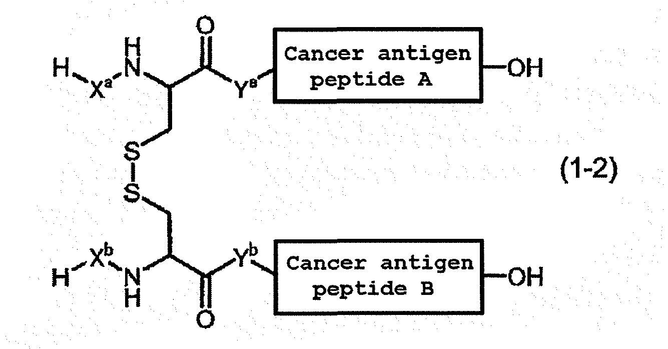 wherein X^{a}, Y^{a} and cancer antigen peptide A have the same meanings as defined above in relation to the formula (1), and X^{b}, Y^{b} and cancer antigen peptide B have the same meanings as defined above in relation to the formula (2).

"Cancer antigen peptide C" is an MHC class I-restricted peptide consisting of 7 to 30 amino acid residues including one cysteine residue, or an MHC class II-restricted peptide consisting of 7 to 30 amino acid residues including one cysteine residue. When R¹ is the cancer antigen peptide C, the sulfur atom of the cysteine residue of the cancer antigen peptide C binds to the sulfur atom in the formula (1) via a disulfide bond. To the N-terminus of the cancer antigen peptide C, a peptide consisting of 1 to 4 amino acid residues (i.e., one amino acid residue or a peptide consisting of 2 to 4 amino acid residues) may bind.

Cancer antigen peptide C includes at least one cysteine residue in its amino acid sequence. The number of cysteine residue(s) is preferably 1 to 3, more preferably 1 to 2, or most preferably one.

In an embodiment, the peptide consisting of 1 to 4 amino acid residues and binding to the N-terminus of the cancer antigen peptide C consists of 1 to 4 amino acid residues independently selected from an alanine residue, an arginine residue, an asparagine residue, a cysteine residue, a glutamine residue, a glutamic acid residue, a histidine residue, an isoleucine residue, a leucine residue, a lysine residue, a methionine residue, a phenylalanine residue, a proline residue, a serine residue, a threonine residue, a tryptophan residue, a tyrosine residue and a valine residue. In a further embodiment, the peptide consisting of 1 to 4 amino acid residues consists of 1 to 4 amino acid residues independently selected from an arginine residue, a glutamine residue, a glutamic acid residue, a histidine residue, a lysine residue, a phenylalanine residue and a tyrosine residue.

In an embodiment, the peptide consisting of 1 to 4 amino acid residues and binding to the N-terminus of cancer antigen peptide C includes one cysteine residue. In a further embodiment, a dipeptide consisting of CA binds to the N-terminus of cancer antigen peptide C.

In an embodiment, one amino acid residue selected from an arginine residue, a glutamine residue, a glutamic acid residue, a histidine residue, a lysine residue, a phenylalanine residue and a tyrosine residue binds to the N-terminus of cancer antigen peptide C.

In an embodiment, the compound represented by the formula (1) is not a homodimer but a heterodimer. In contrast to a homodimer, which means a dimerized product of the same peptide monomers, a heterodimer means a dimerized product of different peptide monomers.

When R¹ is the group represented by the formula (2) and the cancer antigen peptide B includes one cysteine residue, the sulfur atom of the cysteine residue of the cancer antigen peptide B may bind, via a disulfide bond, to
a sulfur atom in the formula (3): 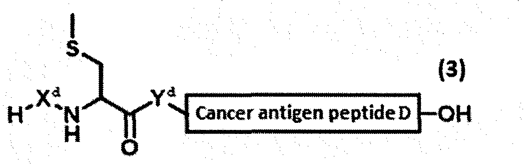 wherein X^{d} and Y^{d} each independently represent a single bond or a divalent peptide group consisting of 1 to 4 amino acid residues, provided that the sum of the numbers of amino acid residues in X^{d} and Y^{d} is an integer of 0 to 4, and cancer antigen peptide D is an MHC class II-restricted peptide consisting of 7 to 30 amino acid residues, wherein an amino group of an N-terminal amino acid of the cancer antigen peptide D binds to Y^{d} in the formula (3), and a carbonyl group of a C-terminal amino acid of the cancer antigen peptide D binds to the hydroxyl group in the formula (3),
or a sulfur atom of a cysteine residue of cancer antigen peptide E, wherein the cancer antigen peptide E is an MHC class II-restricted peptide consisting of 7 to 30 amino acid residues including one cysteine residue.

When R¹ is the cancer antigen peptide C and a peptide consisting of 1 to 4 amino acid residues including one cysteine residue binds to the N-terminus of the cancer antigen peptide C, a sulfur atom of the cysteine residue of the peptide binding to the N-terminus of the cancer antigen peptide C may bind, via a disulfide bond, to
a sulfur atom in the formula (3): 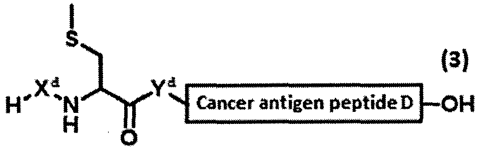 wherein X^{d} and Y^{d} each independently represent a single bond or a divalent peptide group consisting of 1 to 4 amino acid residues, provided that the sum of the numbers of amino acid residues in X^{d} and Y^{d} is an integer of 0 to 4; and cancer antigen peptide D is an MHC class II-restricted peptide consisting of 7 to 30 amino acid residues, wherein an amino group of an N-terminal amino acid of the cancer antigen peptide D binds to Y^{d} in the formula (3), and a carbonyl group of an C-terminal amino acid of the cancer antigen peptide D binds to the hydroxyl group in the formula (3),
or a sulfur atom of a cysteine residue of cancer antigen peptide E, wherein the cancer antigen peptide E is an MHC class II-restricted peptide consisting of 7 to 30 amino acid residues including one cysteine residue.

"Cancer antigen peptide D" is an MHC class II-restricted peptide consisting of 7 to 30 amino acid residues. In the formula (3), the amino group of the N-terminal amino acid of the cancer antigen peptide D binds to Y^{d} in the formula (3) and the carbonyl group of the C-terminal amino acid of the cancer antigen peptide D binds to the hydroxyl group in the formula (3).

"Cancer antigen peptide E" is an MHC class II-restricted peptide consisting of 7 to 30 amino acid residues including one cysteine residue.

"X^{d}" and "Y^{d}" each independently represent a single bond or a divalent peptide group consisting of 1 to 4 amino acid residues. The sum of the numbers of amino acid residues in X^{d} and Y^{d} is an integer of 0 to 4. For example, when the sum of the number of amino acid residues in X^{d} and Y^{d} is an integer of 0, both X^{d} and Y^{d} are single bonds. When the sum of the number of amino acid residues in X^{d} and Y^{d} is an integer of 4, each of X^{d} and Y^{d} may be a divalent peptide group consisting of two amino acid residues; X^{d} may be a divalent peptide group consisting of three amino acid residues and Y^{d} may be a divalent peptide group consisting of one amino acid residue; or X^{d} may be a divalent peptide group consisting of four amino acid residues and Y^{d} may be a single bond.

The sum of the number of amino acid residues in X^{d} and Y^{d} is preferably an integer of 0 to 2, more preferably an integer of 0 to 1, or most preferably zero. That is, most preferably, X^{d} and Y^{d} are both single bonds.

When the sum of the number of amino acid residues in X^{d} and Y^{d} is an integer of 2, X^{d} may be a divalent peptide group consisting of two amino acid residues and Y^{d} may be a single bond; each of X^{d} and Y^{d} may independently be a divalent peptide group consisting of one amino acid residue; or X^{d} may be a single bond and Y^{d} may be a divalent peptide group consisting of two amino acid residues.

When the sum of the number of amino acid residues in X^{d} and Y^{d} is an integer of 1, X^{d} may be a divalent peptide group consisting of one amino acid residue and Y^{d} may be a single bond; or X^{d} may be a single bond and Y^{d} may be a divalent peptide group consisting of one amino acid residue. In a preferred embodiment, X^{d} is a single bond and Y^{d} is a residue of alanine, leucine or methionine, or X^{d} is a residue of alanine, leucine or methionine, and Y^{d} is a single bond.

The "MHC class I-restricted peptide" and "MHC class II-restricted peptide" herein can be a peptide consisting of contiguous amino acid residues in the amino acid sequence of human WT1 of SEQ ID NO: 1 or an altered peptide thereof (herein also referred to as "WT1 peptide").

The term "MHC class I-restricted" means an ability of a peptide to bind to a Major Histocompatibility Complex (MHC) class I molecule and induce CTLs. The term "MHC class I-restricted peptide" herein refers to a peptide being capable of binding to an MHC class I molecule and inducing cytotoxic T cells (CTLs) *in vitro* and/or *in vivo* (that is, a peptide having an ability to induce CTLs). The term "MHC class I-restricted peptide" is also referred to as a "killer peptide".

MHC is called as a human leukocyte-type antigen (HLA) in humans. HLA molecules corresponding to MHC class I-molecules are classified into subtypes such as HLA-A, B, Cw, F and G. Preferable examples of MHC class I restriction include HLA-A, HLA-B and HLA-Cw restriction.

Allelic polymorphism is known for each HLA subtype. Examples of the allelic polymorphism include HLA-A1, HLA-A2, HLA-A24, HLA-A3, HLA-A11, HLA-A33, HLA-B7, HLA-B15, HLA-B27, HLA-B40, HLA-B44, HLA-Cw1, HLA-Cw3, HLA-Cw4, HLA-Cw6 as well as HLA-A*01:01, HLA-A*02:01, HLA-A^{∗}02:06, HLA-A*02:07, HLA-A*24:02, HLA-A*03:01, HLA-A^{∗}11:01, HLA-A*33:01, HLA-A*33:03, HLA-B*15:01, HLA-B*27:05, HLA-B*40:01, HLA-B^{∗}40:02, HLA-B*40:03, HLA-B*40:06, HLA-B*44:03, HLA-Cw*01:01, HLA-Cw*03:01, HLA-Cw*04:0, HLA-Cw*06:02.

The "MHC class I-restricted WT1 peptide" may consists of a sequence of any number of amino acids of any type. However, the longer a peptide chain is, the more susceptible it may be to degradation by a proteolytic enzyme. Also, too small peptide may not successfully be caught in a peptide-binding groove of an MHC class I molecule. The "MHC class I-restricted peptide" typically consists of 7 to 30, preferably 7 to 15, 8 to 12, 8 to 11, or 8 or 9 amino acid residues. In an embodiment, the MHC class I-restricted peptide consists of 9 to 30, preferably, 9 to 15, 9 to 12, 9 to 11, 9 to 10, or 9 amino acid residues.

The term "MHC class I-restricted peptide" includes a peptide that provides an "MHC class I-restricted epitope" through a process such as degradation with proteasome and/or protease, and/or cutting (also referred to as trimming) by ERAP1 to an appropriate peptide length *in vitro* or *in vivo.* The term "MHC class I-restricted epitope" refers to a peptide corresponding to an actual peptide complexed with an MHC class I molecule and presented. That is, the term "MHC class I-restricted WT1 peptide" includes a peptide that produces a peptide being capable of binding to an MHC class I molecule through a process such as degradation or trimming *in vitro* or *in vivo* (that is, a peptide that binds to an MHC class I molecule after a process such as degradation or trimming *in vitro* or *in vivo*).

An "MHC class I-restricted epitope" may be derived from an "MHC class I-restricted peptide" by degradation with proteasome and/or protease, followed by trimming (cutting) by ERAP1, wherein a C-terminal amino acid residue and an N-terminal amino acid residue of the "MHC class I-restricted epitope" may be determined by the action of proteasome/protease and ERAP1, respectively. Accordingly, the "MHC class I-restricted peptide" may be a peptide consisting of 7 to 30 amino acid residues wherein 0 to 23 amino acid residues are attached to a "MHC class I-restricted epitope" consisting of 7 to 12 residues, via its C-terminal carbonyl group.

An "MHC class I restrictive epitope" typically consists of 7 to 12 amino acid residues and preferably 9 amino acid residues. In an embodiment, an "MHC class I-restricted peptide" is a peptide comprising an "MHC class I-restricted epitope" and an amino acid residue(s) attached to its C-terminus. In a further embodiment, the "MHC class I-restricted WT1 peptide" is a peptide consisting of 8 to 30 amino acid residues wherein 1 to 23 amino acid residues are attached to an "MHC class I-restricted epitope" via its C-terminal carbonyl group.

As used herein, a peptide comprising a given amino acid sequence refers to a peptide having the given amino acid sequence which may, as usually understood, optionally have extra sequence(s) of amino acid residue(s) attached to the N-terminal and/or C-terminal amino acid of the given sequence. An "MHC class I-restricted peptide" may have an extra sequence of amino acid residue(s) attached preferably to its C-terminus.

As used herein, the term "altered peptide" refers to a peptide consisting of an amino acid sequence which has one or several amino acid residues altered in the amino acid sequence of the original peptide. In an altered peptide, one or several amino acid residues, for example, 1 to 9 amino acid residues, preferably 1 to 5, 1 to 4 or 1 to 3 amino acid residues, more preferably 1 to 2 amino acid residues, or most preferably one amino acid residue is deleted from, substituted in, and/or added (or inserted) to the amino acid sequence of the original peptide. The number of amino acid(s) deleted from, substituted in, and/or added (or inserted) to the amino acid sequence of the original peptide may preferably be 1 to 5, 1 to 4, or 1 to 3, more preferably 1 to 2, or most preferably one. Amino acid substitution for altering a peptide may be made at any position of amino acid residue in the original sequence with any type of amino acid. Conservative amino acid substitution is preferred. For example, substitution of Asp for Glu; Tyr for Phe; Ile for Leu; Ser for Ala; or Arg for His may be made. Amino acid addition or deletion may preferably be made at N- or C-terminus of a peptide. However, amino acid addition or deletion may be made internally. Amino acid substitution or addition may be made with any of the twenty genetically encoded amino acids or even any non-natural amino acid.

As used herein, a killer peptide consisting of an altered amino acid sequence is also referred to as an "altered killer peptide". When a killer peptide consisting of an amino acid sequence of nine amino acid residues is altered by amino acid substitution, the substitution may be made at position 1 (N-terminus), 2, 3 or 9. When an altered killer peptide has added amino acid residue(s), the number of added amino acid(s) is preferably 1 or 2, or more preferably one. Amino acid addition is preferably made to N-terminus or C-terminus. When a killer peptide is altered by amino acid deletion, the number of deleted amino acid(s) is preferably one.

Peptides that can complex with a polymorphic sequence of an HLA antigen have a specific pattern of amino acid sequence (that is, binding motif) for binding to the polymorphic sequence of the HLA antigen. Amino acid substitution may be made at any of amino acid residues constituting such a binding motif. For example, an HLA-A24-binding peptide that consists of 8 to 11 amino acid residues is known to have Tyr, Phe, Met or Trp at position 2, and Phe, Leu, Ile, Trp or Met at the C-terminus (J. Immunol., 152, p3913, 1994; J. Immunol., 155, p4307, 1994; Immunogenetics, 41, p178, 1995). Therefore, for example, a peptide consisting of nine amino acid residues may be altered by amino acid substitution to have Tyr, Phe, Met or Trp at position 2, and/or Phe, Leu, Ile, Trp or Met at position 9 (C-terminus) to give an altered peptide useful as an altered killer peptide. Also, an HLA-A0201-binding peptide that consists of 8 to 11 amino acid residues is known to have Leu or Met at position 2, and Val or Leu at the C-terminus. Therefore, for example, a peptide consisting of nine amino acid residues may be altered by amino acid substitution to have Leu or Met at position 2, and/or Val or Leu at position 9 (C-terminus) to give an altered peptide useful as an altered killer peptide.

Examples of the "MHC class I-restricted peptide" include a peptide comprising an amino acid sequence selected from:
RMFPNAPYL (SEQ ID NO: 2),
CMTWNQMNL (SEQ ID NO: 3),
ALLPAVPSL (SEQ ID NO: 4),
SLGEQQYSV (SEQ ID NO: 5),
RVPGVAPTL (SEQ ID NO: 6) and
VLDFAPPGA (SEQ ID NO: 7),
or a peptide comprising an amino acid sequence which has one or several amino acid residues altered in the amino acid sequence selected from SEQ ID NOs: 2 to 7 and having an ability to induce CTLs. Further preferably, a peptide consisting of an amino acid sequence selected from SEQ ID NOs: 2 to 7 or a peptide consisting of an amino acid sequence which has one or several amino acid residues altered in the amino acid sequence selected from SEQ ID NOs: 2 to 7 and having an ability to induce CTLs.

Examples of the altered killer peptide include the following peptides:
altered killer peptides of RMFPNAPYL (SEQ ID NO: 2), such as
RYFPNAPYL (SEQ ID NO: 8) (see, WO03/106682);
FMFPNAPYL (SEQ ID NO: 9),
RLFPNAPYL (SEQ ID NO: 10),
RMMPNAPYL (SEQ ID NO: 11),
RMFPNAPYV (SEQ ID NO: 12) or
YMFPNAPYL (SEQ ID NO: 13) (see, WO2009/072610);
altered killer peptides of CMTWNQMNL (SEQ ID NO: 3), such as
CYTWNQMNL (SEQ ID NO: 14) (see WO02/79253);
Xaa-Met-Thr-Trp-Asn-Gln-Met-Asn-Leu (SEQ ID NO: 15) (wherein Xaa is Ser or Ala) or
Xaa-Tyr-Thr-Trp-Asn-Gln-Met-Asn-Leu (SEQ ID NO: 16) (wherein Xaa is Ser, Ala, Abu, Arg, Lys, Orn, Cit, Leu, Phe or Asn) (see, WO2004/026897);
altered killer peptides of ALLPAVPSL (SEQ ID NO: 4) such as AYLPAVPSL (SEQ ID NO: 17) (see, WO2003/106682);
altered killer peptides of SLGEQQYSV (SEQ ID NO: 5) such as
FLGEQQYSV (SEQ ID NO: 18),
SMGEQQYSV (SEQ ID NO: 19), or
SLMEQQYSV (SEQ ID NO: 20) (see, WO2009/072610); or
altered killer peptides of RVPGVAPTL (SEQ ID NO: 6) such as RYPGVAPTL (SEQ ID NO: 21) (see, WO2003/106682).

In an embodiment, an altered killer peptide is an altered peptide wherein 1 to 4 amino acid residues are attached to the N-terminus of a killer peptide. In an embodiment, the amino acid(s) to be added is independently selected from an alanine residue, an arginine residue, an asparagine residue, a cysteine residue, a glutamine residue, a glutamic acid residue, a histidine residue, an isoleucine residue, a leucine residue, a lysine residue, a methionine residue, a phenylalanine residue, a proline residue, a serine residue, a threonine residue, a tryptophan residue, a tyrosine residue, and a valine residue, preferably from an arginine residue, a glutamine residue, a glutamic acid residue, a histidine residue, a lysine residue, a phenylalanine residue, and a tyrosine residue. Preferably 1 to 3 amino acid residues, further preferably 1 to 2 amino acid residues, and most preferably 1 amino acid residue are added.

Examples of the altered killer peptide include a peptide consisting of 10 to 12 amino acids that comprises the amino acid sequence of SEQ ID NO: 3 or SEQ ID NO: 14 and 1 to 3 amino acid residues attached to the amino group of the N-terminal cysteine residue of the sequence.

In an embodiment, an altered killer peptide is a peptide comprising or consisting of an amino acid sequence selected from
RCMTWNQMNL (SEQ ID NO: 22),
RCYTWNQMNL (SEQ ID NO: 23),
QCMTWNQMNL (SEQ ID NO: 24),
QCYTWNQMNL (SEQ ID NO: 25),
ECMTWNQMNL (SEQ ID NO: 26),
ECYTWNQMNL (SEQ ID NO: 27),
HCMTWNQMNL (SEQ ID NO: 28),
HCYTWNQMNL (SEQ ID NO: 29),
KCMTWNQMNL (SEQ ID NO: 30),
KCYTWNQMNL (SEQ ID NO: 31),
FCMTWNQMNL (SEQ ID NO: 32),
FCYTWNQMNL (SEQ ID NO: 33),
YCMTWNQMNL (SEQ ID NO: 34) and
YCYTWNQMNL (SEQ ID NO: 35).

In an embodiment, an "MHC class I-restricted peptide" is a peptide comprising an amino acid sequence selected from
RMFPNAPYL (SEQ ID NO: 2),
CMTWNQMNL (SEQ ID NO: 3),
ALLPAVPSL (SEQ ID NO: 4),
SLGEQQYSV (SEQ ID NO: 5),
RVPGVAPTL (SEQ ID NO: 6) and
VLDFAPPGA (SEQ ID NO: 7)
or a peptide comprising an amino acid sequence which has one or several amino acids deleted from, substituted in, and/or added to the amino acid sequence selected from SEQ ID NOs: 2 to 7 and having an ability to induce CTLs.

In a further embodiment, an "MHC class I-restricted peptide" is a peptide consisting of an amino acid sequence selected from
RMFPNAPYL (SEQ ID NO: 2),
CMTWNQMNL (SEQ ID NO: 3),
ALLPAVPSL (SEQ ID NO: 4),
SLGEQQYSV (SEQ ID NO: 5),
RVPGVAPTL (SEQ ID NO: 6) and
VLDFAPPGA (SEQ ID NO: 7)
or a peptide consisting of an amino acid sequence which has one or several amino acids deleted from, substituted in, and/or added to the amino acid sequence selected from SEQ ID NOs: 2 to 7 and having an ability to induce CTLs.

In a further embodiment, an "MHC class I-restricted peptide" is a peptide comprising an amino acid sequence selected from
RMFPNAPYL (SEQ ID NO: 2),
CMTWNQMNL (SEQ ID NO: 3) and
VLDFAPPGA (SEQ ID NO: 7), or
a peptide comprising an amino acid sequence which has one or several amino acids deleted from, substituted in, and/or added to the amino acid sequence selected from SEQ ID NOs: 2, 3, and 7 and having an ability to induce CTLs.

In a further embodiment, an "MHC class I-restricted peptide" is a peptide consisting of an amino acid sequence selected from
RMFPNAPYL (SEQ ID NO: 2),
CMTWNQMNL (SEQ ID NO: 3) and
VLDFAPPGA (SEQ ID NO: 7), or
a peptide consisting of an amino acid sequence which has one or several amino acids deleted from, substituted in, and/or added to the amino acid sequence selected from SEQ ID NOs: 2, 3, and 7 and having an ability to induce CTLs.

In a further embodiment, an "MHC class I-restricted peptide" is a peptide comprising or consisting of an amino acid sequence selected from
RMFPNAPYL (SEQ ID NO: 2),
RYFPNAPYL (SEQ ID NO: 8),
YMFPNAPYL (SEQ ID NO: 13),
CMTWNQMNL (SEQ ID NO: 3),
CYTWNQMNL (SEQ ID NO: 14) and
VLDFAPPGA (SEQ ID NO: 7).

In a further embodiment, an "MHC class I-restricted peptide" is a peptide comprising or consisting of an amino acid sequence of RMFPNAPYL (SEQ ID NO: 2), RYFPNAPYL (SEQ ID NO: 8) or YMFPNAPYL (SEQ ID NO: 13).

In a further embodiment, an "MHC class I-restricted peptide" is a peptide comprising or consisting of an amino acid sequence of RMFPNAPYL (SEQ ID NO: 2), VLDFAPPGA (SEQ ID NO: 7) or YMFPNAPYL (SEQ ID NO: 13).

In a further embodiment, an "MHC class I-restricted peptide" is a peptide comprising or consisting of an amino acid sequence of RMFPNAPYL (SEQ ID NO: 2).

In a further embodiment, an "MHC class I-restricted peptide" is a peptide consisting of an amino acid sequence selected from
WAPVLDFAPPGASAYGSL (SEQ ID NO: 36),
CWAPVLDFAPPGASAYGSL (SEQ ID NO: 37),
WAPVLDFAPPGASAYGSLC (SEQ ID NO: 38),
SGQARMFPNAPYLPSC (SEQ ID NO: 39),
SGQAYMFPNAPYLPSC (SEQ ID NO: 40),
SGQARMFPNAPYLPSCLES (SEQ ID NO: 41) and
SGQAYMFPNAPYLPSCLES (SEQ ID NO: 42).

In a further embodiment, an "MHC class I-restricted peptide" is a peptide consisting of an amino acid sequence selected from
WAPVLDFAPPGASAYGSL (SEQ ID NO: 36),
CWAPVLDFAPPGASAYGSL (SEQ ID NO: 37) and
WAPVLDFAPPGASAYGSLC (SEQ ID NO: 38).

The term "MHC class II-restricted" means an ability of a peptide to bind to an MHC class II molecule to induce helper T cells. The "MHC class II-restricted peptide" refers to a peptide being capable of binding to an MHC class II molecule and inducing helper T cells *in vitro* and/or *in vivo* (that is, a peptide having an ability to induce helper T cells.). The "MHC class II-restricted peptide" is also referred herein to as a "helper peptide".

HLA molecules corresponding to MHC class II-molecules are classified into subtypes such as HLA-DR, DQ and DP. Preferable examples of "MHC class II-restriction" include HLA-DR restriction, HLA-DQ restriction or HLA-DP restriction.

Allelic polymorphism is known for each HLA subtype. Examples of the allelic polymorphism include DRB1*0101, DRB1*0405, DRB1*0802, DRB1*0803, DRB1^{∗}0901, DRB1*1201, DRB1*1403, DRB1*1501, DRB1*1502, DPB1^{∗}0201, DPB1^{∗}0202, DPB1^{∗}0402, DPB1^{∗}0501, DPB1*0901, DQB1*0301, DQB1*0302, DQB1*0401, DQB1*0501, DQB1*0601, DQB1^{∗}0602 and DRB5*0102. Preferably, DRB1*0101, DRB1*0405, DRB1*1502, DPB1^{∗}0201, DPB1^{∗}0202 or DQB1*0601 is mentioned.

The "MHC class II-restricted peptide" may consists of a sequence of any number of amino acids of any type. However, the longer a peptide chain is, the more susceptible it may be to degradation by a proteolytic enzyme. Also, too small peptide may not successfully be caught in a peptide-binding groove of an MHC class II molecule. The "MHC class II-restricted peptide" typically consists of 9 to 30 amino acid residues, preferably 10 to 25 amino acid residues, more preferably 12 to 24 amino acid residues, or still more preferably 15 to 22 amino acid residues.

The "MHC class II-restricted peptide" includes such a peptide that may be degraded by proteasome and/or protease, and/or be cut or trimmed with ERAP1 to an appropriate peptide length, to provide an "MHC class II-restricted epitope". The term "MHC class II-restricted epitope" refers to a peptide corresponding to an actual peptide complexed with an MHC class II molecule and presented. That is, the term "MHC class II-restricted peptide" includes a peptide that produces a peptide being capable of binding to an MHC class II molecule through a process such as degradation and/or trimming *in vitro* or *in vivo* (that is, a peptide that binds to an MHC class II molecule after a process such as degradation or trimming *in vitro* or *in vivo*)*.*

As used herein, a helper peptide consisting of an altered amino acid sequence herein is also referred to as an "altered helper peptide". When a helper peptide consisting of an amino acid sequence of nine amino acid residues including a binding motif to HLA-DRB1*0405 is altered by amino acid substitution, the substitution may preferably be made at positions 1, 4, 6 and/or 9. More preferably, a helper peptide consisting of a sequence of nine amino acid residues including a binding motif to HLA-DRB1*0405 may be altered by amino acid substitution to have an amino acid residue(s) selected from:
phenylalanine, tryptophan, valine, isoleucine, leucine or methionine at position 1;
valine, isoleucine, methionine, aspartic acid and glutamic acid at position 4;
asparagine, serine, threonine, glutamine, lysine and aspartic acid at position 6; and
aspartic acid, glutamic acid and glutamine at position 9.

Examples of the "MHC class II-restricted peptide" include a peptide comprising an amino acid sequence selected from:
WAPVLDFAPPGASAYGSL (SEQ ID NO: 36),
CWAPVLDFAPPGASAYGSL (SEQ ID NO: 37),
WAPVLDFAPPGASAYGSLC (SEQ ID NO: 38),
SGQARMFPNAPYLPSC (SEQ ID NO: 39),
SGQAYMFPNAPYLPSC (SEQ ID NO: 40),
SGQARMFPNAPYLPSCLES (SEQ ID NO: 41),
SGQAYMFPNAPYLPSCLES (SEQ ID NO: 42),
PGCNKRYFKLSHLQMHSRK (SEQ ID NO: 43),
PGCNKRYFKLSHLQMHSRKH (SEQ ID NO: 44),
PGCNKRYFKLSHLQMHSRKHTG (SEQ ID NO: 45),
CNKRYFKLSHLQMHSRK (SEQ ID NO: 46),
CNKRYFKLSHLQMHSRKH (SEQ ID NO: 47) and
CNKRYFKLSHLQMHSRKHTG (SEQ ID NO: 48), or
a peptide comprising an amino acid sequence which has one or several amino acid residues altered in the amino acid sequence selected from SEQ ID NOs: 36 to 48 and having an ability to induce helper T cell. Further preferably, examples of the "MHC class II-restricted peptide" include a peptide consisting of an amino acid sequence selected from SEQ ID NOs: 36 to 48 and a peptide consisting of an amino acid sequence which has one or several amino acid residues altered in the amino acid sequence selected from SEQ ID NOs: 36 to 48 and having an ability to induce helper T cell.

In an embodiment, R¹ represents cancer antigen peptide C, and the cancer antigen peptide C is a peptide consisting of an amino acid sequence selected from the following amino acid sequences:
CMTWNQMNL (SEQ ID NO: 3),
CYTWNQMNL (SEQ ID NO: 14),
RCMTWNQMNL (SEQ ID NO: 22),
RCYTWNQMNL (SEQ ID NO: 23),
QCMTWNQMNL (SEQ ID NO: 24),
QCYTWNQMNL (SEQ ID NO: 25),
ECMTWNQMNL (SEQ ID NO: 26),
ECYTWNQMNL (SEQ ID NO: 27),
HCMTWNQMNL (SEQ ID NO: 28),
HCYTWNQMNL (SEQ ID NO: 29),
KCMTWNQMNL (SEQ ID NO: 30),
KCYTWNQMNL (SEQ ID NO: 31),
FCMTWNQMNL (SEQ ID NO: 32),
FCYTWNQMNL (SEQ ID NO: 33),
YCMTWNQMNL (SEQ ID NO: 34) and
YCYTWNQMNL (SEQ ID NO: 35).

Examples of the compound represented by the formula (1) include
a compound of formula (6): 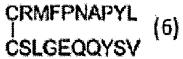 wherein C-C shown in the formula means that the C residues are linked together by a disulfide bond,
a compound of formula (4): 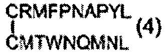 wherein C-C shown in the formula means that the C residues are linked together by a disulfide bond,
a compound of formula (5): 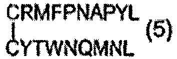 wherein C-C shown in the formula means that the C residues are linked together by a disulfide bond,
a compound of formula (7): 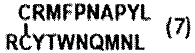 wherein C-C shown in the formula means that the C residues are linked together by a disulfide bond,
a compound of formula (8): 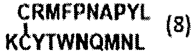 wherein C-C shown in the formula means that the C residues are linked together by a disulfide bond,
a compound of formula (9): 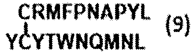 wherein C-C shown in the formula means that the C residues are linked together by a disulfide bond,
a compound of formula (10): 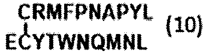 wherein C-C shown in the formula means that the C residues are linked together by a disulfide bond,
a compound of formula (11): 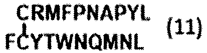 wherein C-C shown in the formula means that the C residues are linked together by a disulfide bond,
a compound of formula (12): 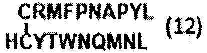 wherein C-C shown in the formula means that the C residues are linked together by a disulfide bond,
a compound of formula (13): 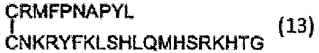 wherein C-C shown in the formula means that the C residues are linked together by a disulfide bond,
a compound of formula (14): 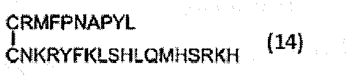 wherein C-C shown in the formula means that the C residues are linked together by a disulfide bond,
a compound of formula (15): 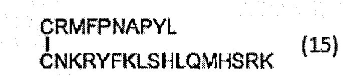 wherein C-C shown in the formula means that the C residues are linked together by a disulfide bond,
a compound of formula (16): 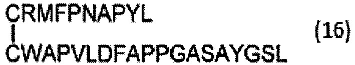 wherein C-C shown in the formula means that the C residues are linked together by a disulfide bond,
a compound of formula (17): 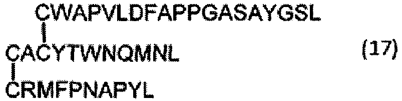 wherein C-C shown in the formula means that the C residues are linked together by a disulfide bond,
a compound of formula (18): 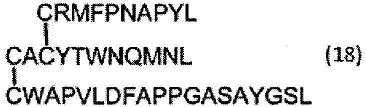 wherein C-C shown in the formula means that the C residues are linked together by a disulfide bond, and
a compound of formula (19): 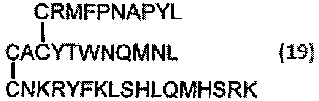 wherein C-C shown in the formula means that the C residues are linked together by a disulfide bond.

Further, the compound represented by the formula (1) can be a peptide comprising or consisting of an amino acid sequence selected from
CRMFPNAPYL (SEQ ID NO: 49),
CCMTWNQMNL (SEQ ID NO: 50),
CCYTWNQMNL (SEQ ID NO: 51),
CALLPAVPSL (SEQ ID NO: 52),
CSLGEQQYSV (SEQ ID NO: 53),
CRVPGVAPTL (SEQ ID NO: 54) and
CVLDFAPPGA (SEQ ID NO: 55).

A peptide may have modification in amino acid residue(s) in its sequence. Modification may be made by a conventional way, for example by esterification, alkylation, halogenation, phosphorylation, sulfonation, or amidation on a functional group in an amino acid residue. Amino acid modification in a peptide can also be addition of any of various moieties to N-terminus and/or C-terminus of a peptide. A peptide may be modified by addition of such a moiety that would modulate solubility of the peptide, stabilize the peptide against, for example, proteolytic degradation, direct the peptide to a specific tissue or organ, or improve capturing of the peptide by antigen presenting cells.

In a peptide, an amino group of its N-terminal amino acid or a carboxyl group of its C-terminal amino acid may be modified. The amino group may be modified, for example by addition of one to three groups selected from C₁₋₆-alkyl, phenyl, cycloalkyl, or acyl such as C₁₋₆-alkanoyl, phenyl-C₁₋₆-alkanoyl, C₅₋₇-cycloalkyl-carbonyl, C₁₋₆-alkylsulfonyl, phenylsulfonyl, C₂₋₆-alkoxy-carbonyl, phenyl-alkoxycarbonyl, C₅₋₇-cycloalkoxy-carbonyl, or phenoxycarbonyl. The carboxyl group of the C-terminal amino acid may be modified, for example by conversion to an ester, such as a C₁₋₆-alkyl ester, a phenyl-C₀₋₆-alkyl ester, or a C₅₋₇-cycloalkyl ester, or to an amide such as a mono- or di-C₁₋₆-alkylamide, a mono- or di-phenyl-C₀₋₆-alkylamide, or a di-substituted amide wherein the two substituents forms together with the nitrogen atom they attach to a 5- to 7-membered azacycloalkane.

In a peptide, a bond between amino acid residues may be peptide bond or other type of bond such as carbon-carbon bond, carbon-nitrogen bond, or carbon-sulfur bond. A peptide as described herein may comprise one or more D-amino acid residues.

The above description about modification in a peptide is illustrative only, and variations thereof would be conceivable to a person skilled in the art. Such a modified peptide would be prepared, tested or used by an ordinarily skilled person in the art.

The ability of a peptide to induce CTLs or helper T cells can be confirmed by a conventional method. Induction of CTLs can be confirmed, for example by counting CTLs by HLA tetramer method (Int. J. Cancer: 100, 565-570 (2002)) or limiting dilution method (Nat. Med.: 4, 321-327 (1998)). Induction of CTLs by an HLA-A24-restricted peptide may also be confirmed by using an HLA-A24 mouse model as described in WO 02/47474 or Int. J. Cancer: 100, 565-570 (2002). Induction of helper T cells can be confirmed, for example by a method as described in Cancer Immunol. Immunother. 51: 271 (2002) or in the Example section herein.

The peptide or compound as described herein, or an intermediate peptide for the synthesis thereof may be synthesized by using a conventional technique for peptide synthesis as described, for example, in Peptide Synthesis, Interscience, New York, 1966; The Proteins, Vol 2, Academic Press Inc., New York, 1976; peptide synthesis, Maruzen Co., LTD., 1975; Basics and Experiment of Peptide Synthesis, Maruzen Co., LTD., 1985; or Development of Pharmaceutical Product subsequent vol. 14, Peptide Synthesis, Hirokawa Shoten, 1991. Examples of such a technique include solid phase synthesis by Fmoc method or Boc method, or liquid phase synthesis by sequential condensation of Boc-amino acid or Z-amino acid in a liquid phase (wherein Fmoc means 9-fluorenylmethoxycarbonyl, Boc means t-butoxycarbonyl, and Z means benzyloxycarbonyl). The peptide may be obtained by a genetic technique by using a nucleotide sequence encoding the peptide in accordance with a conventionally known method as described, for example, in Molecular Cloning, T. Maniatis et al., CSH Laboratory (1983), DNA Cloning, DM. Glover, IRL PRESS (1985).

The compound represented by the formula (1) can be prepared from two different MHC class I-restricted peptides, or from an MHC class I-restricted peptide and an MHC class II-restricted peptide, by linking the peptides by a disulfide bond (WO 2014/157692), for example.

In the course of the synthesis of a compound represented by the formula (1) a functional group on an intermediate compound, such as amino, carboxyl or mercapto may be protected with an appropriate protecting group, or deprotected as needed by a conventional technique. For information about such a protecting group, or a protection or deprotection method, "Protective Groups in Organic Synthesis 2nd Edition (John Wiley & Sons, Inc.; 1990)" may be referred to. As a protecting group for mercapto, an acetamidomethyl group or a trityl group may be useful.

When a compound represented by the formula (1) includes a disulfide bond, the linkage is formed between two different, cysteine-comprising peptide components in the compound, or between a cysteine-comprising peptide component and a cysteine residue in the compound. Such a disulfide bond can be formed by a method as described, for example, in Peptide Synthesis, Interscience, New York, 1966; The Proteins, Vol. 2, Academic Press Inc., New York, 1976; peptide synthesis, Maruzen Co., LTD., 1975; Basics and Experiment of peptide synthesis, Maruzen Co., LTD., 1985; or Development of Pharmaceutical Product sequential vol. 14, Peptide Synthesis, Hirokawa Shoten, 1991.

Specifically, for preparing a compound having a disulfide bond (a disulfide compound) from a peptide having one cysteine residue, the peptide may be subjected to a deprotection reaction for removal of any protecting groups on functional groups including mercapto on the cysteine residue, and then treated in an inert solvent under an oxidative condition for forming a disulfide bond. A disulfide compound may also be prepared from two different, mercapto-having intermediates by treating them in an appropriate solvent under an oxidative condition. Oxidative conditions for disulfide bond formation are known in the field of peptide synthesis. For example, a known method of iodine oxidation, air oxidation under an alkaline condition, or oxidation by an oxidizing agent under an alkaline or acidic condition may be used for forming a disulfide bond. As an oxidizing agent, iodine, dimethyl sulfoxide (DMSO), or potassium ferricyanide may be used. As a solvent for the reaction, water, acetic acid, methanol, chloroform, DMF, or DMSO, or a mixture thereof may be used. Such an oxidative condition often gives a product in the form of a mixture of symmetric and asymmetric disulfide compounds. A desired asymmetric disulfide compound may be recovered or purified by an appropriate chromatographic method or recrystallization. An intermediate having an activated mercapto group, for example a mercapto group bound to an Npys group (3-nitro-2-pyridinesulphenyl group) may be used. For forming a disulfide bond on a given mercapto group on an intermediate, the group may be activated by 2,2'-dithiobis(5-nitropyridine) in advance of coupling with another intermediate (Tetrahedron Letters. Vol. 37. No. 9, pp. 1347-1350).

The methods as described above may be useful for preparing a disulfide compound from a peptide having more than one cysteine residue. In that case, however, a mixture of different disulfide compounds having a disulfide bond between different cysteine residues may be formed. For selectively preparing a product dimerized by a disulfide bond between specific positions of monomers, different protecting groups can be used in combination for protection of functional groups on the cysteine residues. Examples of such combination of protecting groups include a combination of MeBzl (methylbenzyl) and Acm (acetamidomethyl); Trt (trityl) and Acm; Npys (3-nitro-2-pyridylthio) and Acm; and S-Bu-t (S-tert-butyl) and Acm. For example, when a peptide protected with a combination of MeBzl and Acm is used for selective disulfide bond formation, all the MeBzl protecting groups, and the Acm protecting groups on functional groups on amino acid residues other than certain cysteine residues may be removed in the first step. Then, by treating the peptide monomer in a solution under air oxidation condition, a disulfide bond can be formed between selectively deprotected cysteine residues of the monomers. Then, through removal of remaining Acm protecting groups and treatment under oxidative condition with iodine, a further disulfide bond can be formed on the newly deprotected cysteine residues.

The peptide, compound or intermediate synthesized may be purified by any purification method know in the art or in the field of peptide chemistry. Examples of such a purification technique include various types of chromatography (e.g., silica gel column chromatography, ion exchange column chromatography, gel filtration or reversed-phase chromatography), and recrystallization from a solvent, for example an alcohol, such as methanol, ethanol or 2-propanol; an ether, such as diethyl ether; an ester, such as ethyl acetate; an aromatic hydrocarbon, such as benzene or toluene; a ketone, such as acetone; a hydrocarbon, such as hexane; an aprotic solvent, such as dimethylformamide or acetonitrile; water; or a mixture thereof. For further useful purification methods, reference can be made, for example, to Jikken Kagaku Kouza (The Chemical Society of Japan ed., Maruzen) vol. 1. Purification methods for disulfide compounds are also described in Peptide Synthesis, Interscience, New York, 1966; The Proteins, Vol. 2, Academic Press Inc., New York, 1976; peptide synthesis, Maruzen Co., LTD., 1975; Basics and Experiment of peptide synthesis, Maruzen Co., LTD., 1985; or Development of Pharmaceutical Product sequential vol. 14, Peptide Synthesis, Hirokawa Shoten, 1991. Purification by HPLC is preferred.

A compound represented by the formula (1) may have one or more asymmetric centers. Such a compound can be prepared from a starting material (an amino acid) having corresponding asymmetric centers. Also, a compound represented by the formula (1) can be obtained in a high optical purity by inclusion of an optical resolution step in a process for its synthesis. For example, in accordance with a diastereomer method for optical resolution, a compound represented by the formula (1) or an intermediate product can be treated with an optically active acid (e.g., a monocarboxylic acid such as mandelic acid, N-benzyloxyalanine, or lactic acid, a dicarboxylic acid such as tartaric acid, o-diisopropylidenetartaric acid, or malic acid, or a sulfonic acid such as camphorsulfonic acid or bromocamphorsulfonic acid) in an inert solvent (e.g., an alcohol such as methanol, ethanol, or 2-propanol, an ether such as diethyl ether, an ester such as ethyl acetate, a hydrocarbon such as toluene, an aprotic solvent such as acetonitrile, or a mixture thereof) to form salts. For optically resolving a compound represented by the formula (1) or an intermediate having an acidic functional group such as carboxyl, its salts can be formed with an optically active amine (e.g., an organic amine such as α-phenethylamine, kinin, quinidine, cinchonidine, cinchonine, or strychnine).

The salts may be formed at a temperature in the range from room temperature up to the boiling point of a solvent used. For obtaining a product in a highly optically pure form, it may be desirable to raise the temperature to around the boiling point of the solvent for a period of time. Salts formed are crystallized, and then filtered, optionally with cooling for an improved yield. An optically active acid or amine used for the salt formation may be used in an amount of about 0.5 to about 2.0 equivalents, preferably about 1 equivalent, relative to the amount of a compound to optically resolve. A crystalline product may optionally be further purified by recrystallization from an inert solvent (e.g., an alcohol such as methanol, ethanol, or 2-propanol, an ether such as diethyl ether, an ester such as ethyl acetate, a hydrocarbon solvent such as toluene, an aprotic solvent such as acetonitrile, or a mixture thereof). A product recovered in the form of a salt may optionally be converted to a free base or acid by treatment with an acid or base.

The term "pharmaceutically acceptable salt" as used herein includes an acid addition salt and a base addition salt. The acid addition salt may be an inorganic acid salt, such as hydrochloride, hydrobromide, sulfate, hydroiodide, nitrate, or phosphate, or an organic acid salt such as citrate, oxalate, acetate, formate, propionate, benzoate, trifluoroacetate, maleate, tartrate, methanesulfonate, benzenesulfonate, or p-toluenesulfonate. The base addition salt may be a salt with an inorganic base, such as sodium salt, potassium salt, calcium salt, magnesium salt, or ammonium salt, a salt with an organic base, such as triethylammonium salt, triethanolammonium salt, pyridinium salt, or diisopropylammonium salt. The "pharmaceutically acceptable salt" also includes a salt with a basic or acidic amino acid, such as arginine, aspartic acid, or glutamic acid. The term "peptide" or "compound" used herein includes a peptide or compound in the form of a pharmaceutically acceptable salt, unless the context requires otherwise.

The present disclosure further includes a hydrate or a solvate such as an ethanol solvate of the peptide of (a) or the compound represented by the formula (1) or the peptide or a pharmaceutically acceptable salt thereof as described herein. The present disclosure also includes any stereoisomer such as a diastereomer or an enantiomer, and any crystalline form of the compound or the peptide as described herein.

The peptide of (a) or the compound represented by the formula (1) or a pharmaceutically acceptable salt thereof is useful for treating a cancer in an HLA-A^{∗}02:07, HLA-A*03:01, HLA-B*15:01 or HLA-B*27:05-positive subject. Accordingly, the present disclosure provides, in an embodiment, a pharmaceutical composition comprising the peptide of (a) or the compound represented by the formula (1) or a pharmaceutically acceptable salt thereof for treating a cancer in an HLA-A*02:07, HLA-A*03:01, HLA-B*15:01 or HLA-B*27:05-positive subject.

The HLA-A^{∗}02:07-positive subject, HLA-A^{∗}03:01-positive subject, HLA-B^{∗}15:01-positive subject and HLA-B^{∗}27:05-positive subject refer to subjects having HLA haplotypes: HLA-A*02:07, HLA-A*03:01, HLA-B*15:01 and HLA-B*27:05, respectively. The HLA alleles (HLA types) are specified by HLA typing (particularly, genotyping) ordinarily used. Examples of the "subject" include human and non-human animals such as non-human primate, ovine, canine, feline, equine, and bovine. A human subject is preferred.

As used herein, the treating a cancer include completely or partially inhibiting progression of a cancer and at least partially reducing one or more symptoms of a cancer. The treating a cancer also include induction of remission, maintenance of remission and suppression of recurrence.

In an embodiment, the pharmaceutical composition is a cancer vaccine. In another embodiment, the pharmaceutical composition is a composition for inducing CTLs in cellular immunotherapy for a cancer.

In an embodiment, the cancer refers to a cancer in which WT1 is expressed or a cancer associated with an elevated expression level of WT1 gene.

In an embodiment, the cancer refers to a blood cancer or a solid cancer. In a further embodiment, the cancer refers to a cancer selected from chronic or acute leukemia including acute myeloid leukemia, chronic myeloid leukemia, acute lymphocytic leukemia, acute lymphoblastic leukemia and chronic lymphocytic leukemia, myelodysplastic syndrome, multiple myeloma, malignant lymphoma, stomach cancer, colon cancer, lung cancer, breast cancer, germ cell carcinoma, liver cancer, skin cancer, bladder cancer, prostate cancer, uterine cancer, cervical cancer, ovarian cancer, brain tumor, glioma, central nervous system primary malignant lymphoma, Hodgkin lymphoma, non-Hodgkin lymphoma, T cell lymphoma, lymphocytic lymphoma, T cell lymphoma, bone cancer, pancreatic cancer, head and neck cancer, skin or intraorbital malignant melanoma, rectal cancer, anal cancer, testicular cancer, carcinoma of fallopian tube, endometrial carcinoma, cervical carcinoma, vaginal carcinoma, vulvar carcinoma, esophageal cancer, small intestinal cancer, endocrine cancer, thyroid cancer, parathyroid cancer, adrenal cancer, sarcoma of soft tissue, urethral cancer, penile cancer, childhood solid tumor, kidney cancer or ureteral cancer, renal pelvic carcinoma, central nervous system tumor, tumor angiogenesis, spinal tumor, brain stem glioma, pituitary adenoma, Kaposi's sarcoma, epidermoid cancer, squamous cell cancer, environmentally induced cancer including asbestos-induced cancer and combinations of these cancers. In a further embodiment, the cancer is selected from chronic or acute leukemia including acute myeloid leukemia, chronic myeloid leukemia, acute lymphoblastic leukemia and chronic lymphocytic leukemia, myelodysplastic syndrome, multiple myeloma, malignant lymphoma, stomach cancer, colon cancer, lung cancer, breast cancer, germ cell carcinoma, liver cancer, skin cancer, bladder cancer, prostate cancer, uterine cancer, cervical cancer, ovarian cancer, brain tumor and glioma.

The peptide of (a) or the compound represented by the formula (1) or a pharmaceutically acceptable salt thereof or, a pharmaceutical composition comprising the same, may be used in combination with at least one different cancer antigen peptide, in particular, an MHC class I-restricted peptide or an MHC class II-restricted peptide, a conjugate thereof, or a pharmaceutically acceptable salt thereof. Examples of different cancer antigen peptides or conjugates include peptides or derivatives thereof, or conjugates thereof as described in the following publications: WO2000/006602, WO2002/079253, WO2003/106682, WO2004/026897, WO2004/063903, WO2007/063903, WO2010/123065, WO2014/157692, WO2005/053618, WO2007/047764, WO2007/120673, WO2005/045027, WO2010/037395, WO2000/018795, WO2002/028414, WO2003/037060 and WO2004/100870.

In an embodiment, the peptide of (a) or the compound represented by the formula (1) or a pharmaceutically acceptable salt thereof, or a pharmaceutical composition comprising the same, is used in combination with an MHC class II-restricted peptide. In an embodiment, the MHC class II-restricted peptide comprises or consists of the amino acid sequence represented by WAPVLDFAPPGASAYGSL (SEQ ID NO: 36).

The peptide of (a) or the compound represented by the formula (1) or a pharmaceutically acceptable salt thereof, or a pharmaceutical composition comprising the same, may be used in combination with at least one different drug (herein also referred to as coadministration drug).

The coadministration drug may be an "immunomodulator". As used herein, the term "immunomodulator" means any agent that controls transmission of a costimulatory signal generated during T cell activation by antigen-presenting cells by interacting with a molecule that is involved in the transmission of the costimulatory signal and present on the antigen-presenting cells and/or T cells, as well as any agent that directly or indirectly controls function of a molecule involved in establishment of immune tolerance (immunosuppression) in the immune system. Since a cancer antigen peptide is effective for increasing tumor-reactive CTLs in a tumor, it is potentially useful as an agent for coadministration with an immunomodulator, for lowering a necessary dose of an immunomodulator or reducing adverse event caused by an immunomodulator. Thus, the present disclosure provides a therapy having improved efficacy and safety through the use of a cancer antigen peptide in combination with an immunomodulator.

The "immunomodulator" can be an agent in the form of an antibody, a nucleic acid, a protein, a peptide, or a small compound, but is not limited thereto. The "antibody" as the "immunomodulator" includes an antibody fragment. Examples of the antibody fragment include heavy and light chain variable regions of an antibody (VH and VL), F(ab')2, Fab', Fab, Fv, Fd, sdFv, and scFV. The "protein" as the "immunomodulator" means any protein other than antibodies. Examples of the "immunomodulator" include immune checkpoint inhibitors, costimulatory molecule agonists, immune activating agents, and low-molecular inhibitors.

The "immune checkpoint inhibitor" inhibits immunosuppressive effect induced by cancer cells or antigen presenting cells. Examples of the immune checkpoint inhibitor include, but are not limited to, agents against a molecule selected from the group consisting of: (1) CTLA-4 (e.g., ipilimumab and tremelimumab); (2) PD-1 (e.g., nivolumab, pembrolizumab, AMP-224, AMP-514 (MEDI0680), and pidilizumab (CT-011)); (3) LAG-3 (e.g., IMP-321 and BMS-986016); (4) BTLA; (5) KIR (e.g., IPH2101); (6) TIM-3; (7) PD-L1 (e.g., durvalumab (MEDI4736), MPDL3280A, BMS-936559, and avelumab (MSB0010718C)); (8) PD-L2; (9) B7-H3 (e.g., MGA-271); (10) B7-H4; (11) HVEM; (12) GAL9; (13) CD160; (14) VISTA; (15) BTNL2; (16) TIGIT; (17) PVR; (18) BTN1A1; (19) BTN2A2; (20) BTN3A2 (Nat Rev Drug Discov. 2013; 12: 130-146; Nikkei Medical Cancer Review 2014; 9; Nat Rev Immunol. 2014; 14: 559-69); and (21) CSF1-R.

The "costimulatory molecule agonist" enhances T cell activation by transmission of an auxiliary signal via a costimulatory molecule on the T cells and/or antigen presenting cells, and attenuates the immunosuppressive effect of cancer cells or antigen presenting cells. Examples of the costimulatory molecule agonist include, but are not limited to, agents against a molecule selected from the group consisting of: (1) 4-1BB; (2) 4-1BB-L; (3) OX40 (4) OX40-L; (5) GITR; (6) CD28; (7) CD40; (8) CD40-L; (9) ICOS; (10) ICOS-L; (11) LIGHT; and (12) CD27.

The "immune activating agent" efficiently stimulates CTLs in the lymph nodes by directly or indirectly activating immune cells such as T cells and dendritic cells. Examples of the immune activating agent include, but are not limited to, Toll-like receptor (TLR) agonists, stimulator of interferon genes (STING) agonists, cytokines, and agents against heat shock protein (HSP).

Examples of the "Toll-like receptor (TLR) agonist" include, but are not limited to, TLR1/2 agonists, TLR2 agonists, TLR3 agonists (e.g., PolyI:C), TLR4 agonists (e.g., S-type lipopolysaccharide, paclitaxel, lipid A, and monophosphoryl lipid A), TLR5 agonists (e.g., flagellin), TLR6/2 agonists (e.g., MALP-2), TLR7 agonist (e.g., gardiquimod, imiquimod, loxoribine), TLR7/8 agonists (e.g., resiquimod (R848)), TLR7/9 agonists, TLR8 agonists (e.g., motolimod (VTX-2337)), TLR9 agonists (e.g., CpG-ODN), and TLR11 agonists (e.g., profilin).

Examples of the "cytokine" include, but are not limited to, IL-1α, IL-1β, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (INF)-α, INF-β, INF-γ, SCF, GM-CSF, G-CSF, M-CSF, erythropoietin, thrombopoietin, macrophage inflammatory protein (MIP), and monocyte chemoattractant protein (MCP).

Examples of the "heat shock protein (HSP)" include, but are not limited to, HSP70, HSP90, HSP90α, HSP90β, HSP105, HSP72, and HSP40. Agents against a heat shock protein include HSP inhibitors. Examples of inhibitors to HSP90, for example, include, but are not limited to, tanespimycin (17-AAG), luminespib (AUY-922, NVP-AUY922), alvespimycin (17-DMAG) hydrochloride, ganetespib (STA-9090), BIIB021, onalespib (AT13387), geldanamycin, NVP-BEP800, SNX-2112 (PF-04928473), PF-4929113 (SNX-5422), KW-2478, XL888, VER155008, VER-50589, CH5138303, VER-49009, NMS-E973, PU-H71, HSP990 (NVP-HSP990) and KNK437.

Examples of the "low-molecular inhibitor" include, but are not limited to, histone deacetylase inhibitors, histone demethylase inhibitors, histone acetyltransferase inhibitors, histone methyltransferase inhibitors, DNA methyltransferase inhibitors, anthracycline antibiotics, platinum formulations, MAPK inhibitors, β-catenin inhibitors, STATS inhibitors, NF-kB inhibitors, JAK inhibitors, mTOR inhibitors, IDO inhibitors, COX-2 inhibitors, CXCR4 inhibitors, and arginase inhibitors.

Examples of the "histone deacetylase inhibitor" include, but are not limited to, vorinostat (SAHA, MK0683), entinostat (MS-275), panobinostat (LBH589), trichostatin A (TSA), mocetinostat (MGCD0103), BG45, BRD73954, belinostat (PXD101), romidepsin (FK228, depsipeptide), 4SC-202, HPOB, LMK-235, CAY10603, tasquinimod, TMP269, nexturastat A, rocilinostat (ACY-1215), RGFP966, RG2833 (RGFP109), scriptaid, tubastatin A, pracinostat (SB939), CUDC-101, M344, PCI-34051, dacinostat (LAQ824), tubastatin A hydrochloride, abexinostat (PCI-24781), CUDC-907, AR-42, sodium phenylbutyrate, resminostat, tubacin, quisinostat (JNJ-26481585) dihydrochloride, MC1568, givinostat (ITF2357), droxinostat, chidamide (C S055, HBI-8000), CHR-2485, CHR-3996, DAC-060, FRM-0334 (EVP-0334), MGCD-290, CXD-101 (AZD-9468), CG200745, arginine butyrate, sulforaphane, SHP-141, CUDC-907, YM753 (OBP-801), sodium valproate, apicidin, and CI994 (tacedinaline).

Examples of the "histone demethylase inhibitor" include, but are not limited to, GSK J4 HCl, OG-L002, JIB-04, IOX1, SP2509, ORY-1001 (RG-6016), GSK J1, ML324, and GSK-LSD1 2HCl.

Examples of the "histone acetyltransferase inhibitor" include, but are not limited to, C646, MG149, remodelin, and anacardic acid.

Examples of the "histone methyltransferase inhibitor" include, but are not limited to, pinometostat (EPZ5676), EPZ005678, GSK343, BIX01294, tazemetostat (EPZ6438), 3-deazaneplanocin A (DZNeP) HCl, UNC1999, MM-102, SGC0946, entacapone, EPZ015666, UNC0379, EI1, MI-2 (menin-MLL inhibitor), MI-3 (menin-MLL inhibitor), PFI-2, GSK126, EPZ04777, BRD4770, GSK-2816126, and UNC0631.

Examples of the "DNA methyltransferase inhibitor" include, but are not limited to, decitabine, azatidine, RG108, thioguanine, zebularine, SGI-110, CC-486, SGI-1027, lomeguatrib, and procainamide hydrochloride.

The "anthracycline antibiotic" is intercalated between DNA strands to inhibit DNA relaxation. Examples of the anthracycline antibiotic include, but are not limited to, doxorubicin, liposomal doxorubicin, daunorubicin, pirarubicin, epirubicin, idarubicin, aclarubicin, amrubicin, aloin, and mitoxantrone.

Examples of the "platinum formulation" include, but are not limited to, cisplatin, carboplatin, miboplatin, nedaplatin, satraplatin (JM-126), oxaliplatin (ELOXATIN), triplatin tetranitrate, and DDS formulations thereof.

Examples of the "MAPK inhibitor" include, but are not limited to, SB203580, doramapimod (BIRB796), SB202190 (FHPI), LY2228820, VX-702, SB239063, pexmetinib (ARRY-614), PH-797804, VX-745, and TAK-715.

Examples of the "β-catenin inhibitor" include, but are not limited to, XAV-939, ICG-001, IWR-1-endo, Wnt-C59 (C59), LGK-974, KY02111, IWP-2, IWP-L6, WIKI4, and FH535.

Examples of the "STAT3 inhibitor" include, but are not limited to, S3I-201, Stattic, niclosamide, nifuroxazide, napabucasin (BBI608), cryptotanshinone, HO-3867, WHI-P154, FLLL32, STA-21, WP1066, and SH-4-54.

Examples of the "NF-kB inhibitor" include, but are not limited to, QNZ (EVP4593), sodium 4-aminosalicylate, JSH-23, phenethyl caffeate, sodium salicylate, andrographolide, and SC75741.

Examples of the "JAK inhibitor" include, but are not limited to, ruxolitinib (INCB018424), tofacitinib (CP-690550) citrate, AZD1480, fedratinib (SAR302503, TG101348), AT9283, tyrphostin B42 (AG-490), momelotinib (CYT387), tofacitinib (CP-690550, tasocitinib), WP1066, TG101209, gandotinib (LY2784544), NVP-BSK805 2HCl, baricitinib (LY3009104, INCB02850), AZ960, CEP-33779, pacritinib (SB1518), WHI-P154, XL019, S-ruxolitinib (INCB018424), ZM39923 HCl, decernotinib (VX-509), cerdulatinib (PRT062070, PRT2070), filgotinib (GLPG0634), FLLL32, peficitinib (ASP015K, JNJ-54781532), GLPG0634 analogue, Go6976, and Curcumol.

Examples of the "mTOR inhibitor" include, but are not limited to, sirolimus (rapamycin), deforolimus (AP23573, MK-8669), everolimus (RAD-001), temsirolimus (CCI-779, NSC683864), zotarolimus (ABT-578), biolimus A9 (umirolimus), AZD8055, KU-0063794, voxtalisib (XL765, SAR245409), MHY1485, dactolisib (BEZ235, NVP-BEZ235), PI-103, and torkinib (PP242).

Examples of the "IDO inhibitor" include, but are not limited to, NLG919, INCB024360 analog, indoximod (NLG-8189), and epacadostat (INCB024360).

Examples of the "COX-2 inhibitor" include, but are not limited to, valdecoxib, rofecoxib, carprofen, celecoxib, lumiracoxib, tolfenamic acid, nimesulide, niflumic acid, asaraldehyde, lornoxicam, sodium meclofenamate, amfenac sodium hydrate, diclofenac sodium, ketoprofen, ketorolac, naproxen sodium, indomethacin, ibuprofen, aspirin, mefenamic acid, bromfenac sodium, oxaprozin, zaltoprofen, and nepafenac.

Examples of the "CXCR4 inhibitor" include, but are not limited to, WZ811, plerixafor (AMD3100), and plerixafor 8HCl (AMD3100 8HCl).

The coadministration drug may also be one or more drugs selected from the group consisting of "hormone therapy agent", "immunotherapeutic agent", "biopharmaceutical", "cell growth factor", "cell growth factor inhibitor", "cell growth factor receptor inhibitor", "radiotherapeutic agent", "auxiliary agent", and "chemotherapeutic agent". For example, one to five drugs, one to three drugs, or one drug selected from the above group of drugs may be used in combination with the peptide of (a) or the compound represented by the formula (1) or a pharmaceutically acceptable salt thereof, or a pharmaceutical composition comprising the same.

Examples of the "hormone therapy agent" include adrenal cortical hormone agents (e.g., steroidal antiinflammatory agents, estrogen preparations, progesterone preparations, and androgen preparations), anti-estrogen agents, estrogen-controlling agents, estrogen synthesis inhibitors, anti-androgen agents, androgen-controlling agents, androgen synthesis inhibitors, LH-RH agonist preparations, LH-RH antagonist preparations, aromatase inhibitors, steroid-lactonase inhibitors, contraceptive pills, retinoids, and agents that delay metabolism of a retinoid.

Examples of the "hormone therapy agent" include fosfestrol, diethylstilbestrol, fluoxymesterol, chlorotrianisene, methyl testosterone, medroxyprogesterone acetate, megestrol acetate, chlormadinone acetate, cyproterone acetate, danazol, allylestrenol, gestrinone, mepartricin, raloxifene, ormeloxifene, levormeloxifene, tamoxifen citrate, toremifene citrate, iodoxyfene, contraceptive pills, mepitiostane, testololactone, aminoglutethimide, goserelin acetate, buserelin, leuprorelin, leuprolide, droloxifene, epitiostanol, ethinylestradiol sulfonate, estramustine, fadrozole hydrochloride, anastrozole, tetrazole, ketoconazole, letrozole, exemestane, vorozole, formestane, flutamide, bicalutamide, nilutamide, enzalutamide, mifepristone, finasteride, dexamethasone, prednisolone, betamethasone, triamcinolone, abiraterone, liarozole, bexarotene, and DN101.

Examples of the "immunotherapeutic agent" include picibanil, krestin, sizofiran, lentinan, ubenimex, interferon (IFN)-α, interferon (IFN)-β, interferon (IFN)-y, interleukin, macrophage colony stimulating factor, granulocyte-colony stimulating factor, erythropoietin, lymphotoxin, BCG vaccine, *Corynebacterium parvum*, levamisole, polysaccharide K, procodazole, anti-CTLA4 antibody, anti-PD-1 antibody, and TLR agonists (e.g., TLR7 agonists, TLR8 agonists, TLR9 agonists).

Examples of the "biopharmaceutical" include, but are not limited to, interleukin-2 (aldesleukin), interferon-α, interferon-β, interferon-γ, erythropoietin (EPO), granulocyte-colony stimulating factor (filgrastim), granulocyte-macrophage-colony stimulating factor (sargramostim), IL13-PE38QQR, Bacille Calmette-Guerin, levamisole, octreotide, CPG7909, Provenge, GVAX, Myvax, Favld, lenalidomide, trastuzumab, rituximab, gemtuzumab ozogamicin, alemtuzumab, endostatin, ibritumomab tiuxetan, tositumomab, cetuximab, zanolimumab, ofatumumab, HGS-ETR1, pertuzumab, M200, SGN-30, matuzumab, adecatumumab, denosumab, zalutumumab, MDX-060, nimotuzumab, MORAb-003, Vitaxin, MDX-101, MDX-010, DPC4 antibodies, NF-1 antibodies, NF-2 antibodies, Rb antibodies, p53 antibodies, WT1 antibodies, BRCA1 antibodies, BRCA2 antibodies, ganglioside (GM2), prostate specific antigens (PSA), α-fetoprotein (AFP), carcinoembryonic antigens (CEA), melanoma-associated antigens (MART-1, gap100, MAGE 1,3 tyrosine), papilloma virus E6 and E7 fragments, and DDS formulations thereof.

Regarding the "cell growth factor", "cell growth factor inhibitor" and "cell growth factor receptor inhibitor", the cell growth factor may be any agent that promotes cell proliferation. For example, the cell growth factor may be a peptide that has a molecular weight of not more than 20,000 and can bind to a receptor and function at a low concentration.

Examples of the "cell growth factor" include, but are not limited to, epidermal growth factor (EGF), insulin-like growth factor (IGF (e.g., insulin, IGF-1, and IGF-2)), transforming growth factor (TGF (e.g., TGF-α and TGF-β)), nerve growth factor (NGF), brain-derived neurotrophic factor (BDNF), vascular endothelial growth factor (VEGF), colony stimulating factor (CSF (e.g., granulocyte-colony stimulating factor (G-CSF)), granulocyte-macrophage-colony stimulating factor (GM-CSF)), platelet-derived growth factor (PDGF), erythropoietin (EPO), fibroblast growth factor (FGF (e.g., acidic FGF, basic FGF, keratinocyte growth factor (KGK), and FGF-10)), hepatocyte growth factor (HGF), heregulin, and angiopoietin. The term "cell growth factor" is synonymous with the term "growth factor".

Examples of the "cell growth factor inhibitor" include, but are not limited to, epidermal growth factor inhibitors (EGF inhibitors), insulin-like growth factor inhibitors (IGF inhibitors), nerve growth factor inhibitors (NGF inhibitors), brain-derived neurotrophic factor inhibitors (BDNF inhibitors), vascular endothelial cell growth factor inhibitors (VEGF inhibitors), colony stimulating factor inhibitors (CSF inhibitors), platelet-derived growth factor inhibitors (PDGF inhibitors), erythropoietin inhibitors (EPO inhibitors), fibroblast growth factor inhibitors (FGF inhibitors), hepatocyte growth factor inhibitors (HGF inhibitors), heregulin inhibitors, and angiopoietin inhibitors. The term "cell growth factor inhibitor" is synonymous with the term "growth factor inhibitor".

Examples of the "cell growth factor receptor inhibitor" include, but are not limited to, epidermal growth factor receptor inhibitors (EGFR inhibitors), insulin-like growth factor receptor inhibitors (IGFR inhibitors), nerve growth factor receptor inhibitors (NGFR inhibitors), brain-derived neurotrophic factor receptor inhibitors (BDNFR inhibitors), vascular endothelial cell growth factor receptor inhibitors (VEGFR inhibitors), colony stimulating factor receptor inhibitors (CSFR inhibitors), platelet-derived growth factor receptor inhibitors (PDGFR inhibitors), erythropoietin receptor inhibitors (EPOR inhibitors), fibroblast growth factor receptor inhibitors (FGFR inhibitors), hepatocyte growth factor receptor inhibitors (HGFR inhibitors), heregulin receptor inhibitors, and angiopoietin receptor inhibitors. The term "cell growth factor receptor inhibitor" is synonymous with the term "growth factor receptor inhibitor".

Examples of the "radiotherapeutic agent" include, but are not limited to, radioactive materials and radiosensitizers.

The "auxiliary agent" is an agent used together with an anticancer agent for suppressing a side effect or vomiting caused by the anticancer agent. Examples of the "auxiliary agent" include, but are not limited to, aprepitant, ondansetron, lorazepam, dexamethasone, diphenhydramine, ranitidine, cimetidine, ranitidine, famotidine, cimetidine, Procrit, epoetin alfa, filgrastim, oprelvekin, leucovorin, and granulocyte-macrophage-colony stimulating factor (GM-CSF).

Examples of the "chemotherapeutic agent" include, but are not limited to, alkylating agents, platinum formulations, antimetabolites, topoisomerase inhibitors, DNA intercalators, antimitotic agents, antitumor antibiotics, plant-derived anticancer agents, epigenome drugs, immunomodulating drugs, molecular targeted drugs, angiogenesis inhibitors, and other chemotherapeutic agents. Some typical examples of chemotherapeutic agent are listed below.

Examples of the "alkylating agent" include, but are not limited to, nitrogen mustard, nitrogen mustard N-oxide hydrochloride, chlorambucil, cyclophosphamide, ifosfamide, thiotepa, carboquone, improsulfan tosylate, busulfan, nimustine hydrochloride, mitobronitol, melphalan, dacarbazine, procarbazine, ranimustine, estramustine sodium phosphate, triethylenemelamine, carmustine, lomustine, streptozocin, pipobroman, etoglucid, altretamine, ambamustine, dibrospidium hydrochloride, fotemustine, prednimustine, bendamustine, uramustine, semustine, pumitepa, ribomustin, temozolomide, treosulfan, trofosfamide, zinostatin stimalamer, adozelesin, cystemustine, bizelesin, mechlorethamine, uracil mustard, trabectedin, chlormethine, mannosulfan, triaziquone, procarbazine, canfosfamide, nitrosoureas, and DDS formulations thereof.

Examples of the "platinum formulation" include, but are not limited to, cisplatin, carboplatin, miboplatin, nedaplatin, satraplatin, oxaliplatin, triplatin tetranitrate, and DDS formulations thereof.

Examples of the "antimetabolite" include, but are not limited to, antifolates, pyrimidine metabolism inhibitors, purine metabolism inhibitors, ribonucleotide reductase inhibitors, and nucleotide analogs.

Examples of the "antimetabolite" include, but are not limited to, mercaptopurine, 6-mercaptopurine riboside, thioinosine, methotrexate, pemetrexed, eoshitabin, enocitabine, cytarabine, cytarabine ocfosfate, ancitabine hydrochloride, 5-FU agents (e.g., fluorouracil, Carzonal, Bennan, Lunachol, Lunapon, tegafur, tegafur-uracil, tegafur-gimeracil-oteracil potassium (TS-1), UFT, doxifluridine, carmofur, gallocitabine, emitefur, and capecitabine), aminopterin, nelarabine, leucovorin calcium, Tabloid, butocine, folinate calcium, levofolinate calcium, cladribine, emitefur, fludarabine, gemcitabine, hydroxycarbamide, pentostatin, piritrexim, idoxuridine, mitoguazone, tiazofurine, ambamustine, bendamustine, floxuridine, leucovorin, hydroxyurea, thioguanine, asparaginase, bortezomib, raltitrexed, clofarabine, enocitabine, sapacitabine, azacytidine, sulfadiazine, sulfamethoxazole, trimethoprim, Liproxstatin-1, D4476, Xanthohumol, Epacadostat (INCB024360), Vidofludimus, P7C3, GMX1778 (CHS828), NCT-501, SW033291, Ro61-8048, and DDS formulations thereof.

Examples of the "topoisomerase inhibitor" include, but are not limited to, doxorubicin, daunorubicin, epirubicin, idarubicin, anthracenedione, mitoxantrone, mitomycin C, bleomycin, dactinomycin, plicatomycin, irinotecan, camptothecin, rubitecan, belotecan, etoposide, teniposide, topotecan, amsacrine, and DDS formulations thereof.

Examples of the "DNA intercalator" include, but are not limited to, proflavine, doxorubicin (adriamycin), daunorubicin, dactinomycin, thalidomide, and DDS formulations thereof.

Examples of the "antimitotic agent" include, but are not limited to, paclitaxel, paclitaxel derivatives (e.g., DHA paclitaxel, paclitaxel polyglutamate, nab-paclitaxel, micellar paclitaxel, 7α-glucosyloxyacetylpaclitaxel, and BMS-275183), docetaxel, vinorelbine, vincristine, vinblastine, vindesine, vinzolidine, etoposide, teniposide, ixabepilone, larotaxel, ortataxel, tesetaxel, ispinesib, colchicine, vinflunine, and DDS formulations thereof.

Examples of the "antitumor antibiotic" include, but are not limited to, actinomycin D, actinomycin C, mitomycin C, chromomycin A3, mithramycin A, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, amrubicin hydrochloride, neocarzinostatin, zinostatin stimalamer, mithramycin, sarkomycin, carzinophilin, mitotane, zorubicin hydrochloride, mitoxantrone hydrochloride, idarubicin hydrochloride, liposomal doxorubicin, and DDS formulations thereof.

Examples of the "plant-derived anticancer agent" include, but are not limited to, irinotecan, nogitecan, etoposide, etoposide phosphate, eribulin, sobuzoxane, vinblastine sulfate, vincristine sulfate, vindesine sulfate, teniposide, paclitaxel, paclitaxel injection, docetaxel, DJ-927, vinorelbine, topotecan, and DDS formulations thereof.

Examples of the "epigenome drug" include, but are not limited to, DNA methylation inhibitors, histone deacetylase (HDAC) inhibitors, DNA methyl transferase (DNMT) inhibitors, histone deacetylase activators, histone demethylase inhibitors, and methylated nucleotides.

Specific examples of the "epigenome drug" include, but are not limited to, vorinostat, belinostat, mocetinostat (MGCD0103), entinostat (SNDX-275), romidepsin, azacytidine, decitabine, GSK2879552 2Hl, SGC707, ORY-1001 (RG-6016), PFI-4, SirReal2, GSK2801, CPI-360, GSK503, AMI-1, CPI-169, and DDS formulations thereof.

Examples of the "immunomodulating drug" include, but are not limited to, thalidomide, lenalidomide, pomalidomide, and DDS formulations thereof.

The "molecular targeted drug" can be a small compound or an antibody. Examples of the "molecular targeted drug" include, but are not limited to, kinase inhibitors, proteasome inhibitors, monoclonal antibodies, mTOR inhibitors, TNF inhibitors, and T-cell inhibitors.

Examples of the "kinase inhibitor" include, but are not limited to, tyrosine kinase inhibitors, serine/threonine kinase inhibitors, Raf kinase inhibitors, cyclin-dependent kinase (CDK) inhibitors, and mitogen-activated protein kinase (MEK) inhibitors.

Specific examples of the "kinase inhibitor" include, but are not limited to, imatinib, gefitinib, erlotinib, afatinib, dasatinib, bosutinib, vandetanib, sunitinib, axitinib, pazopanib, lenvatinib, lapatinib, nintedanib, nilotinib, crizotinib, ceritinib, alectinib, ruxolitinib, tofacitinib, ibrutinib, sorafenib, vemurafenib, dabrafenib, palbociclib, trametinib, regorafenib, cedivanib, lestaurtinib, bandetinib, vatalanib, seliciclib, tivantinib, canertinib, pelitinib, tesevatinib, cediranib, motesanib, midostaurin, foretinib, cabozantinib, selumetinib, neratinib, volasertib, saracatinib, enzastaurin, tandutinib, semaxanib, alvocidib, ICR-62, AEE788, PD0325901, PD153035, TK787, amcasertib (BBI503), E6201, E7050, and DDS formulations thereof.

Examples of the "proteasome inhibitor" include, but are not limited to, bortezomib, carfilzomib, and DDS formulations thereof.

Examples of the "monoclonal antibody" include, but are not limited to, anti-CD22 antibodies, anti-CD20 antibodies, anti-CD25 antibodies, anti-CD30 antibodies, anti-CD33 antibodies, anti-CD5 antibodies, anti-CD52 antibodies, anti-epidermal growth factor receptor antibodies (EGFR antibodies), anti-vascular endothelial cell growth factor antibodies (VEGF antibodies), anti-TNF-α antibodies, anti-IL-1 receptor antibodies, anti-IL-2 receptor antibodies, anti-IL-5 receptor antibodies, anti-IL-6 receptor antibodies, anti-HER2 antibodies, anti-IgE antibodies, anti-IgG antibodies, anti-RS virus antibodies, anti-CCR4 antibodies, anti-cytotoxic T lymphocyte-associated antigen 4 (CTLA-4, CD152) antibodies, anti-PD-1 antibodies, anti-receptor activator of nuclear factor κB ligand (RANKL) antibodies, anti-c-Met antibodies, and anti-CXCR4 antibodies.

Specific examples of the "monoclonal antibody" include, but are not limited to, ibritumomab tiuxetan, rituximab, cetuximab, infliximab, basiliximab, brentuximab vedotin, tocilizumab, trastuzumab, bevacizumab, omalizumab, mepolizumab, gemtuzumab ozogamicin, palivizumab, ranibizumab, certolizumab, ocrelizumab, mogamulizumab, eculizumab, pertuzumab, alemtuzumab, inotuzumab, panitumumab, ofatumumab, golimumab, adalimumab, ramucirumab, nivolumab, anakinra, denosumab, ipilimumab, pembrolizumab, matuzumab, farletuzumab, MORAb-004, MORAb009, and DDS formulations thereof.

Examples of the "mTOR inhibitor" include, but are not limited to, everolimus (RAD001), rapamycin (sirolimus), AZD8055, temsirolimus (CCI-779, NSC683864), KU-0063794, voxtalisib (XL-765, SAR245409), MHY1485, dactolisib (BEZ235), PI-103, torkinib (PP242), ridaforolimus (deforolimus, MK-8669), INK-128 (MLN0128), Torin1, omipalisib (GSK2126458, GSK458), OSI-027, PF-04691502, apitolisib (GDC-0980, RG7422), GSK1059615, gedatolisib (PF-05212384, PKI-587), WYE-132, PP121, WYE-354, AZD2014, Torin2, WYE-687, CH5132799, WAY-600, ETP-46464, GDC-0349, XL388, zotarolimus (ABT-578), tacrolimus (FK506), BGT226 (NVP-BGT226), Palomid 529 (P529), chrysophanic acid, and DDS formulations thereof.

Examples of the "TNF inhibitor" include, but are not limited to, etanercept, lenalidomide (CC-5013), pomalidomide, thalidomide, necrostatin-1, and QNZ (EVP4593).

Examples of the "T-cell inhibitor" include, but are not limited to, abatacept.

Examples of the "angiogenesis inhibitor" include, but are not limited to, CM101, IFN-α, IL-12, platelet factor-4, suramin, semaxanib, thrombospondin, VEGFR antagonists, combinations of an angiostatic steroid and heparin, cartilage-derived angiogenesis inhibitors, matrix metalloproteinase inhibitors, batimastat, marimastat, angiostatin, endostatin, 2-methoxyestradiol, tecogalan, thrombospondin, αVβ3 inhibitors, linomide, ADH-1, E7820, and DDS formulations thereof.

Examples of the "other chemotherapeutic agent" include, but are not limited to, finasteride, sobuzoxane, obatoclax, efaproxiral, tipifarnib, and lonafarnib.

When the peptide of (a) or the compound represented by the formula (1) or a pharmaceutically acceptable salt thereof as described herein is used in combination with a different cancer antigen peptide or a pharmaceutically acceptable salt thereof and/or coadministration drug, these active agents may be formulated in separate compositions or incorporated in a single composition. In an embodiment, the peptide of (a) or the compound represented by the formula (1) or a pharmaceutically acceptable salt thereof and a different cancer antigen peptide are incorporated in a single composition. In another embodiment, the peptide of (a) or the compound represented by the formula (1) or a pharmaceutically acceptable salt thereof and a different cancer antigen peptide are formulated in separate compositions. A composition may comprise one or more types of the peptide of (a) or the compound represented by the formula (1) or pharmaceutically acceptable salts thereof and/or one or more different cancer antigen peptides. A composition comprising any of the active agents may be provided together with instructions of dosage and administration for use of the composition in combination with the other active agent(s). Compositions each comprising any of the active agents may be incorporated in a single kit. Such a kit may further comprise instructions of dosage and administration for use of the compositions in combination, or may be packaged. In administration of more than one active agent in combination, the agents may be administered on the same administration schedule or different administration schedules.

The composition of the disclosure may comprise a pharmaceutically acceptable carrier in addition to the peptide of (a) or the compound represented by the formula (1) or a pharmaceutically acceptable salt thereof. Also, the composition of the disclosure may further comprise, or be administered in combination with, an appropriate adjuvant for enhancing the induction of CTLs and/or helper T cells by the composition.

The "pharmaceutically acceptable carrier" refers to a carrier that is non-toxic to a cell or a mammal exposed to the carrier at an amount or concentration it is used. In some embodiments, a pH buffered aqueous solution is used as a pharmaceutically acceptable carrier. Examples of the "pharmaceutically acceptable carrier" include buffering agents (such as phosphate, citrate, lactate, tartrate, trifluoroacetate and other organic acids); antioxidants (such as ascorbic acid); low molecular weight polypeptides (less than about 10 residues); proteins (such as serum albumin, gelatin or immunoglobulin); hydrophilic polymers (such as polyvinylpyrrolidone); amino acids (such as glycine, glutamine, asparagine, arginine, methionine or lysine); monosaccharides, disaccharides and other carbohydrates (such as glucose, mannose or dextrin); chelating agents (such as EDTA); sugar alcohols (such as mannitol, trehalose or sorbitol); stabilizers (such as diethylenetriaminepentaacetic acid); salt forming counterions (such as sodium); solubilizing agents (such as polysorbate 800), and/or nonionic surfactants (such as TWEEN^{®}, polyethylene glycol (PEG) and PLURONICS^{®}). A macromolecular material that is metabolized slowly, such as a protein, a polypeptide, a liposome, a polysaccharide, polylactide, polyglycolic acid, polymeric amino acids, amino acid copolymers, and inactive virus particles may also be useful as a pharmaceutically acceptable carrier. For administration, the compound represented by the formula (1) or the peptide as described herein may be formulated in a liposome preparation, attached to beads having a diameter of a micrometer order, or associated with a lipid carrier.

The adjuvant may be any of adjuvants as described in Clin. Microbiol. Rev., 7: 277-289, 1994. Specifically, the adjuvant may be a microorganism-derived agent, GM-CSF, a cytokine such as interleukin-2, interleukin-7, or interleukin-12, a plant-derived agent, a marine organism-derived agent, a mineral gel such as aluminum hydroxide, lysolecithin, a surfactant such as pluronic polyol, a polyanion, a peptide, or an oil emulsion (an emulsion preparation). Examples of the microorganism-derived agent include lipid A, monophosphoryl lipid A, which is a derivative of lipid A, killed bacteria (e.g., Mycobacterium bacteria such as BCG bacteria), bacterium-derived proteins, polynucleotides, Freund's incomplete adjuvant, Freund's complete adjuvant, cell wall skeleton components (e.g., BCG-CWS), trehalose dimycolate (TDM).

The adjuvant may also be a sedimentary adjuvant or an oil adjuvant. A sedimentary adjuvant can be a suspension of an inorganic substance to which a peptide can be adsorbed. Examples of the sedimentary adjuvant include sodium hydroxide, aluminum hydroxide (Alum), calcium phosphate, aluminum phosphate, alum, Pepesu, and carboxyvinyl polymer. An oil adjuvant can be an oil emulsifier that is able to emulsify a peptide by forming micelles comprising an aqueous peptide solution phase encapsulated in a mineral oil membrane. Examples of the oil adjuvant include, but are not limited to, liquid paraffin, lanolin, Freund's adjuvant (Freund's complete adjuvant, and Freund's incomplete adjuvant), Montanide, and a W/O emulsion (see WO2006/078059).

The composition of the disclosure may be provided as a dosage form for oral administration or parenteral administration. Examples of dosage forms for parenteral administration include an injectable preparation, an external preparation, a suppository, an inhalable preparation, or a nasal preparation. In a preferred embodiment, the composition of the disclosure is provided as an injectable preparation.

An injectable preparation may be in the form of a solution, a suspension, or an emulsion, which comprises one or more active agents dissolved, dispersed or emulsified in a liquid for injection, or may be provided as a solid formulation comprising active agent(s) to be dissolved or dispersed in a liquid for injection before use. A liquid for injection may comprise distilled water for injection, physiological saline, a vegetable oil, propylene glycol, polyethylene glycol, an alcohol such as ethanol, or a combination thereof. An injectable preparation may additionally comprise a stabilizer, a solubilizing aid (such as glutamic acid, aspartic acid, or polysorbate 80^{®}), a dispersant, an emulsifier, an analgesic, a buffer, a preservative, or other appropriate additive. For providing an injectable preparation as a sterilized preparation, it may be subjected to sterilization in the final step of its production, or produced aseptically throughout its production. A formulation for injection may be provided as a sterilized solid formulation, for example a lyophilized formulation, which may be reconstituted in sterilized water for injection or other appropriate sterilized liquid before use.

An external preparation may be in the form of an ointment, a gel, a cream, a plaster, a patch, a liniment, a spray, an inhalant, an aerosol, an eye drop, or a nasal drop, which may be prepared in accordance with a conventionally known preparation method, and may comprise one or more active agents.

An ointment may be prepared in accordance with a conventionally known preparation method, for example by incorporating one or more active agents in an ointment base by grinding or melting. For preparing an ointment, any conventionally used ointment base may be used, which may comprise a higher fatty acid or fatty acid ester (such as adipic acid, myristic acid, palmitic acid, stearic acid, or oleic acid, or an ester thereof), a wax (such as beeswax, spermaceti, or ceresin), a surfactant (such as polyoxyethylene alkyl ether phosphate), a higher alcohol (such as cetanol, stearyl alcohol, or cetostearyl alcohol), a silicone oil (such as dimethylpolysiloxane), a hydrocarbon (such as a hydrophilic petrolatum, white petrolatum, purified lanolin, or liquid paraffin), a glycol (such as ethylene glycol, diethylene glycol, propylene glycol, polyethylene glycol, or macrogol), a vegetable oil (such as castor oil, olive oil, sesame oil, or turpentine oil), an animal oil (such as mink oil, egg-yolk oil, squalane, or squalene), water, an absorption enhancer, a skin protective agent, or a combination thereof. An ointment may additionally comprise a humectant, a preservative, a stabilizer, an antioxidant, a fragrance, or other appropriate additive.

The pharmaceutical composition in a gel form may be prepared in accordance with a conventionally known preparation method, for example by incorporating one or more active agents in a gel base by melting. For preparing a gel, any conventionally used pharmaceutical gel base may be used, which may comprise a lower alcohol (such as ethanol, or isopropyl alcohol), a gelling agent (such as carboxymethyl cellulose, hydroxyethyl cellulose, hydroxypropyl cellulose, or ethyl cellulose), a neutralizing agent (such as triethanolamine, or diisopropanolamine), a surfactant (such as polyoxyethylene glycol monostearate), a gum, water, an absorption enhancer, a skin protective agent, or a combination thereof. A gel may additionally comprise a preservative, an antioxidant, a fragrance, or any other appropriate additive(s).

The pharmaceutical composition in a cream form may be prepared in accordance with a conventionally known preparation method, for example by incorporating one or more active agents in a pharmaceutical cream base by melting or emulsification. For preparing a cream, any conventionally used pharmaceutical cream base may be used, which may comprise a higher fatty acid ester, a lower alcohol, a hydrocarbon, a polyhydric alcohol (such as propylene glycol, or 1,3-butylene glycol), a higher alcohol (such as 2-hexyldecanol, or cetanol), an emulsifier (such as a polyoxyethylene alkyl ether, or a fatty acid ester), water, an absorption enhancer, a skin protective agent, or a combination thereof. A cream may additionally comprise a preservative, an antioxidant, a fragrance, or other appropriate additive.

The pharmaceutical composition in the form of a plaster may be prepared in accordance with a conventionally known preparation method, for example by incorporating one or more active agents in a plaster base by melting, and applying the mixture onto a support. For preparing a plaster, any conventionally used pharmaceutical plaster base may be used, which may comprise a thickening agent (such as polyacrylic acid, polyvinylpyrrolidone, gum arabic, starch, gelatin, or methyl cellulose), a humectant (such as urea, glycerol, or propylene glycol), a filler (such as kaolin, zinc oxide, talc, calcium, or magnesium), water, a solubilizing aid, a tackifier, a skin protective agent, or a combination thereof. A plaster may additionally comprise a preservative, an antioxidant, a fragrance, or other appropriate additive.

The pharmaceutical composition in the form of a patch may be prepared in accordance with a conventionally known preparation method, for example by incorporating one or more active agents in a patch base by melting, and applying the mixture onto a support. For preparing a patch, any conventionally used pharmaceutical patch base may be used, which may comprise a polymer, an oil or fat, a higher fatty acid, a tackifier, a skin protective agent, or a combination thereof. A patch may additionally comprise a preservative, an antioxidant, a fragrance, or other appropriate additive.

The pharmaceutical composition in the form of a liniment may be prepared in accordance with a conventionally known preparation method, for example by dissolving, dispersing or emulsifying one or more active agents in a vehicle that may comprise water, an alcohol (such as ethanol, or polyethylene glycol), a higher fatty acid, glycerol, soap, an emulsifier, a dispersant, or a combination thereof. A liniment may additionally comprise a preservative, an antioxidant, a fragrance, or other appropriate additive.

The pharmaceutical composition in the form of a spray, or an inhalant may comprise active agent(s), and optionally a stabilizing agent such as sodium hydrogen sulfite, or a tonicity agent or buffer, such as sodium chloride, sodium citrate or citric acid, in a vehicle.

The pharmaceutical composition in a dosage form for inhalation may be in the form of an aerosol, an inhalable powder, or an inhalable liquid, or may be provided as a liquid concentrate that is to be dissolved or dispersed in water or other appropriate vehicle to form an inhalable preparation before use. A preparation for inhalation may be prepared in accordance with a conventionally known preparation method. An inhalable liquid may optionally comprise a preservative (such as benzalkonium chloride, or paraben), a coloring agent, a buffer (such as sodium phosphate, or sodium acetate), a tonicity agent (such as sodium chloride, or concentrated glycerin), a thickening agent (such as a carboxyvinyl polymer), an absorption enhancer, or other appropriate additive. An inhalable powder may optionally comprise a lubricant (such as stearic acid, or a salt thereof), a binder (such as starch, or dextrin), a filler (such as lactose, or cellulose), a coloring agent, a preservative (such as benzalkonium chloride, or paraben), an absorption enhancer, or other appropriate additive. For administration of an inhalable liquid, a spray device (such as an atomizer, or a nebulizer) is usually used. An inhalable powder is usually dispensed from a powder inhalation device.

The pharmaceutical composition in the form of a spray may comprise active agent(s), and optionally a stabilizing agent (such as sodium hydrogen sulfite), or a tonicity agent or buffer (such as sodium chloride, sodium citrate, or citric acid) in a vehicle. A spray may be prepared in accordance with a preparation method as described, for example, in US 2,868,691, or US 3,095,355.

Other parenteral dosage forms include a rectal suppository or a vaginal pessary.

In one embodiment, the composition comprising the peptide of (a) or the compound represented by the formula (1) or a pharmaceutically acceptable salt thereof comprises one or more pharmaceutically acceptable carriers selected from the group consisting of trehalose, mannitol, methionine, citric acid, lactic acid, tartaric acid, acetic acid, trifluoroacetic acid, and a pH adjusting agent.

The peptide of (a) or the compound represented by the formula (1) or a pharmaceutically acceptable salt thereof, and if any, a coadministration drug, can be administered to a subject by an appropriate method depending on the disease to treat, condition of the subject, target site of the administration, or other factors. For example, parenteral administration, preferably, intravenous, intramuscular, intradermal, or subcutaneous administration by injection or infusion may be useful. The peptide or compound or a pharmaceutically acceptable salt thereof as described herein may be used in a lymphocyte therapy or a DC (dendritic cell) therapy.

Frequency of dose, or dosing interval may be appropriately selected depending on the disease to treat, condition of the subject, route of administration, or other factor. Administration is usually repeated, preferably every few days or few months.

The peptide of (a) or the compound represented by the formula (1) or a pharmaceutically acceptable salt thereof, and if any, a coadministration drug, can be administered to a subject in an appropriate amount depending on the disease to treat, condition of the subject, route of the administration, or other factors, respectively. The peptide or compound or a pharmaceutically acceptable salt thereof may usually be administered in an amount of 0.0001 mg to 1000 mg, preferably 0.001 mg to 1000 mg, more preferably 0.1 mg to 10 mg, at one time. A coadministration drug may be administered in an amount appropriately selected on the basis of a known clinical dose of the drug. For example, an immunomodulator as a coadministration drug may usually be administered in an amount of 0.0001 mg to 1000 mg per kg body weight, preferably 0.001 mg to 1000 mg per kg body weight, more preferably 0.1 mg to 10 mg per kg body weight.

When more than one active agent is incorporated in a single composition, they may be incorporated at an amount ratio appropriately selected depending on the disease to treat, condition of the subject, route of administration, or other factor. For example, for treating a human subject, a coadministration drug such as an immunomodulator may be used in an amount of 0.01 to 100 parts by weight relative to the peptide and/or the compound as described herein.

The term "effective amount" as used herein means an amount of an active agent that can completely or partially inhibit progression of a cancer or at least partially reduce one or more symptoms of a cancer, or that can provide induction of remission, maintenance of remission and/or suppression of recurrence. An effective amount of an agent is determined depending on the age or sex of the subject, the type or severity of condition to treat with the agent, a desired outcome of the treatment with the agent, or other factors. An effective amount for a particular subject can be determined by a person skilled in the art according to any known method.

The peptide of (a) or the compound represented by the formula (1) or a pharmaceutically acceptable salt thereof may be administered in combination with a non-drug therapy, or even more than one non-drug therapy selected, for example, from surgery, radiotherapy, gene therapy, hyperthermia, cryotherapy, or laser burning therapy. For example, the peptide of (a) or the compound represented by the formula (1) or a pharmaceutically acceptable salt thereof may be administered before or after a non-drug therapy such as surgery, or before or after a combination of two or three non-drug therapies.

The peptide of (a) or the compound represented by the formula (1) or a pharmaceutically acceptable salt thereof can be further used in combination with an agent to reduce an unwanted side effect, if any, such as an antiemetic agent, sleep-inducing agent, or anticonvulsant.

In another embodiment, the present disclosure relates to antigen-presenting cells (for example, dendritic cells, B-lymphocytes, or macrophages) presenting the peptide of (a) via HLA-A*02:07, HLA-A*03:01, HLA-B*15:01 or HLA-B^{∗}27:05. CTLs can be induced in an HLA-A*02:07, HLA-A*03:01, HLA-B^{∗}15:01 or HLA-B*27:05-positive subject by using such antigen-presenting cells. The antigen-presenting cells presenting the peptide of (a) via HLA-A^{∗}02:07, HLA-A*03:01, HLA-B*15:01 or HLA-B*27:05 can be obtained by culturing HLA-A*02:07, HLA-A^{∗}03:01, HLA-B*15:01 or HLA-B*27:05-positive immature antigen-presenting cells in the presence of the peptide of (a) or the compound represented by the formula (1) or a pharmaceutically acceptable salt thereof.

In another embodiment, the present disclosure provides a method of inducing antigen presenting cells, comprising culturing HLA-A*02:07, HLA-A*03:01, HLA-B*15:01 or HLA-B*27:05-positive immature antigen presenting cells in the presence of the peptide of (a) or the compound represented by the formula (1) or a pharmaceutically acceptable salt thereof. The term "immature antigen presenting cells" herein refers to cells that can mature into antigen presenting cells (for example, dendritic cells, B-lymphocytes or macrophages). Since the immature antigen-presenting cells are contained, for example, in PBMCs, PBMCs may be cultured in the presence of the peptide or compound or a pharmaceutically acceptable salt thereof.

In another embodiment, the present disclosure provides a method of treating a cancer in an HLA-A*02:07, HLA-A*03:01, HLA-B*15:01 or HLA-B*27:05-positive subject, comprising administering antigen-presenting cells presenting the peptide of (a) via HLA-A*02:07, HLA-A*03:01, HLA-B*15:01 or HLA-B*27:05 to a subject positive for the corresponding HLA subtype. The antigen presenting cells may be administered by any method appropriately selected depending on a disease to treat, a condition of the subject, or a target site of the administration, or other factor. The antigen presenting cells may be administered intravenously, intradermally, subcutaneously, intramuscularly, or intranasally, or by other administration route.

In another embodiment, the present disclosure relates to CTLs induced by the peptide of (a) or the compound represented by the formula (1) or a pharmaceutically acceptable salt thereof. The CTLs can damage cancer cells in a HLA-A*02:07, HLA-A*03:01, HLA-B*15:01 or HLA-B*27:05-positive subject.

In another embodiment, the present disclosure relates to a method of inducing CTLs, comprising culturing PBMCs of an HLA-A^{∗}02:07, HLA-A*03:01, HLA-B*15:01 or HLA-B*27:05-positive subject in the presence of the peptide of (a) or the compound represented by the formula (1) or a pharmaceutically acceptable salt thereof. When PBMCs are cultured in the presence of the peptide or compound or a pharmaceutically acceptable salt, peptide-specific CTLs are induced from precursor cells in the PBMCs. The peptide-specific CTLs obtained by the method can be administered to an HLA-A^{∗}02:07, HLA-A*03:01, HLA-B*15:01 or HLA-B*27:05-positive subject to treat a cancer in the subject.

In another embodiment, the present disclosure relates to a method of treating a cancer in an HLA-A*02:07, HLA-A*03:01, HLA-B^{∗}15:01 or HLA-B*27:05-positive subject, comprising administering CTLs specific to the peptide of (a) to a subject. The CTLs specific to the peptide can be administered by a method appropriately selected depending on a disease to treat, a condition of the subject, or a target site of the administration, or other factor. The CTLs may be administered intravenously, intradermally, subcutaneously, intramuscularly, intranasally, or orally, or by other administration route.

In another embodiment, the present disclosure relates to a kit comprising the peptide of (a) or the compound represented by the formula (1) or a pharmaceutically acceptable salt thereof as a component. In an embodiment, the kit is used for treating a cancer in an HLA-A*02:07, HLA-A*03:01, HLA-B*15:01 or HLA-B*27:05-positive subject. In another embodiment, the kit is used in the method of inducing antigen-presenting cells or the method of inducing CTLs herein described. The kit may contain, other than the peptide or compound or a pharmaceutically acceptable salt thereof, a means for taking a sample (for example, peripheral blood mononuclear cells) from a subject, an adjuvant or a container for reaction. An instruction booklet is usually attached to the kit.

In an embodiment, the present disclosure provides a method of treating a cancer in an HLA-A*02:07, HLA-A*03:01, HLA-B*15:01 or HLA-B*27:05-positive subject, comprising administering the peptide of (a) or the compound represented by the formula (1) or a pharmaceutically acceptable salt thereof to an HLA-A*02:07, HLA-A*03:01, HLA-B*15:01 or HLA-B*27:05-positive subject.

In an embodiment, the present disclosure provides the peptide of (a) or the compound represented by the formula (1) or a pharmaceutically acceptable salt thereof for use in treating a cancer in an HLA-A*02:07, HLA-A`03:01, HLA-B*15:01 or HLA-B*27:05-positive subject.

In an embodiment, the present disclosure provides use of the peptide of (a) or the compound represented by the formula (1) or a pharmaceutically acceptable salt thereof for the manufacture of a medicament for treating a cancer in an HLA-A^{∗}02:07, HLA-A*03:01, HLA-B*15:01 or HLA-B^{∗}27:05-positive subject.

In another embodiment, the present disclosure provides a pharmaceutical composition comprising the peptide of (a) or the compound represented by the formula (1) or a pharmaceutically acceptable salt thereof for treating a benign tumor in an HLA-A*02:07, HLA-A*03:01, HLA-B*15:01 or HLA-B*27:05-positive subject. This embodiment can be carried out in accordance with the above description about the treatment of a cancer.

The "benign tumor" is a tumor that has no pathologically malignant findings and is understood to be different from a malignant tumor. It is said that a benign tumor does not show metastasis or infiltration tendency. Diagnosis as a benign tumor does not necessarily mean a good clinical prognosis. For example, a low-grade meningioma that develops in the brain stem is a benign tumor, but it is difficult to treat, and it is clinically malignant because it compresses the brain stem and shows a poor prognosis, and thus often requires treatment or prevention.

Benign tumors include, but not limited to, familial adenomatous polyposis, non-hereditary colorectal adenoma, intraductal papillary mucinous neoplasm, meningioma, schwannoma, epithelial adenoma of an organ, papilloma, non-epithelial myoma, lipoma, chondroma, and hemangioma.

In an embodiment, the benign tumor is familial adenomatous polyposis. Familial adenomatous polyposis is a hereditary disease characterized by mutation of a tumor suppressor gene APC (adenoma polyposis coli) and a large number of adenomas formed in the intestinal tract. The terms "familial adenomatous polyposis", "familial polyposis coli", "familial adenomatous polyposis coli" and "FAP" can be used interchangeably. Familial adenomatous polyposis also includes diseases that coincide with tumors in tissues other than the intestinal tract, such as Gardner's syndrome.

In a further embodiment, the present disclosure provides a method of treating a benign tumor in an HLA-A^{∗}02:07, HLA-A*03:01, HLA-B*15:01 or HLA-B*27:05-positive subject, comprising administering the peptide of (a) or the compound represented by the formula (1) or a pharmaceutically acceptable salt thereof to an HLA-A^{∗}02:07, HLA-A*03:01, HLA-B*15:01 or HLA-B*27:05-positive subject; the peptide of (a) or the compound represented by the formula (1) or a pharmaceutically acceptable salt thereof for use in treating a benign tumor in an HLA-A*02:07, HLA-A*03:01, HLA-B*15:01 or HLA-B*27:05-positive subject; and use of the peptide of (a) or the compound represented by the formula (1) or a pharmaceutically acceptable salt thereof for the manufacture of a medicament for treating a benign tumor in an HLA-A*02:07, HLA-A*03:01, HLA-B*15:01 or HLA-B*27:05-positive subject.

Embodiments of the present disclosure will be more specifically described below.
[1] A pharmaceutical composition for treating a cancer in an HLA-A*02:07, HLA-A*03:01, HLA-B*15:01, or HLA-B*27:05-positive subject, comprising:
   (a) an MHC class I-restricted peptide consisting of 7 to 30 amino acid residues or a pharmaceutically acceptable salt thereof, wherein the MHC class I-restricted peptide is a peptide comprising an amino acid sequence selected from RMFPNAPYL (SEQ ID NO: 2), CMTWNQMNL (SEQ ID NO: 3), ALLPAVPSL (SEQ ID NO: 4), SLGEQQYSV (SEQ ID NO: 5), RVPGVAPTL (SEQ ID NO: 6) and VLDFAPPGA (SEQ ID NO: 7), or a peptide comprising an amino acid sequence which has one or several amino acids deleted from, substituted in, and/or added to the amino acid sequence selected from SEQ ID NOs: 2 to 7 and having an ability to induce CTLs; or
   (b) a compound represented by the formula (1) or a pharmaceutically acceptable salt thereof: 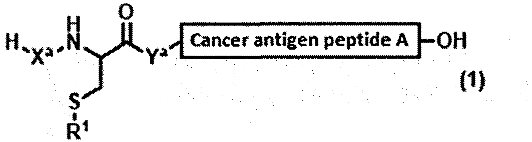 wherein X^{a} and Y^{a} each independently represent a single bond or a divalent peptide group consisting of 1 to 4 amino acid residues, provided that the sum of the numbers of amino acid residues in X^{a} and Y^{a} is an integer of 0 to 4;

   cancer antigen peptide A is an MHC class I-restricted peptide consisting of 7 to 30 amino acid residues, wherein an amino group of an N-terminal amino acid of the cancer antigen peptide A binds to Y^{a} in the formula (1), and a carbonyl group of a C-terminal amino acid of the cancer antigen peptide A binds to the hydroxyl group in the formula (1),
   R¹ is a hydrogen atom,
   a group represented by the formula (2): 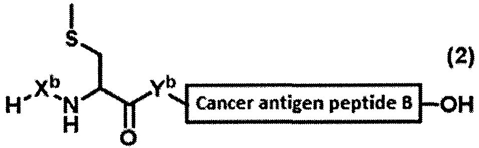 wherein X^{b} and Y^{b} each independently represent a single bond or a divalent peptide group consisting of 1 to 4 amino acid residues, provided that the sum of the numbers of amino acid residues in X^{b} and Y^{b} is an integer of 0 to 4,
   cancer antigen peptide B is an MHC class I-restricted peptide consisting of 7 to 30 amino acid residues, wherein an amino group of an N-terminal amino acid of the cancer antigen peptide B binds to Y^{b} in the formula (2), and a carbonyl group of a C-terminal amino acid of the cancer antigen peptide B binds to the hydroxyl group in the formula (2), and
   the sulfur atom in the formula (2) binds to the sulfur atom in the formula (1) via a disulfide bond,
   or cancer antigen peptide C, wherein the cancer antigen peptide C is an MHC class I-restricted peptide consisting of 7 to 30 amino acid residues including one cysteine residue or an MHC class II-restricted peptide consisting of 7 to 30 amino acid residues including one cysteine residue, and a sulfur atom of the cysteine residue of the cancer antigen peptide C binds to the sulfur atom in the formula (1) via a disulfide bond, and optionally a peptide consisting of 1 to 4 amino acid residues binds to an N-terminus of the cancer antigen peptide C,
   provided that when R¹ is a hydrogen atom, the cancer antigen peptide A is a peptide comprising an amino acid sequence selected from RMFPNAPYL (SEQ ID NO: 2), CMTWNQMNL (SEQ ID NO: 3), ALLPAVPSL (SEQ ID NO: 4), SLGEQQYSV (SEQ ID NO: 5), RVPGVAPTL (SEQ ID NO: 6) and VLDFAPPGA (SEQ ID NO: 7), or a peptide comprising an amino acid sequence which has one or several amino acids deleted from, substituted in, and/or added to the amino acid sequence selected from SEQ ID NOs: 2 to 7 and having an ability to induce CTLs;
   when R¹ is the group represented by the formula (2), the cancer antigen peptide A and/or cancer antigen peptide B is a peptide comprising an amino acid sequence selected from RMFPNAPYL (SEQ ID NO: 2), CMTWNQMNL (SEQ ID NO: 3), ALLPAVPSL (SEQ ID NO: 4), SLGEQQYSV (SEQ ID NO: 5), RVPGVAPTL (SEQ ID NO: 6) and VLDFAPPGA (SEQ ID NO: 7), or a peptide comprising an amino acid sequence which has one or several amino acids deleted from, substituted in, and/or added to the amino acid sequence selected from SEQ ID NOs: 2 to 7 and having an ability to induce CTLs;
   when R¹ is the cancer antigen peptide C, the cancer antigen peptide A and/or cancer antigen peptide C is a peptide comprising an amino acid sequence selected from RMFPNAPYL (SEQ ID NO: 2), CMTWNQMNL (SEQ ID NO: 3), ALLPAVPSL (SEQ ID NO: 4), SLGEQQYSV (SEQ ID NO: 5), RVPGVAPTL (SEQ ID NO: 6) and VLDFAPPGA (SEQ ID NO: 7), or a peptide comprising an amino acid sequence which has one or several amino acids deleted from, substituted in, and/or added to the amino acid sequence selected from SEQ ID NOs: 2 to 7 and having an ability to induce CTLs;
   when R¹ is the group represented by the formula (2) and the cancer antigen peptide B includes one cysteine residue, a sulfur atom of the cysteine residue of the cancer antigen peptide B optionally binds, via a disulfide bond, to
   a sulfur atom in the formula (3): 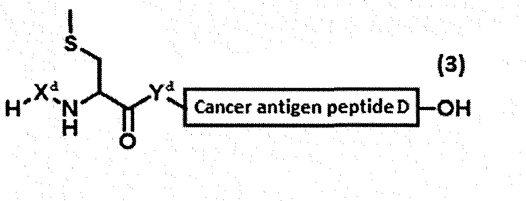 wherein X^{d} and Y^{d} each independently represent a single bond or a divalent peptide group consisting of 1 to 4 amino acid residues, provided that the sum of the numbers of amino acid residues in X^{d} and Y^{d} is an integer of 0 to 4, and
   cancer antigen peptide D is an MHC class II-restricted peptide consisting of 7 to 30 amino acid residues, wherein an amino group of an N-terminal amino acid of the cancer antigen peptide D binds to Y^{d} in the formula (3), and a carbonyl group of a C-terminal amino acid of the cancer antigen peptide D binds to the hydroxyl group in the formula (3),
   or a sulfur atom of a cysteine residue of cancer antigen peptide E, wherein the cancer antigen peptide E is an MHC class II-restricted peptide consisting of 7 to 30 amino acid residues including one cysteine residue; and
   when R¹ is the cancer antigen peptide C and a peptide consisting of 1 to 4 amino acid residues including one cysteine residue binds to the N terminus of the cancer antigen peptide C, a sulfur atom of the cysteine residue of the peptide binding to the N terminus of the cancer antigen peptide C optionally binds, via a disulfide bond, to
   a sulfur atom in the formula (3): 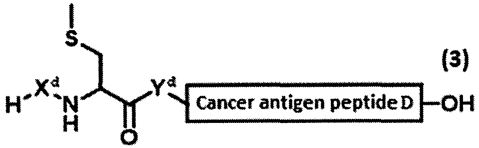 wherein X^{d} and Y^{d} each independently represent a single bond or a divalent peptide group consisting of 1 to 4 amino acid residues, provided that the sum of the numbers of amino acid residues in X^{d} and Y^{d} is an integer of 0 to 4, and
   cancer antigen peptide D is an MHC class II-restricted peptide consisting of 7 to 30 amino acid residues, wherein an amino group of an N-terminal amino acid of the cancer antigen peptide D binds to Y^{d} in the formula (3), and a carbonyl group of a C-terminal amino acid of the cancer antigen peptide D binds to the hydroxyl group in the formula (3),
   or a sulfur atom of a cysteine residue of cancer antigen peptide E, wherein the cancer antigen peptide E is an MHC class II-restricted peptide consisting of 7 to 30 amino acid residues including one cysteine residue.
[2] The pharmaceutical composition according to item [1], wherein the subject is an HLA-A*03:01, HLA-B*15:01 or HLA-B*27:05-positive subject.
[3] The pharmaceutical composition according to item [1], wherein the subject is an HLA-A*02 : 07-positive subject.
[4] The pharmaceutical composition according to item [1], wherein the subject is an HLA-A^{∗}03:01-positive subject.
[5] The pharmaceutical composition according to item [1], wherein the subject is an HLA-B*15:01-positive subject.
[6] The pharmaceutical composition according to item [1], wherein the subject is an HLA-B*27:05-positive subject.
[7] The pharmaceutical composition according to any one of items [1] to [6], comprising the peptide or a pharmaceutically acceptable salt thereof of (a).
[8] The pharmaceutical composition according to item [7], wherein the peptide of (a) is a peptide consisting of an amino acid sequence selected from RMFPNAPYL (SEQ ID NO: 2), CMTWNQMNL (SEQ ID NO: 3) and VLDFAPPGA (SEQ ID NO: 7), or a peptide consisting of an amino acid sequence which has one or several amino acids deleted from, substituted in, and/or added to the amino acid sequence selected from SEQ ID NOs: 2, 3 and 7 and having an ability to induce CTLs.
[9] The pharmaceutical composition according to item [7] or [8], wherein the peptide of (a) is a peptide consisting of an amino acid sequence selected from RMFPNAPYL (SEQ ID NO: 2), RYFPNAPYL (SEQ ID NO: 8), YMFPNAPYL (SEQ ID NO: 13), CMTWNQMNL (SEQ ID NO: 3), CYTWNQMNL (SEQ ID NO: 14) and VLDFAPPGA (SEQ ID NO: 7).
[10] The pharmaceutical composition according to any one of items [7] to [9], wherein the peptide of (a) is a peptide consisting of an amino acid sequence of RMFPNAPYL (SEQ ID NO: 2), RYFPNAPYL (SEQ ID NO: 8), or YMFPNAPYL (SEQ ID NO: 13).
[11] The pharmaceutical composition according to any one of items [7] to [10], wherein the peptide of (a) is a peptide consisting of the amino acid sequence of RMFPNAPYL (SEQ ID NO: 2).
[12] The pharmaceutical composition according to any one of items [1] to [6], comprising the compound represented by the formula (1) or a pharmaceutically acceptable salt thereof of (b).
[13] The pharmaceutical composition according to item [12], wherein X^{a} is a divalent peptide group consisting of two amino acid residues and Y^{a} is a single bond; X^{a} and Y^{a} each independently a divalent peptide group consisting of one amino acid residue; X^{a} is a single bond and Y^{a} is a divalent peptide group consisting of two amino acid residues; X^{a} is a divalent peptide group consisting of one amino acid residue and Y^{a} is a single bond; X^{a} is a single bond and Y^{a} is a divalent peptide group consisting of one amino acid residue; or X^{a} and Y^{a} are single bonds.
[14] The pharmaceutical composition according to item [12] or [13], wherein X^{a} is a single bond and Y^{a} is a single bond, an alanine residue, a leucine residue or a methionine residue.
[15] The pharmaceutical composition according to any one of items [12] to [14], wherein X^{a} is a single bond or a divalent peptide group consisting of one amino acid residue and Y^{a} is a single bond.
[16] The pharmaceutical composition according to any one of items [12] to [15], wherein X^{a} and Y^{a} are single bonds.
[17] The pharmaceutical composition according to any one of items [12] to [16], wherein the cancer antigen peptide A is a peptide comprising an amino acid sequence selected from
   RMFPNAPYL (SEQ ID NO: 2),
   CMTWNQMNL (SEQ ID NO: 3),
   ALLPAVPSL (SEQ ID NO: 4),
   SLGEQQYSV (SEQ ID NO: 5),
   RVPGVAPTL (SEQ ID NO: 6) and
   VLDFAPPGA (SEQ ID NO: 7), or
   a peptide comprising an amino acid sequence which has one or several amino acids deleted from, substituted in, and/or added to the amino acid sequence selected from SEQ ID NOs: 2 to 7 and having an ability to induce CTLs.
[18] The pharmaceutical composition according to any one of items [12] to [17], wherein the cancer antigen peptide A is a peptide comprising or consisting of an amino acid sequence selected from
   RMFPNAPYL (SEQ ID NO: 2),
   CMTWNQMNL (SEQ ID NO: 3),
   ALLPAVPSL (SEQ ID NO: 4),
   SLGEQQYSV (SEQ ID NO: 5),
   RVPGVAPTL (SEQ ID NO: 6) and
   VLDFAPPGA (SEQ ID NO: 7).
[19] The pharmaceutical composition according to item [18], wherein the cancer antigen peptide A is a peptide comprising the amino acid sequence of RMFPNAPYL (SEQ ID NO: 2).
[20] The pharmaceutical composition according to item [18], wherein the cancer antigen peptide A is a peptide consisting of the amino acid sequence of RMFPNAPYL (SEQ ID NO: 2).
[21] The pharmaceutical composition according to any one of items [12] to [20], wherein R¹ is a hydrogen atom.
[22] The pharmaceutical composition according to item [21], wherein the compound represented by the formula (1) is a peptide comprising or consisting of an amino acid sequence selected from
   CRMFPNAPYL (SEQ ID NO: 49),
   CCMTWNQMNL (SEQ ID NO: 50),
   CCYTWNQMNL (SEQ ID NO: 51),
   CALLPAVPSL (SEQ ID NO: 52),
   CSLGEQQYSV (SEQ ID NO: 53),
   CRVPGVAPTL (SEQ ID NO: 54) and
   CVLDFAPPGA (SEQ ID NO: 55).
[23] The pharmaceutical composition according to any one of items [12] to [20], wherein R¹ is a group represented by the formula (2).
[24] The pharmaceutical composition according to item [23], wherein X^{b} is a divalent peptide group consisting of two amino acid residues and Y^{b} is a single bond; X^{b} and Y^{b} are each independently a divalent peptide group consisting of one amino acid residue; X^{b} is a single bond and Y^{b} is a divalent peptide group consisting of two amino acid residues; X^{b} is a divalent peptide group consisting of one amino acid residue and Y^{b} is a single bond and X^{b} is a single bond and Y^{b} is a divalent peptide group consisting of one amino acid residue; or X^{b} and Y^{b} are single bonds.
[25] The pharmaceutical composition according to item [23] or [24], wherein X^{b} is a single bond and Y^{b} is a single bond, an alanine residue, a leucine residue or a methionine residue.
[26] The pharmaceutical composition according to any one of items [23] to [25], wherein X^{b} is a single bond or a divalent peptide group consisting of one amino acid residue and Y^{b} is a single bond.
[27] The pharmaceutical composition according to any one of items [23] to [26], wherein X^{b} and Y^{b} are single bonds.
[28] The pharmaceutical composition according to any one of items [23] to [27], wherein the cancer antigen peptide B is a peptide comprising an amino acid sequence selected from
   RMFPNAPYL (SEQ ID NO: 2),
   CMTWNQMNL (SEQ ID NO: 3),
   ALLPAVPSL (SEQ ID NO: 4),
   SLGEQQYSV (SEQ ID NO: 5),
   RVPGVAPTL (SEQ ID NO: 6) and
   VLDFAPPGA (SEQ ID NO: 7), or
   a peptide comprising an amino acid sequence which has one or several amino acids deleted from, substituted in, and/or added to the amino acid sequence selected from SEQ ID NOs: 2 to 7 and having an ability to induce CTLs.
[29] The pharmaceutical composition according to any one of items [23] to [28], wherein the cancer antigen peptide B is a peptide comprising or consisting of an amino acid sequence selected from
   RMFPNAPYL (SEQ ID NO: 2),
   CMTWNQMNL (SEQ ID NO: 3),
   ALLPAVPSL (SEQ ID NO: 4),
   SLGEQQYSV (SEQ ID NO: 5),
   RVPGVAPTL (SEQ ID NO: 6) and
   VLDFAPPGA (SEQ ID NO: 7).
[30] The pharmaceutical composition according to item [29], wherein the cancer antigen peptide B is a peptide comprising the amino acid sequence of RMFPNAPYL (SEQ ID NO: 2).
[31] The pharmaceutical composition according to [29], wherein the cancer antigen peptide B is a peptide consisting of the amino acid sequence of RMFPNAPYL (SEQ ID NO: 2).
[32] The pharmaceutical composition according to any one of items [23] to [29], wherein the compound represented by the formula (1) is
   a compound of formula (6): 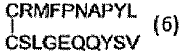 wherein C-C shown in the formula means that the C residues are linked together by a disulfide bond.
[33] The pharmaceutical composition according to any one of items [12] to [20], wherein R¹ is the cancer antigen peptide C.
[34] The pharmaceutical composition according to item [33], wherein the cancer antigen peptide C is an MHC class I-restricted peptide.
[35] The pharmaceutical composition according to item [34], wherein the cancer antigen peptide C is a peptide comprising the amino acid sequence of CMTWNQMNL (SEQ ID NO: 3) or a peptide comprising an amino acid sequence which has one or several amino acids deleted from, substituted in, and/or added to the amino acid sequence of SEQ ID NO: 3 and having an ability to induce CTLs.
[36] The pharmaceutical composition according to items [34] or [35], wherein the cancer antigen peptide C is a peptide comprising or consisting of an amino acid sequence selected from
   CMTWNQMNL (SEQ ID NO: 3) and
   CYTWNQMNL (SEQ ID NO: 14).
[37] The pharmaceutical composition according to any one of items [34] to [36], wherein the cancer antigen peptide C is a peptide consisting of the amino acid sequence of CMTWNQMNL (SEQ ID NO: 3).
[38] The pharmaceutical composition according to any one of items [34] to [36], wherein the cancer antigen peptide C is a peptide consisting of the amino acid sequence of CYTWNQMNL (SEQ ID NO: 14).
[39] The pharmaceutical composition according to any one of items [33] to [38], wherein the compound represented by the formula (1) is
   a compound of formula (4): 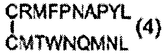 wherein C-C shown in the formula means that the C residues are linked together by a disulfide bond, or
   a compound of formula (5): 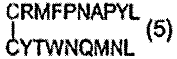 wherein C-C shown in the formula means that the C residues are linked together by a disulfide bond.
[40] The pharmaceutical composition according to item [39], wherein the compound represented by the formula (1) is
   a compound of formula (5): 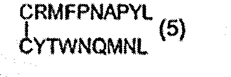 wherein C-C shown in the formula means that the C residues are linked together by a disulfide bond.
[41] The pharmaceutical composition according to any one of items [33] to [38], wherein a peptide consisting of 1 to 4 amino acid residues binds to the N-terminus of the cancer antigen peptide C.
[42] The pharmaceutical composition according to item [41], wherein the compound represented by the formula (1) is
   a compound of formula (7): 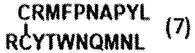 wherein C-C shown in the formula means that the C residues are linked together by a disulfide bond;
   a compound of formula (8): 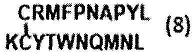 wherein C-C shown in the formula means that the C residues are linked together by a disulfide bond;
   a compound of formula (9): 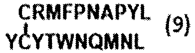 wherein C-C shown in the formula means that the C residues are linked together by a disulfide bond;
   a compound of formula (10): 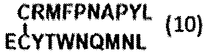 wherein C-C shown in the formula means that the C residues are linked together by a disulfide bond;
   a compound of formula (11): 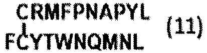 wherein C-C shown in the formula means that the C residues are linked together by a disulfide bond; or
   a compound of formula (12): 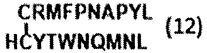 wherein C-C shown in the formula means that the C residues are linked together by a disulfide bond.
[43] The pharmaceutical composition according to item [33], wherein the cancer antigen peptide C is the MHC class II-restricted peptide.
[44] The pharmaceutical composition according to item [43], wherein the cancer antigen peptide C is a peptide comprising or consisting of an amino acid sequence selected from
   WAPVLDFAPPGASAYGSL (SEQ ID NO: 36),
   CWAPVLDFAPPGASAYGSL (SEQ ID NO: 37),
   WAPVLDFAPPGASAYGSLC (SEQ ID NO: 38),
   SGQARMFPNAPYLPSC (SEQ ID NO: 39),
   SGQAYMFPNAPYLPSC (SEQ ID NO: 40),
   SGQARMFPNAPYLPSCLES (SEQ ID NO: 41),
   SGQAYMFPNAPYLPSCLES (SEQ ID NO: 42),
   PGCNKRYFKLSHLQMHSRK (SEQ ID NO: 43),
   PGCNKRYFKLSHLQMHSRKH (SEQ ID NO: 44),
   PGCNKRYFKLSHLQMHSRKHTG (SEQ ID NO: 45),
   CNKRYFKLSHLQMHSRK (SEQ ID NO: 46),
   CNKRYFKLSHLQMHSRKH (SEQ ID NO: 47), and
   CNKRYFKLSHLQMHSRKHTG (SEQ ID NO: 48).
[45] The pharmaceutical composition according to item [44], wherein the compound represented by the formula (1) is
   a compound of formula (13): 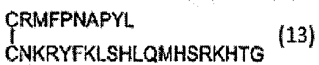 wherein C-C shown in the formula means that the C residues are linked together by a disulfide bond;
   a compound of formula (14): 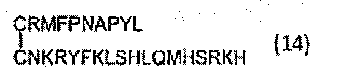 wherein C-C shown in the formula means that the C residues are linked together by a disulfide bond;
   a compound of formula (15): 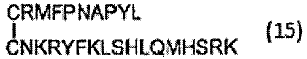 wherein C-C shown in the formula means that the C residues are linked together by a disulfide bond; or
   a compound of formula (16): 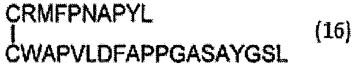 wherein C-C shown in the formula means that the C residues are linked together by a disulfide bond.
[46] The pharmaceutical composition according to any one of items [23] to [30], wherein R¹ is a group represented by the formula (2), the cancer antigen peptide B includes one cysteine residue, and the sulfur atom of the cysteine residue of the cancer antigen peptide B binds to the sulfur atom in the formula (3) or the sulfur atom of the cysteine residue of the cancer antigen peptide E via a disulfide bond.
[47] The pharmaceutical composition according to any one of items [33] to [38], wherein R¹ is the cancer antigen peptide C, the peptide consisting of 1 to 4 amino acid residues including one cysteine residue binds to the N-terminus of the cancer antigen peptide C, and the sulfur atom of the cysteine residue of the peptide binding to the N-terminus of the cancer antigen peptide C binds to the sulfur atom in the formula (3) or the sulfur atom of the cysteine residue of the cancer antigen peptide E via a disulfide bond.
[48] The pharmaceutical composition according to item [47], wherein the peptide consisting of 1 to 4 amino acid residues including one cysteine residue binding to the N-terminus of the cancer antigen peptide C is a dipeptide consisting of CA.
[49] The pharmaceutical composition according to any one of items [46] to [48], wherein X^{d} is a divalent peptide group consisting of two amino acid residues and Y^{d} is a single bond; X^{d} and Y^{d} each independently are a divalent peptide group consisting of one amino acid residue; X^{d} is a single bond and Y^{d} is a divalent peptide group consisting of two amino acid residues; X^{d} is a divalent peptide group consisting of one amino acid residue and Y^{d} is a single bond; X^{d} is a single bond and Y^{d} is a divalent peptide group consisting of one amino acid residue; or X^{d} and Y^{d} are single bonds.
[50] The pharmaceutical composition according to any one of items [46] to [49], wherein X^{d} is a single bond and Y^{d} is a single bond, an alanine residue, a leucine residue or a methionine residue.
[51] The pharmaceutical composition according to any one of items [46] to [50], wherein X^{d} is a single bond or a divalent peptide group consisting of one amino acid residue and Y^{d} is a single bond.
[52] The pharmaceutical composition according to any one of items [46] to [51], wherein X^{d} and Y^{d} are single bonds.
[53] The pharmaceutical composition according to any one of items [46] to [52], wherein the cancer antigen peptide D is a peptide comprising or consisting of an amino acid sequence selected from
   WAPVLDFAPPGASAYGSL (SEQ ID NO: 36),
   CWAPVLDFAPPGASAYGSL (SEQ ID NO: 37),
   WAPVLDFAPPGASAYGSLC (SEQ ID NO: 38),
   SGQARMFPNAPYLPSC (SEQ ID NO: 39),
   SGQAYMFPNAPYLPSC (SEQ ID NO: 40),
   SGQARMFPNAPYLPSCLES (SEQ ID NO: 41),
   SGQAYMFPNAPYLPSCLES (SEQ ID NO: 42),
   PGCNKRYFKLSHLQMHSRK (SEQ ID NO: 43),
   PGCNKRYFKLSHLQMHSRKH (SEQ ID NO: 44),
   PGCNKRYFKLSHLQMHSRKHTG (SEQ ID NO: 45),
   CNKRYFKLSHLQMHSRK (SEQ ID NO: 46),
   CNKRYFKLSHLQMHSRKH (SEQ ID NO: 47), and
   CNKRYFKLSHLQMHSRKHTG (SEQ ID NO: 48).
[54] The pharmaceutical composition according to any one of items [46] to [52], wherein
   the cancer antigen peptide E is a peptide comprising or consisting of an amino acid sequence selected from
   WAPVLDFAPPGASAYGSL (SEQ ID NO: 36),
   CWAPVLDFAPPGASAYGSL (SEQ ID NO: 37),
   WAPVLDFAPPGASAYGSLC (SEQ ID NO: 38),
   SGQARMFPNAPYLPSC (SEQ ID NO: 39),
   SGQAYMFPNAPYLPSC (SEQ ID NO: 40),
   SGQARMFPNAPYLPSCLES (SEQ ID NO: 41),
   SGQAYMFPNAPYLPSCLES (SEQ ID NO: 42),
   PGCNKRYFKLSHLQMHSRK (SEQ ID NO: 43),
   PGCNKRYFKLSHLQMHSRKH (SEQ ID NO: 44),
   PGCNKRYFKLSHLQMHSRKHTG (SEQ ID NO: 45),
   CNKRYFKLSHLQMHSRK (SEQ ID NO: 46),
   CNKRYFKLSHLQMHSRKH (SEQ ID NO: 47), and
   CNKRYFKLSHLQMHSRKHTG (SEQ ID NO: 48).
[55] The pharmaceutical composition according to item [46], wherein the compound represented by the formula (1) is
   a compound of formula (17): 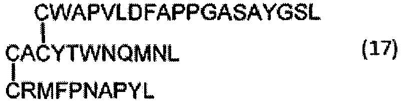 wherein C-C shown in the formula means that the C residues are linked together by a disulfide bond.
[56] The pharmaceutical composition according to item [47], wherein the compound represented by the formula (1) is
   a compound of formula (18): 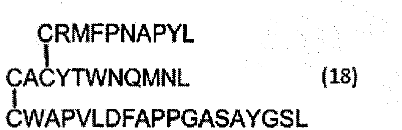 wherein C-C shown in the formula means that the C residues are linked together by a disulfide bond.
[57] The pharmaceutical composition according to item [47], wherein the compound represented by the formula (1) is
   a compound of formula (19): 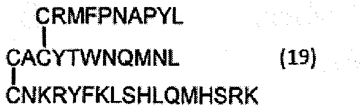 wherein C-C shown in the formula means that the C residues are linked together by a disulfide bond.
[58] The pharmaceutical composition according to any one of items [1] to [57], wherein the pharmaceutical composition further comprises or is used in combination with an MHC class II-restricted peptide.
[59] The pharmaceutical composition according to item [58], wherein
   the MHC class II-restricted peptide is a peptide comprising or consisting of an amino acid sequence selected from
   WAPVLDFAPPGASAYGSL (SEQ ID NO: 36),
   CWAPVLDFAPPGASAYGSL (SEQ ID NO: 37),
   WAPVLDFAPPGASAYGSLC (SEQ ID NO: 38),
   SGQARMFPNAPYLPSC (SEQ ID NO: 39),
   SGQAYMFPNAPYLPSC (SEQ ID NO: 40),
   SGQARMFPNAPYLPSCLES (SEQ ID NO: 41),
   SGQAYMFPNAPYLPSCLES (SEQ ID NO: 42),
   PGCNKRYFKLSHLQMHSRK (SEQ ID NO: 43),
   PGCNKRYFKLSHLQMHSRKH (SEQ ID NO: 44),
   PGCNKRYFKLSHLQMHSRKHTG (SEQ ID NO: 45),
   CNKRYFKLSHLQMHSRK (SEQ ID NO: 46),
   CNKRYFKLSHLQMHSRKH (SEQ ID NO: 47), and
   CNKRYFKLSHLQMHSRKHTG (SEQ ID NO: 48),
   or a pharmaceutically acceptable salt thereof.
[60] The pharmaceutical composition according to item [59], wherein the MHC class II-restricted peptide is a peptide comprising the amino acid sequence of WAPVLDFAPPGASAYGSL (SEQ ID NO: 36) or a pharmaceutically acceptable salt thereof.
[61] The pharmaceutical composition according to item [59], wherein the MHC class II-restricted peptide is a peptide consisting of the amino acid sequence of WAPVLDFAPPGASAYGSL (SEQ ID NO: 36) or a pharmaceutically acceptable salt thereof.
[62] The pharmaceutical composition according to any one of items [58] to [61], wherein
   the compound represented by the formula (1) is a compound of formula (4): 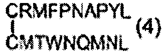 wherein C-C shown in the formula means that the C residues are linked together by a disulfide bond; and
   the MHC class II-restricted peptide is a peptide consisting of an amino acid sequence of WAPVLDFAPPGASAYGSL (SEQ ID NO: 36) or a pharmaceutically acceptable salt thereof.
[63] The pharmaceutical composition according to any one of items [58] to [61], wherein
   the compound represented by the formula (1) is a compound of formula (5): 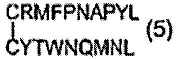 wherein C-C shown in the formula means that the C residues are linked together by a disulfide bond; and
   the MHC class II-restricted peptide is a peptide consisting of an amino acid sequence of WAPVLDFAPPGASAYGSL (SEQ ID NO: 36) or a pharmaceutically acceptable salt thereof.
[64] The pharmaceutical composition according to any one of items [1] to [63], wherein the cancer is a cancer in which WT1 is expressed or cancer associated with an elevated expression level of WT1.
[65] The pharmaceutical composition according to any one of items [1] to [64], wherein the cancer is a blood cancer or a solid cancer.
[66] The pharmaceutical composition according to any one of items [1] to [65], wherein the cancer is selected from chronic or acute leukemia including acute myeloid leukemia, chronic myeloid leukemia, acute lymphocytic leukemia, acute lymphoblastic leukemia and chronic lymphocytic leukemia, myelodysplastic syndrome, multiple myeloma, malignant lymphoma, stomach cancer, colon cancer, lung cancer, breast cancer, germ cell carcinoma, liver cancer, skin cancer, bladder cancer, prostate cancer, uterine cancer, cervical cancer, ovarian cancer, brain tumor, glioma, central nervous system primary malignant lymphoma, Hodgkin lymphoma, non-Hodgkin lymphoma, T cell lymphoma, lymphocytic lymphoma, T cell lymphoma, bone cancer, pancreatic cancer, head and neck cancer, skin or intraorbital malignant melanoma, rectal cancer, anal cancer, testicular cancer, carcinoma of fallopian tube, endometrial carcinoma, cervical carcinoma, vaginal carcinoma, vulvar carcinoma, esophageal cancer, small intestinal cancer, endocrine cancer, thyroid cancer, parathyroid cancer, adrenal cancer, sarcoma of soft tissue, urethral cancer, penile cancer, childhood solid tumor, kidney cancer or ureteral cancer, renal pelvic carcinoma, central nervous system tumor, tumor angiogenesis, spinal tumor, brain stem glioma, pituitary adenoma, Kaposi's sarcoma, epidermoid cancer, squamous cell cancer, environmentally induced cancer including asbestos-induced cancer and a combination of any of these cancers.
[67] The pharmaceutical composition according to item [66], wherein the cancer is selected from chronic or acute leukemia including acute myeloid leukemia, chronic myeloid leukemia, acute lymphoblastic leukemia and chronic lymphocytic leukemia, myelodysplastic syndrome, multiple myeloma, malignant lymphoma, stomach cancer, colon cancer, lung cancer, breast cancer, germ cell carcinoma, liver cancer, skin cancer, bladder cancer, prostate cancer, uterine cancer, cervical cancer, ovarian cancer, brain tumor and glioma.
[68] The pharmaceutical composition according to any one of items [1] to [67], wherein the pharmaceutical composition is used as a cancer vaccine.
[69] The pharmaceutical composition according to any one of items [1] to [67], wherein the pharmaceutical composition is used as a composition for inducing CTLs in cellular immunotherapy for a cancer.
[70] A method of treating a cancer in an HLA-A^{∗}02:07, HLA-A*03:01, HLA-B*15:01 or HLA-B*27:05-positive subject, comprising administering the peptide or a pharmaceutically acceptable salt thereof of (a) or the compound represented by the formula (1) or a pharmaceutically acceptable salt thereof of (b) according to item [1] to an HLA-A^{∗}02:07, HLA-A^{∗}03:01, HLA-B^{∗}15:01 or HLA-B^{∗}27:05-positive subject.
[71] The method according to item [70], wherein the subject is an HLA-A*03:01, HLA-B*15:01 or HLA-B*27:05-positive subject.
[72] The method according to item [70], wherein the subject is an HLA-A*02:07-positive subject.
[73] The method according to item [70], wherein the subject is an HLA-A*03:01-positive subject.
[74] The method according to item [70], wherein the subject is an HLA-B*15:01-positive subject.
[75] The method according to item [70], wherein the subject is an HLA-B*27:05-positive subject.
[76] The method according to any one of items [70] to [75], comprising administering the compound represented by the formula (1) or a pharmaceutically acceptable salt thereof of (b) to the subject.
[77] The method according to any one of items [70] to [76], wherein the compound represented by the formula (1) is
   a compound of formula (4): 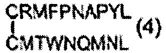 wherein C-C shown in the formula means that the C residues are linked together by a disulfide bond.
[78] The method according to any one of items [70] to [76], wherein the compound represented by the formula (1) is
   a compound of formula (5): 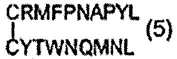 wherein C-C shown in the formula means that the C residues are linked together by a disulfide bond.
[79] The method according to any one of items [70] to [78], further comprising administering an MHC class II-restricted peptide.
[80] The method according to item [79], wherein the peptide or a pharmaceutically acceptable salt thereof of (a) or the compound represented by the formula (1) or a pharmaceutically acceptable salt thereof of (b) and the MHC class II-restricted peptide are contained in a single composition.
[81] The method according to item [79], wherein the peptide or a pharmaceutically acceptable salt thereof of (a) or the compound represented by the formula (1) or a pharmaceutically acceptable salt thereof of (b) and the MHC class II-restricted peptide are contained in separate compositions.
[82] The method according to any one of items [79] to [81], wherein the MHC class II-restricted peptide is a peptide consisting of the amino acid sequence of WAPVLDFAPPGASAYGSL (SEQ ID NO: 36) or a pharmaceutically acceptable salt thereof.
[83] The peptide or a pharmaceutically acceptable salt thereof of (a) or the compound represented by the formula (1) or a pharmaceutically acceptable salt thereof of (b) according to item [1] for use in treating a cancer in an HLA-A^{∗}02:07, HLA-A^{∗}03:01, HLA-B^{∗}15:01 or HLA-B^{∗}27:05-positive subject.
[84] The peptide or compound or a pharmaceutically acceptable salt thereof according to item [83], wherein the subject is an HLA-A*03:01, HLA-B*15:01 or HLA-B*27:05-positive subject.
[85] The peptide or compound or a pharmaceutically acceptable salt thereof according to item [83], wherein the subject is an HLA-A*02:07-positive subject.
[86] The peptide or compound or a pharmaceutically acceptable salt thereof according to item [83], wherein the subject is an HLA-A*03:01-positive subject.
[87] The peptide or compound or a pharmaceutically acceptable salt thereof according to item [83], wherein the subject is an HLA-B*15:01-positive subject.
[88] The peptide or compound or a pharmaceutically acceptable salt thereof according to item [83], wherein the subject is an HLA-B*27:05-positive subject.
[89] The peptide or compound or a pharmaceutically acceptable salt thereof according to any one of items [83] to [88], wherein the peptide or compound or a pharmaceutically acceptable salt thereof is the compound represented by the formula (1) or a pharmaceutically acceptable salt thereof of (b).
[90] The peptide or compound or a pharmaceutically acceptable salt thereof according to any one of items [83] to [89], wherein the compound represented by the formula (1) is
   a compound of formula (4): 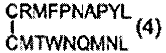 wherein C-C shown in the formula means that the C residues are linked together by a disulfide bond.
[91] The peptide or compound or a pharmaceutically acceptable salt thereof according to any one of items [83] to [89], wherein the compound represented by the formula (1) is
   a compound of formula (5): 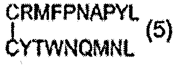 wherein C-C shown in the formula means that the C residues are linked together by a disulfide bond.
[92] The peptide or compound or a pharmaceutically acceptable salt thereof according to any one of items [83] to [91], wherein the peptide or compound or a pharmaceutically acceptable salt thereof is used in combination with an MHC class II-restricted peptide.
[93] The peptide or compound or a pharmaceutically acceptable salt thereof according to item [92], wherein the peptide or a pharmaceutically acceptable salt thereof of (a) or the compound represented by the formula (1) or a pharmaceutically acceptable salt thereof of (b) and the MHC class II-restricted peptide are contained in a single composition.
[94] The peptide or compound or a pharmaceutically acceptable salt thereof according to item [92], wherein the peptide or a pharmaceutically acceptable salt thereof of (a), or the compound represented by the formula (1) or a pharmaceutically acceptable salt thereof of (b) and the MHC class II-restricted peptide are contained in separate compositions.
[95] The peptide or compound or a pharmaceutically acceptable salt thereof according to any one of items [92] to [94], wherein the MHC class II-restricted peptide is a peptide consisting of the amino acid sequence of WAPVLDFAPPGASAYGSL (SEQ ID NO: 36) or a pharmaceutically acceptable salt thereof.
[96] Use of the peptide or a pharmaceutically acceptable salt thereof of (a) or the compound represented by the formula (1) or a pharmaceutically acceptable salt thereof of (b) according to item [1] for the manufacture of a medicament for treating a cancer in an HLA-A^{∗}02:07, HLA-A^{∗}03:01, HLA-B^{∗}15:01 or HLA-B^{∗}27:05-positive subject.
[97] The use according to item [96], wherein the subject is an HLA-A^{∗}03:01, HLA-B^{∗}15:01 or HLA-B^{∗}27:05-positive subject.
[98] The use according to item [96], wherein the subject is an HLA-A^{∗}02:07-positive subject.
[99] The use according to item [96], wherein the subject is an HLA-A^{∗}03:01-positive subject.
[100] The use according to item [96], wherein the subject is an HLA-B^{∗}15:01-positive subject.
[101] The use according to item [96], wherein the subject is an HLA-B^{∗}27:05-positive subject.
[102] The use according to any one of items [96] to [101], wherein the use is use of the compound represented by the formula (1) or a pharmaceutically acceptable salt thereof of (b).
[103] The use according to any one of items [96] to [102], wherein the compound represented by the formula (1) is
   a compound of formula (4): 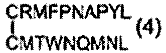 wherein C-C shown in the formula means that the C residues are linked together by a disulfide bond.
[104] The use according to any one of items [96] to [102], wherein the compound represented by the formula (1) is
   a compound of formula (5): 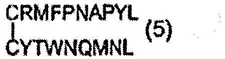 wherein C-C shown in the formula means that the C residues are linked together by a disulfide bond.
[105] The use according to any one of items [96] to [104], wherein the medicament further comprises an MHC class II-restricted peptide or is used in combination with an MHC class II-restricted peptide.
[106] The use according to item [105], wherein the peptide or a pharmaceutically acceptable salt thereof of (a) or the compound represented by the formula (1) or a pharmaceutically acceptable salt thereof of (b) and the MHC class II-restricted peptide are contained in a single composition.
[107] The use according to item [105], wherein the peptide or a pharmaceutically acceptable salt thereof of (a) or the compound represented by the formula (1) or a pharmaceutically acceptable salt thereof of (b) and the MHC class II-restricted peptide are contained in separate compositions.
[108] The use according to any one of items [105] to [107], wherein the MHC class II-restricted peptide is a peptide consisting of the amino acid sequence of WAPVLDFAPPGASAYGSL (SEQ ID NO: 36) or a pharmaceutically acceptable salt thereof.

The present invention will be described by way of the following Examples, which should not be construed as limiting the present invention in any sense.

### Examples

As a first step of the mechanism where CTLs specific to a cancer antigen peptide are stimulated by the peptide and proliferate, it is necessary for the peptide to stably bind to an HLA class I molecule. Each of HLA class I (subtype) molecules requires a peptide binding motif and forms a stable three-dimensional structure as a result of interaction with an amino acid residue present at a predetermined position of an antigen peptide. A plurality of algorithms have been developed for predicting the binding activity between a peptide and an HLA class I molecule based on the motif; for example, NetMHC4.0, SYFPEITHI or BIMAS are known. Use of these algorithms makes it possible to simply list up HLA class I-restricted T cell epitope candidates based on amino acid sequence information of a target protein. To determine whether the epitope candidates obtained by such an algorithm are actually useful for development of cancer immunotherapy, the reactivity of T cells to each epitope candidate is tested by using patient's peripheral blood mononuclear cells.

WT1₁₂₆₋₁₃₄ (RMFPNAPYL (SEQ ID NO: 2)) is a WT1-derived cancer antigen peptide and reported to have a high clinical effect in HLA-A^{∗}02:01 positive cancer patient (Blood. 2009; 113: 6541-8). Actually, the binding affinity of WT1₁₂₆₋₁₃₄ to HLA-A^{∗}02:01 calculated by NetMHC4.0 was 7.14 nM, and WT1₁₂₆₋₁₃₄ was predicted as a peptide having a strong binding affinity to an HLA-A^{∗}02:01 (Table 1).

HLA-A^{∗}02:01 is one of HLA class I subtypes highly frequently observed in Europe and America. HLA-A^{∗}02:07, HLA-A^{∗}03:01, HLA-B^{∗}15:01 and HLA-B^{∗}27:05 are relatively highly frequently observed in Europe and America although these frequencies are lower than HLA-A^{∗}02:01 (Hum Immunol. 2001; 62: 1009-30.). The binding affinities of WT1₁₂₆₋₁₃₄ to HLA-A^{∗}02:07, HLA-A^{∗}03:01, HLA-B^{∗}15:01 and HLA-B^{∗}27:05 calculated by NetMHC4.0 were 28,729.70 nM, 1,755.02 nM, 245.03 nM, and 2,060.74 nM, respectively (Table 1). As described, the affinities of WT1₁₂₆₋₁₃₄ to HLA-A^{∗}02:07, HLA-A^{∗}03:01, HLA-B^{∗}15:01 and HLA-B^{∗}27:05 were about 4,023.8, 245.8, 34.3 and 288.6 times as low as that to HLA-A^{∗}02:01.

The binding affinities of an altered peptide of WT1₁₂₆₋₁₃₄ represented by YMFPNAPYL (SEQ ID NO: 13) to HLA-A^{∗}02:01, HLA-A^{∗}02:07, HLA-A^{∗}03:01, HLA-B^{∗}15:01 and HLA-B^{∗}27:05 calculated by NetMHC4.0 were 2.73 nM, 19,806.32 nM, 8,341.40 nM, 190.54 nM and 6,182.73 nM, respectively (Table 2). As described, the affinities of the altered peptide to HLA-A^{∗}02:07, HLA-A^{∗}03:01, HLA-B^{∗}15:01 and HLA-B^{∗}27:05 were about 7,255.1, 3,055.5, 69.8 and 2,264.7 times as low as that to HLA-A^{∗}02:01.

According to these results, it was very unlikely that WT1₁₂₆₋₁₃₄ and the altered peptide thereof represented by YMFPNAPYL (SEQ ID NO: 13) could be used in cancer immunotherapy for an HLA-A^{∗}02:07, HLA-A^{∗}03:01, HLA-B^{∗}15:01 or HLA-B^{∗}27:05-positive subject.

**Table 1**

| HLA class I | Sequence | Affinity (nM) |
|---|---|---|
| HLA-A^{∗}02:01 | RMFPNAPYL (SEQ ID NO: 2) | 7.14 |
| HLA-A^{∗}02:07 | RMFPNAPYL (SEQ ID NO: 2) | 28,729.70 |
| HLA-A^{∗}03:01 | RMFPNAPYL (SEQ ID NO: 2) | 1,755.02 |
| HLA-B^{∗}15:01 | RMFPNAPYL (SEQ ID NO: 2) | 245.03 |
| HLA-B^{∗}27:05 | RMFPNAPYL (SEQ ID NO: 2) | 2,060.74 |

**Table 2**

| HLA class I | Sequence | Affinity (nM) |
|---|---|---|
| HLA-A^{∗}02:01 | YMFPNAPYL (SEQ ID NO: 13) | 2.73 |
| HLA-A^{∗}02:07 | YMFPNAPYL (SEQ ID NO: 13) | 19,806.32 |
| HLA-A^{∗}03:01 | YMFPNAPYL (SEQ ID NO: 13) | 8,341.40 |
| HLA-B^{∗}15:01 | YMFPNAPYL (SEQ ID NO: 13) | 190.54 |
| HLA-B^{∗}27:05 | YMFPNAPYL (SEQ ID NO: 13) | 6,182.73 |

Next, the reactivity of T cells derived from peripheral blood of a cancer patient to WT1₁₂₆₋₁₃₄ was analyzed. PBMCs used herein were prepared from peripheral blood provided by cancer patients registered in clinical trials (ClinicalTrials.gov Identifier: NCT03149003 and NCT02436252). Specifically, of the cancer patients registered, HLA-A^{∗}03:01-positive patients (4 patients), an HLA-B^{∗}15:01-positive patient (1 patient) and an HLA-A^{∗}02:07-positive patient (1 patient) whose blood could be collected before and after administration of the medicinal agent of the clinical trial were selected and their PBMCs were used. The medicinal agent is a cancer peptide vaccine containing a trifluoro acetate of a compound represented by the following formula (5): 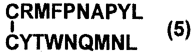 and an acetate of a peptide consisting of the amino acid sequence represented by WAPVLDFAPPGASAYGSL (SEQ ID NO: 36).

PBMCs were prepared before or after administration of the medicinal agent, frozen, thawed in a warm bath of 37°C, and then, suspended in AIM-V medium. To individual wells of a 96-well U-bottom microplate, living cells were seeded at a density (concentration) of 1.8 to 2.2 × 10⁶ cells/mL (volume: 200 µL/well). At this time, human IL-2 (100 U/mL) and the compound of formula (5) or the peptide of SEQ ID NO: 13 (40 µg/mL) were added. Thereafter, culture was initiated at 37°C in a 5% CO₂ incubator. Three days after initiation of the culture, the supernatant (100 µL) was removed from individual wells, AIM-V medium (100 µL) containing human IL-2 (100 U/mL) and the compound of formula (5) or the peptide of SEQ ID NO: 13 (40 µg/mL) was added. Seven days after initiation of the culture, PBMCs were recovered and a part of the PBMCs was stained with WT1₁₂₆₋₁₃₄/HLA-A^{∗}03:01 tetramer, WT1₁₂₆₋₁₃₄/HLA-B^{∗}15:01 tetramer or WT1₁₂₆₋₁₃₄/HLA-A^{∗}02:07 tetramer and an anti-human CD8 antibody. Tetramer-positive cells in CD8 positive T cells were detected by MACSQuant Analyzer. The result was determined to be positive when the ratio of tetramer-positive T cells was 0.1% or more.

PBMCs provided by HLA-A*03:01-positive patients before administration of the medicinal agent were cultured in the presence of the compound of formula (5) and stained with an anti-CD8 antibody and WT1₁₂₆₋₁₃₄/HLA-A^{∗}03:01 tetramer. HLA-A^{∗}03:01-restricted WT1₁₂₆₋₁₃₄-specific CD8-positive T cells were not detected in any of the PBMC samples derived from the patients. In Figure 1, the horizontal axis represents staining with an FITC-labeled anti-CD8 antibody and the vertical axis represents staining with a PE-labeled WT1₁₂₆₋₁₃₄/HLA-A^{∗}03:01 tetramer. Similarly, when PBMCs provided by an HLA-B*15:01-positive patient before administration of the medicinal agent were cultured in the presence of the compound of formula (5) and stained with an anti-CD8 antibody and a WT1₁₂₆₋₁₃₄/HLA-B^{∗}15:01 tetramer, HLA-B^{∗}15:01-restricted WT1₁₂₆₋₁₃₄-specific CD8-positive T cells were not detected. In Figure 2, the horizontal axis represents staining with an FITC-labeled anti-CD8 antibody and the vertical axis represents staining with a PE-labeled WT1₁₂₆₋₁₃₄/HLA-B^{∗}15:01 tetramer. Similarly, when PBMCs provided by an HLA-A^{∗}02:07-positive patient before administration of the medicinal agent were cultured in the presence of the compound of formula (5) and stained with an anti-CD8 antibody and WT1₁₂₆₋₁₃₄/HLA-A^{∗}02:07 tetramer, HLA-A*02:07-restricted WT1₁₂₆₋₁₃₄-specific CD8-positive T cells were not detected. In Figure 7, the horizontal axis represents staining with an FITC-labeled anti-CD8 antibody and the vertical axis represents staining with a PE-labeled WT1₁₂₆₋₁₃₄/HLA-A^{∗}02:07 tetramer. As described, in the assays using T cells derived from peripheral blood samples of cancer patients, WT1₁₂₆₋₁₃₄ was not confirmed useful as an antigen peptide for cancer immunotherapy for an HLA-A^{∗}03:01, HLA-B^{∗}15:01 or HLA-A^{∗}02:07-positive patient.

As described above, from the prediction on HLA binding and the assays using peripheral blood samples of cancer patients before administration of the medicinal agent, it was considered difficult to develop WT1₁₂₆₋₁₃₄ as an agent for treating a cancer of HLA-A^{∗}03:01-positive patient, HLA-B^{∗}15:01-positive patient or HLA-A^{∗}02:07-positive patient.

However, in assays using peripheral blood samples of cancer patients after administration of the medicinal agent, different results were obtained. When PBMCs of the HLA-A^{∗}03:01-positive patients after administration of the medicinal agent were cultured in the presence of the compound of formula (5) and stained with an anti-CD8 antibody and a WT1₁₂₆₋₁₃₄/HLA-A^{∗}03:01 tetramer in accordance with above method, surprisingly, HLA-A^{∗}03:01-restricted WT1₁₂₆₋₁₃₄-specific CD8-positive T cells were detected. In Figure 3, the horizontal axis represents staining with an FITC-labeled anti-CD8 antibody and the vertical axis represents staining with a PE-labeled WT1₁₂₆₋₁₃₄/HLA-A^{∗}03:01 tetramer. Similarly, when PBMCs of the HLA-B^{∗}15:01-positive patient after administration of the medicinal agent were cultured in the presence of the compound of formula (5) and stained with an anti-CD8 antibody and a WT1₁₂₆₋₁₃₄/HLA-B^{∗}15:01 tetramer, HLA-B^{∗}15:01-restricted WT1₁₂₆₋₁₃₄-specific CD8-positive T cells were detected. In Figure 4, the horizontal axis represents staining with an FITC-labeled anti-CD8 antibody and the vertical axis represents staining with a PE-labeled WT1₁₂₆₋₁₃₄/HLA-B^{∗}15:01 tetramer. Similarly, when PBMCs of the HLA-A^{∗}02:07-positive patient after administration of the medicinal agent were cultured in the presence of the compound of formula (5) and stained with an anti-CD8 antibody and a WT1₁₂₆₋₁₃₄/HLA-A^{∗}02: 07 tetramer, HLA-A^{∗}02:07-restricted WT1₁₂₆₋₁₃₄-specific CD8-positive T cells were detected. In Figure 8, the horizontal axis represents staining with an FITC-labeled anti-CD8 antibody and the vertical axis represents staining with a PE-labeled WT1₁₂₆₋₁₃₄/HLA-A^{∗}02:07 tetramer.

Further, HLA-A^{∗}03:01, HLA-B^{∗}15:01 or HLA-A^{∗}02:07-restricted WT1₁₂₆₋₁₃₄ specific T cells proliferated by culturing were analyzed by IFNγ ELISPOT assay to determine if they responded to cancer cells presenting WT1₁₂₆₋₁₃₄. To describe more specifically, PBMCs containing WT1₁₂₆₋₁₃₄ specific T cells were stimulated with HLA-A^{∗}03:01, HLA-B^{∗}15:01 or HLA-A^{∗}02:07 expressing K562 cells pulsed with WT1₁₂₆₋₁₃₄ peptide (referred to as "K562-A3 + Pep", "K562-B15 + Pep" or "K562-A2.7 + Pep") or HLA-A^{∗}03:01, HLA-B^{∗}15:01 or HLA-A^{∗}02:07 expressing K562 cells not pulsed with WT1₁₂₆₋₁₃₄ peptide (referred to as "K562-A3", "K562-B15", or "K562-A2.7"). After the cells were incubated at 37°C in a 5% CO₂ incubator for about 18 hours, individual wells were photographed by ImmunoSpot S5 Versa.

When PBMCs containing HLA-A^{∗}03:01-restricted WT1₁₂₆₋₁₃₄-specific CD8-positive T cells were cultured for stimulation with K562-A3 or K562-A3 + Pep, the cells were more responsive to K562-A3 + Pep than K562-A3 (Figure 5). From this result, HLA-A^{∗}03:01-restricted WT1₁₂₆₋₁₃₄ specific T cells were found to recognize and react with HLA-A^{∗}03:01 positive cancer cells presenting WT1₁₂₆₋₁₃₄. Similarly, when PBMCs containing HLA-B^{∗}15:01-restricted WT1₁₂₆₋₁₃₄-specific CD8-positive T cells were cultured for stimulation with K562-B15 or K562-B15 + Pep, the cells were more responsive to K562-B15 + Pep than K562-B15 (Figure 6). From this result, HLA-B^{∗}15:01-restricted WT1₁₂₆₋₁₃₄ specific T cells were found to recognize/react with HLA-B^{∗}15:01 positive cancer cells presenting WT1₁₂₆₋₁₃₄. Similarly, when PBMCs containing HLA-A^{∗}02:07-restricted WT1₁₂₆₋₁₃₄-specific CD8-positive T cells were cultured for stimulation with K562-A2.7 or K562-A2.7 + Pep, the cells were more responsive to K562-A2.7 + Pep than K562-A2.7 (Figure 9). From this result, HLA-A^{∗}02:07-restricted WT1₁₂₆₋₁₃₄ specific T cells were found to recognize/react with HLA-A^{∗}02:07-positive cancer cells presenting WT1₁₂₆₋₁₃₄.

As described in the above, by using PBMC samples derived from cancer patients who received the medicinal agent, it was demonstrated that WT1₁₂₆₋₁₃₄ could be an agent for treating a cancer of not only HLA-A^{∗}02:01-positive patient but also HLA-A^{∗}03:01, . HLA-B^{∗}15:01 or HLA-A^{∗}02:07-positive patients.

Also, when PBMCs of HLA-A^{∗}03:01-positive patients after administration of the medicinal agent were cultured in the presence of the peptide of SEQ ID NO: 13 and stained with an anti-CD8 antibody and a WT1₁₂₆₋₁₃₄/HLA-A^{∗}03:01 tetramer, HLA-A^{∗}03:01-restricted WT1₁₂₆₋₁₃₄-specific CD8-positive T cells were detected. In Figure 10, the horizontal axis represents staining with an FITC-labeled anti-CD8 antibody and the vertical axis represents staining with a PE-labeled WT1₁₂₆₋₁₃₄/HLA-A^{∗}03:01 tetramer. Similarly, when PBMCs of an HLA-B^{∗}15:01-positive patient after administration of the medicinal agent were cultured in the presence of the peptide of SEQ ID NO: 13 and stained with an anti-CD8 antibody and a WT1₁₂₆₋₁₃₄/HLA-B^{∗}15:01 tetramer, HLA-B^{∗}15:01-restricted WT1₁₂₆₋₁₃₄-specific CD8-positive T cells were detected. In Figure 11, the horizontal axis represents staining with an FITC-labeled anti-CD8 antibody and the vertical axis represents staining with a PE-labeled WT1₁₂₆₋₁₃₄/HLA-B^{∗}15:01 tetramer. Similarly, when PBMCs of an HLA-A^{∗}02:07-positive patient after administration of the medicinal agent were cultured in the presence of the peptide of SEQ ID NO: 13 and stained with an anti-CD8 antibody and a WT1₁₂₆₋₁₃₄/HLA-A^{∗}02:07 tetramer, HLA-A^{∗}02:07-restricted WT1₁₂₆₋₁₃₄-specific CD8-positive T cells were detected. In Figure 12, the horizontal axis represents staining with an FITC-labeled anti-CD8 antibody and the vertical axis represents staining with a PE-labeled WT1₁₂₆₋₁₃₄/HLA-A^{∗}02:07 tetramer.

From these results, it was demonstrated that an altered killer peptide of WT1₁₂₆₋₁₃₄ activated and proliferated HLA-A^{∗}03:01-restricted WT1₁₂₆₋₁₃₄-specific CD8-positive T cells, HLA-B^{∗}15:01-restricted WT1₁₂₆₋₁₃₄-specific CD8-positive T cells and HLA-A^{∗}02:07-restricted WT1₁₂₆₋₁₃₄-specific CD8-positive T cells. Similar to WT1₁₂₆₋₁₃₄, an altered killer peptide of WT1₁₂₆₋₁₃₄ was shown to be useful as an agent for treating cancers in not only HLA-A^{∗}02:01-positive patients but also HLA-A^{∗}03:01, HLA-B^{∗}15:01 or HLA-A^{∗}02:07-positive patients.

## Claims

1. A pharmaceutical composition for treating a cancer in an HLA-A^{∗}02:07, HLA-A^{∗}03:01, HLA-B^{∗}15:01, or HLA-B^{∗}27:05-positive subject, comprising:
(a) an MHC class I-restricted peptide consisting of 7 to 30 amino acid residues or a pharmaceutically acceptable salt thereof, wherein the MHC class I-restricted peptide is a peptide comprising an amino acid sequence selected from RMFPNAPYL (SEQ ID NO: 2), CMTWNQMNL (SEQ ID NO: 3), ALLPAVPSL (SEQ ID NO: 4), SLGEQQYSV (SEQ ID NO: 5), RVPGVAPTL (SEQ ID NO: 6) and VLDFAPPGA (SEQ ID NO: 7), or a peptide comprising an amino acid sequence which has one or several amino acids deleted from, substituted in, and/or added to the amino acid sequence selected from SEQ ID NOs: 2 to 7 and having an ability to induce CTLs; or
(b) a compound represented by the formula (1) or a pharmaceutically acceptable salt thereof: 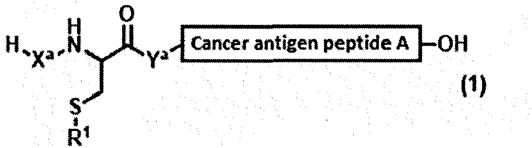 wherein X^{a} and Y^{a} each independently represent a single bond or a divalent peptide group consisting of 1 to 4 amino acid residues, provided that the sum of the numbers of amino acid residues in X^{a} and Y^{a} is an integer of 0 to 4;
cancer antigen peptide A is an MHC class I-restricted peptide consisting of 7 to 30 amino acid residues, wherein an amino group of an N-terminal amino acid of the cancer antigen peptide A binds to Y^{a} in the formula (1), and a carbonyl group of a C-terminal amino acid of the cancer antigen peptide A binds to the hydroxyl group in the formula (1),
R¹ is a hydrogen atom,
a group represented by the formula (2): 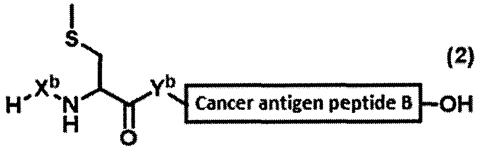 wherein X^{b} and Y^{b} each independently represent a single bond or a divalent peptide group consisting of 1 to 4 amino acid residues, provided that the sum of the numbers of amino acid residues in X^{b} and Y^{b} is an integer of 0 to 4,
cancer antigen peptide B is an MHC class I-restricted peptide consisting of 7 to 30 amino acid residues, wherein an amino group of an N-terminal amino acid of the cancer antigen peptide B binds to Y^{b} in the formula (2), and a carbonyl group of a C-terminal amino acid of the cancer antigen peptide B binds to the hydroxyl group in the formula (2), and
the sulfur atom in the formula (2) binds to the sulfur atom in the formula (1) via a disulfide bond,
or cancer antigen peptide C, wherein the cancer antigen peptide C is an MHC class I-restricted peptide consisting of 7 to 30 amino acid residues including one cysteine residue or an MHC class II-restricted peptide consisting of 7 to 30 amino acid residues including one cysteine residue, and a sulfur atom of the cysteine residue of the cancer antigen peptide C binds to the sulfur atom in the formula (1) via a disulfide bond, and optionally a peptide consisting of 1 to 4 amino acid residues binds to an N-terminus of the cancer antigen peptide C,
provided that when R¹ is a hydrogen atom, the cancer antigen peptide A is a peptide comprising an amino acid sequence selected from RMFPNAPYL (SEQ ID NO: 2), CMTWNQMNL (SEQ ID NO: 3), ALLPAVPSL (SEQ ID NO: 4), SLGEQQYSV (SEQ ID NO: 5), RVPGVAPTL (SEQ ID NO: 6) and VLDFAPPGA (SEQ ID NO: 7), or a peptide comprising an amino acid sequence which has one or several amino acids deleted from, substituted in, and/or added to the amino acid sequence selected from SEQ ID NOs: 2 to 7 and having an ability to induce CTLs;
when R¹ is the group represented by the formula (2), the cancer antigen peptide A and/or cancer antigen peptide B is a peptide comprising an amino acid sequence selected from RMFPNAPYL (SEQ ID NO: 2), CMTWNQMNL (SEQ ID NO: 3), ALLPAVPSL (SEQ ID NO: 4), SLGEQQYSV (SEQ ID NO: 5), RVPGVAPTL (SEQ ID NO: 6) and VLDFAPPGA (SEQ ID NO: 7), or a peptide comprising an amino acid sequence which has one or several amino acids deleted from, substituted in, and/or added to the amino acid sequence selected from SEQ ID NOs: 2 to 7 and having an ability to induce CTLs;
when R¹ is the cancer antigen peptide C, the cancer antigen peptide A and/or cancer antigen peptide C is a peptide comprising an amino acid sequence selected from RMFPNAPYL (SEQ ID NO: 2), CMTWNQMNL (SEQ ID NO: 3), ALLPAVPSL (SEQ ID NO: 4), SLGEQQYSV (SEQ ID NO: 5), RVPGVAPTL (SEQ ID NO: 6) and VLDFAPPGA (SEQ ID NO: 7), or a peptide comprising an amino acid sequence which has one or several amino acids deleted from, substituted in, and/or added to the amino acid sequence selected from SEQ ID NOs: 2 to 7 and having an ability to induce CTLs;
when R¹ is the group represented by the formula (2) and the cancer antigen peptide B includes one cysteine residue, a sulfur atom of the cysteine residue of the cancer antigen peptide B optionally binds, via a disulfide bond, to
a sulfur atom in the formula (3): 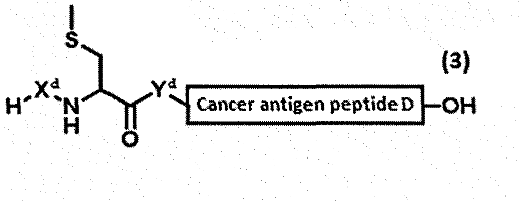 wherein X^{d} and Y^{d} each independently represent a single bond or a divalent peptide group consisting of 1 to 4 amino acid residues, provided that the sum of the numbers of amino acid residues in X^{d} and Y^{d} is an integer of 0 to 4, and
cancer antigen peptide D is an MHC class II-restricted peptide consisting of 7 to 30 amino acid residues, wherein an amino group of an N-terminal amino acid of the cancer antigen peptide D binds to Y^{d} in the formula (3), and a carbonyl group of a C-terminal amino acid of the cancer antigen peptide D binds to the hydroxyl group in the formula (3),
or a sulfur atom of a cysteine residue of cancer antigen peptide E, wherein the cancer antigen peptide E is an MHC class II-restricted peptide consisting of 7 to 30 amino acid residues including one cysteine residue; and
when R¹ is the cancer antigen peptide C and a peptide consisting of 1 to 4 amino acid residues including one cysteine residue binds to the N terminus of the cancer antigen peptide C, a sulfur atom of the cysteine residue of the peptide binding to the N terminus of the cancer antigen peptide C optionally binds, via a disulfide bond, to
a sulfur atom in the formula (3): 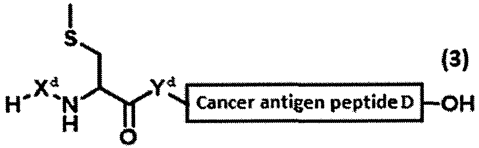 wherein X^{d} and Y^{d} each independently represent a single bond or a divalent peptide group consisting of 1 to 4 amino acid residues, provided that the sum of the numbers of amino acid residues in X^{d} and Y^{d} is an integer of 0 to 4, and
cancer antigen peptide D is an MHC class II-restricted peptide consisting of 7 to 30 amino acid residues, wherein an amino group of an N-terminal amino acid of the cancer antigen peptide D binds to Y^{d} in the formula (3), and a carbonyl group of a C-terminal amino acid of the cancer antigen peptide D binds to the hydroxyl group in the formula (3),
or a sulfur atom of a cysteine residue of cancer antigen peptide E, wherein the cancer antigen peptide E is an MHC class II-restricted peptide consisting of 7 to 30 amino acid residues including one cysteine residue.

2. The pharmaceutical composition according to claim 1, wherein the subject is an HLA-A^{∗}03:01, HLA-B^{∗}15:01 or HLA-B^{∗}27:05-positive subject.

3. The pharmaceutical composition according to claim 1, wherein the subject is an HLA-A^{∗}02:07-positive subject.

4. The pharmaceutical composition according to claim 1, wherein the subject is an HLA-A^{∗}03:01-positive subject.

5. The pharmaceutical composition according to claim 1, wherein the subject is an HLA-B^{∗}15:01-positive subject.

6. The pharmaceutical composition according.to claim 1, wherein the subject is an HLA-B^{∗}27:05-positive subject.

7. The pharmaceutical composition according to any one of claims 1 to 6, comprising the compound represented by the formula (1) or a pharmaceutically acceptable salt thereof of (b).

8. The pharmaceutical composition according to claim 7, wherein the cancer antigen peptide A is a peptide comprising an amino acid sequence selected from
RMFPNAPYL (SEQ ID NO: 2),
CMTWNQMNL (SEQ ID NO: 3),
ALLPAVPSL (SEQ ID NO: 4),
SLGEQQYSV (SEQ ID NO: 5),
RVPGVAPTL (SEQ ID NO: 6) and
VLDFAPPGA (SEQ ID NO: 7), or
a peptide comprising an amino acid sequence which has one or several amino acids deleted from, substituted in, and/or added to the amino acid sequence selected from SEQ ID NOs: 2 to 7 and having an ability to induce CTLs.

9. The pharmaceutical composition according to claim 8, wherein the cancer antigen peptide A is a peptide consisting of an amino acid sequence of RMFPNAPYL (SEQ ID NO: 2).

10. The pharmaceutical composition according to any one of claims 7 to 9, wherein R¹ is the cancer antigen peptide C.

11. The pharmaceutical composition according to claim 10, wherein the cancer antigen peptide C is the MHC class I-restricted peptide.

12. The pharmaceutical composition according to claim 11, wherein the cancer antigen peptide C is a peptide comprising the amino acid sequence of CMTWNQMNL (SEQ ID NO: 3) or a peptide comprising an amino acid sequence which has one or several amino acids deleted from, substituted in, and/or added to the amino acid sequence of SEQ ID NO: 3 and having an ability to induce CTLs.

13. The pharmaceutical composition according to claim 12, wherein the cancer antigen peptide C is a peptide comprising or consisting of an amino acid sequence selected from
CMTWNQMNL (SEQ ID NO: 3) and
CYTWNQMNL (SEQ ID NO: 14).

14. The pharmaceutical composition according to any one of claims 7 to 13, wherein the compound represented by the formula (1) is
a compound of formula (4): 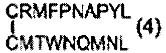 wherein C-C shown in the formula means that the C residues are linked together by a disulfide bond, or
a compound of formula (5): 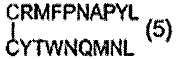 wherein C-C shown in the formula means that the C residues are linked together by a disulfide bond.

15. The pharmaceutical composition according to claim 14, wherein the compound represented by the formula (1) is
a compound of formula (5): 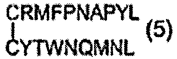 wherein C-C shown in the formula means that the C residues are linked together by a disulfide bond,

16. The pharmaceutical composition according to any one of claims 1 to 15, wherein the pharmaceutical composition further comprises or is used in combination with an MHC class II-restricted peptide.

17. The pharmaceutical composition according to claim 16, wherein the MHC class II-restricted peptide is a peptide comprising or consisting of an amino acid sequence selected from
WAPVLDFAPPGASAYGSL (SEQ ID NO: 36),
CWAPVLDFAPPGASAYGSL (SEQ ID NO: 37),
WAPVLDFAPPGASAYGSLC (SEQ ID NO: 38),
SGQARMFPNAPYLPSC (SEQ ID NO: 39),
SGQAYMFPNAPYLPSC (SEQ ID NO: 40),
SGQARMFPNAPYLPSCLES (SEQ ID NO: 41),
SGQAYMFPNAPYLPSCLES (SEQ ID NO: 42),
PGCNKRYFKLSHLQMHSRK (SEQ ID NO: 43),
PGCNKRYFKLSHLQMHSRKH (SEQ ID NO: 44),
PGCNKRYFKLSHLQMHSRKHTG (SEQ ID NO: 45),
CNKRYFKLSHLQMHSRK (SEQ ID NO: 46),
CNKRYFKLSHLQMHSRKH (SEQ ID NO: 47), and
CNKRYFKLSHLQMHSRKHTG (SEQ ID NO: 48), or
a pharmaceutically acceptable salt thereof.

18. The pharmaceutical composition according to claim 17, wherein the MHC class II-restricted peptide is a peptide consisting of the amino acid sequence of WAPVLDFAPPGASAYGSL (SEQ ID NO: 36) or a pharmaceutically acceptable salt thereof.

19. The pharmaceutical composition according to any one of claims 16 to 18, wherein
the compound represented by the formula (1) is a compound of formula (4): 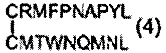 wherein C-C shown in the formula means that the C residues are linked together by a disulfide bond; and
the MHC class II-restricted peptide is a peptide consisting of the amino acid sequence of WAPVLDFAPPGASAYGSL (SEQ ID NO: 36) or a pharmaceutically acceptable salt thereof.

20. The pharmaceutical composition according to any one of claims 16 to 18, wherein
the compound represented by the formula (1) is a compound of formula (5): 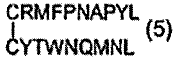 wherein C-C shown in the formula means that the C residues are linked together by a disulfide bond; and
the MHC class II-restricted peptide is a peptide consisting of the amino acid sequence of WAPVLDFAPPGASAYGSL (SEQ ID NO: 36) or a pharmaceutically acceptable salt thereof.

21. The pharmaceutical composition according to any one of claims 1 to 20, wherein the cancer is a cancer in which WT1 is expressed or a cancer with an elevated expression level of WT1.

22. The pharmaceutical composition according to any one of claims 1 to 21, wherein the cancer is selected from chronic or acute leukemia including acute myeloid leukemia, chronic myeloid leukemia, acute lymphocytic leukemia, acute lymphoblastic leukemia and chronic lymphocytic leukemia, myelodysplastic syndrome, multiple myeloma, malignant lymphoma, stomach cancer, colon cancer, lung cancer, breast cancer, germ cell carcinoma, liver cancer, skin cancer, bladder cancer, prostate cancer, uterine cancer, cervical cancer, ovarian cancer, brain tumor, glioma, central nervous system primary malignant lymphoma, Hodgkin lymphoma, non-Hodgkin lymphoma, T cell lymphoma, lymphocytic lymphoma, T cell lymphoma, bone cancer, pancreatic cancer, head and neck cancer, skin or intraorbital malignant melanoma, rectal cancer, anal cancer, testicular cancer, carcinoma of fallopian tube, endometrial carcinoma, cervical carcinoma, vaginal carcinoma, vulvar carcinoma, esophageal cancer, small intestinal cancer, endocrine cancer, thyroid cancer, parathyroid cancer, adrenal cancer, sarcoma of soft tissue, urethral cancer, penile cancer, childhood solid tumor, kidney cancer or ureteral cancer, renal pelvic carcinoma, central nervous system tumor, tumor angiogenesis, spinal tumor, brain stem glioma, pituitary adenoma, Kaposi's sarcoma, epidermoid cancer, squamous cell cancer, environmentally induced cancer including asbestos-induced cancer and a combination of any of these cancers.

23. The pharmaceutical composition according to any one of claims 1 to 22, wherein the pharmaceutical composition is used as a cancer vaccine.

24. The pharmaceutical composition according to any one of claims 1 to 23, wherein the pharmaceutical composition is used as a composition for inducing CTLs in cellular immunotherapy for a cancer.
